(19) Europäisches Patentamt
European Patent Office
Office européen des brevets

(11) EP 2 772 489 A1

(12) EUROPÄISCHE PATENTANMELDUNG

(43) Veröffentlichungstag:
**03.09.2014 Patentblatt 2014/36**

(21) Anmeldenummer: **13192811.1**

(22) Anmeldetag: **14.11.2013**

(51) Int Cl.:
***C07D 307/12*** (2006.01) ***C07C 29/17*** (2006.01)
***B01J 21/04*** (2006.01) ***B01J 23/46*** (2006.01)
*C07C 31/20* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **28.02.2013 DE 102013203420**

(71) Anmelder: **Evonik Industries AG**
**45128 Essen (DE)**

(72) Erfinder:
- **Omeis, Marianne**
  **46286 Dorsten (DE)**
- **Neumann, Manfred**
  **45770 Marl (DE)**
- **Brehme, Volker**
  **48301 Nottuln (DE)**
- **Theis, Christoph**
  **53859 Niederkassel (DE)**
- **Wolf, Dorit**
  **61440 Oberursel (DE)**
- **Claus, Peter**
  **13187 Berlin (DE)**
- **Lucas, Martin**
  **64285 Darmstadt (DE)**
- **Eckert, Rene**
  **64347 Griesheim (DE)**

# (54) Hydrogenolyse von Furfurylalkohol zu 1,2-Pentandiol oder zu Tetrahydofurfurylalkohol

(57) Diese Erfindung betrifft ein Verfahren zur Herstellung von 1,2-Pentandiol oder Tetrahydrofurfurylalkohol, welches dadurch gekennzeichnet ist, dass man Furfurylalkohol mit Wasserstoff in Gegenwart eines Katalysatorsystems umsetzt. Das Katalysatorsystem kann dabei Platinoxid sein oder Ruthenium, welches auf Aluminiumoxid oder Aktivkohle geträgert ist. Die Erfindung betrifft auch die jeweiligen Katalysatoren sowie Verfahren zu deren Herstellung.

EP 2 772 489 A1

## Beschreibung

Diese Erfindung betrifft ein Verfahren zur Herstellung von 1,2-Pentandiol, welches dadurch gekennzeichnet ist, dass man Furfurylalkohol mit Wasserstoff in Gegenwart eines Katalysatorsystems umsetzt. Bei den verschiedenen Katalysatorsystemen handelt es sich um heterogene Katalysatoren. Das Katalysatorsystem ist dabei in einem ersten Aspekt der Erfindung Ruthenium, welches auf einem Träger aus Aluminiumoxid geträgert ist. In einem zweiten Aspekt der Erfindung ist das Katalysatorsystem Ruthenium, welches auf einem Träger aus Aktivkohle geträgert ist. In einem dritten Aspekt der Erfindung ist das Katalysatorsystem Pt(IV)-Oxid.

Die Erfindung betrifft daneben auch Verfahren zur Herstellung eines Katalysatorsystems, wobei das Katalysatorsystem Ruthenium, welches auf einem Träger aus Aluminiumoxid oder Aktivkohle geträgert ist, umfasst. Die Erfindung betrifft auch die nach dem Verfahren zur Herstellung des Katalysatorsystems hergestellten Katalysatoren.

Das erfindungsgemäße Verfahren zur Herstellung von 1,2-Pentandiol kann gemäß der Literatur im Batchreaktor, im Multibatchreaktor oder in einer Glasapparatur durchgeführt.

Diese Erfindung beschreibt die wichtigsten Nebenprodukte und ein umfassendes Reaktionsschema für die Hydrogenolyse von Furfurylalkohol an Ruthenium-Trägerkatalysatoren, die auch für Pt-Trägerkatalysatoren gelten.

Diese Erfindung betrifft in einer bevorzugten Ausführungsform ein Verfahren zur Herstellung von 1,2-Pentandiol, welches dadurch gekennzeichnet ist, dass man Furfurylalkohol mit Wasserstoff in Gegenwart eines Katalysatorsystems umfassend Ruthenium, welches auf einem Träger aus Aluminiumoxid geträgert ist, umsetzt. Diese Erfindung beschreibt insbesondere in bevorzugten Ausführungsformen den Einfluss von Temperatur, Druck, Katalysatormasse, Lösungsmittel, pH-Wert, Rührgeschwindigkeit und Eduktkonzentration auf die Produktverteilung. Zusätzlich beschreibt diese Erfindung die Präparierung bimetallischer Ru-Me-Trägerkatalysatoren sowie deren Performance im erfindungsgemäßen Verfahren. Zudem beschreibt die vorliegende Erfindung den Einfluss geringer Mengen ionischer Flüssigkeiten im Sinne des SCILL-Konzepts. Die Umsätze und Selektivitäten wurden bestimmt, in dem die flüssigen Proben der Reaktionsgemische mittels GC und GC-MS analysiert wurden.

## Hintergrund der Erfindung

Der Großteil der chemischen Industrie basiert heutzutage auf fossilen Rohstoffen. Die wichtigste Rolle spielt dabei das Erdöl, aus dem die Kohlenwasserstoffe gewonnen werden, die als Grundstoffe die Basis für die Erzeugung von chemischen Zwischen- und Endprodukten darstellen. Die steigende Nachfrage nach fossilen Kraftstoffen, vor allem in aufstrebenden Wirtschaftsräumen wie Indien oder China, wird in den nächsten Jahrzehnten zu einer Verknappung des Erdöls führen. [1] (Die jeweiligen Literaturstellen sind im Literaturverzeichnis angegeben).Vor diesem Hintergrund bilden nachwachsende Rohstoffe eine vielversprechende Alternative für die chemische Industrie. In Deutschland wurde der Begriff der "nachwachsenden Rohstoffe" 1973 im Kontext der Ölkrise geprägt, als die negativen Auswirkungen der Abhängigkeit von importiertem Erdöl besonders deutlich wurden. Inzwischen wird die Investition in nachwachsende Rohstoffe auch zunehmend von staatlichen Einrichtungen [2, 3] und internationalen Organisationen wie der SUSCHEM [4] gefördert. Es wird erwartet, dass bereits 2025 in Europa 30% aller Chemikalien aus Biomasse hergestellt werden. [5] Die Natur produziert jährlich rund 170 Billionen Tonnen Biomasse durch Photosynthese, von denen nur 3 - 4 % vom Menschen genutzt werden. Mit 75 % bilden Kohlenhydrate davon den Löwenanteil. [6] Sie kommen vor allem in Form von Polysacchariden vor, die aus C5- oder C6-Monosacchariden zusammengesetzt sind. Die Polysaccharide Hemicellulose (C5) und Cellulose (C6) bilden durch Einlagerung des phenolischen Biopolymers Lignin die sogenannte Lignocellulose, das Strukturgerüst aller verholzten Pflanzen. Sie fällt in großen Mengen als Abfallprodukt in der Agrar- und Forstwirtschaft an. Furfural (Strukturformel siehe Schema 1) ist ein chemisches Zwischenprodukt, das großtechnisch ausschließlich aus solchen Bioabfällen hergestellt wird. [7] Es ist derzeit die einzige ungesättigte Bulk-Chemikalie, die aus Kohlenhydraten produziert wird. [8]

Die Nutzung von nachwachsenden Rohstoffen spielt eine wichtige Rolle in der *Grünen Chemie,* einer seit den 90er Jahren stetig an Bedeutung gewinnenden Philosophie, die eine nachhaltige und sichere chemische Industrie anstrebt. [9], [55], [56] Ein weiterer Grundpfeiler der grünen Chemie ist die Katalyse. Der Einsatz möglichst aktiver und selektiver Katalysatoren bietet eine Reihe von Vorteilen, die für eine nachhaltige Chemie von Bedeutung sind: Die Verwendung stöchiometrischer Reagenzien wird vermieden, der Energiebedarf durch Herabsetzung der Reaktionstemperatur reduziert und die Bildung von entsorgungsbedürftigen Nebenprodukten verringert. Besonders attraktiv sind in diesem Zusammenhang heterogene Katalysatoren, da sie in der Regel leicht vom Produkt abzutrennen sind, ohne dass große Energiemengen für thermische Trennverfahren aufgewendet werden müssen. [10]

Furfural wurde erstmals 1831 vom deutschen Chemiker Wolfgang Döbereiner als Nebenprodukt bei der Ameisensäuresynthese isoliert, als er Kohlenhydrate mit Schwefelsäure und $MnO_2$ behandelte. [11] Die Strukturformel wurde 1901 von Carl Harries aufgeklärt. Die erste kommerzielle Produktion von Furfural erfolgte 1922 aus Haferspelzen. [7] Als Ausgangsstoff zur Furfuralproduktion dienen heute Bioabfälle, die zum Beispiel beim Zucker- und Maisanbau anfallen. Diese enthalten Hemicellulose, welches als Bestandteil der Lignocellulose in den Zellwänden der meisten Pflanzen

enthalten ist. Hemicellulose ist ein Gemisch von Polysacchariden in veränderlicher Zusammensetzung, welches als Monomere hauptsächlich Pentosen - Einfachzucker mit fünf Kohlenstoffatomen - enthält. Durch Behandlung mit heißer Schwefelsäure wird Hemicellulose hydrolysiert und die Monosaccharide werden freigesetzt. Unter diesen Reaktionsbedingungen tritt als Folgereaktion eine Dehydratisierung der Pentosen zu Furfural auf. 2003 wurden weltweit 200.000 Tonnen Furfural produziert, über die Hälfte davon in China. Etwa zwei Drittel des produzierten Furfurals werden zur Herstellung von Furfurylalkohol verwendet. [7, 12] Die Hydrierung von Furfural zu Furfurylalkohol erfolgt industriell an Kupferchromit-Katalysatoren und kann sowohl in der Flüssigphase als auch in der Gasphase durchgeführt werden. [13, 14] Das wichtigste Anwendungsgebiet von Furfurylalkohol ist die Herstellung von Gießereibindemitteln. Daneben findet er unter anderem Anwendung in der Modifikation von Holz und als Ausgangsstoff für die Synthese von Feinchemikalien. [7, 15]

1,2-Pentandiol (Strukturformel siehe Schema 1), auch als Pentylenglykol bezeichnet, ist ein chirales Diol mit einem stereogenen Zentrum. Die Herstellung des Racemats erfolgt üblicherweise durch Behandlung einer Mischung aus 1-Penten und Ameisensäure mit Wasserstoffperoxid. [16] Eine Synthese der Enantiomere ist zum Beispiel durch Verwendung chiraler Übergangsmetallkomplexe als Katalysatoren möglich. [17] Wie andere 1,2-Diole zeichnet sich Pentylenglykol durch einen starken antibakteriellen Effekt aus. Es findet unter anderem Anwendung in der Synthese von Pestiziden, Pflanzenschutzmitteln und Pharmazeutika. [16]

Im Stand der Technik ist neben der Hydrogenolyse von Tetrahydrofurfurylalkohol auch die Hydrogenolyse von Furfurylalkohol als Möglichkeit zur Synthese von 1,2-Pentandiol beschrieben.

Für die Hydrogenolyse von Furfurylalkohol sind dabei eine Reihe unterschiedlicher heterogener Katalysatoren beschrieben. Neben Kupfer- und Nickelkatalysatoren kommen in diesem Zusammenhang vor allem Platin- und Rutheniumkatalysatoren zum Einsatz.

Die eingesetzten Platinkatalysatoren unterteilen sich in Pt(IV)-Oxid-Katalysatoren und Platin-Trägerkatalysatoren.

Der sogenannte *Adams-Katalysator,* benannt nach seinem Entwickler Roger Adams, ist ein beliebter Katalysator für Hydrier- und Hydrogenolysereaktionen. Er besteht aus Platin(IV)-Oxid, welches in situ zu feinem Platinpulver ("Platinmohr"), dem eigentlichen aktiven Katalysator, reduziert wird. [18], [57] an diesem setzte Adams Furfural bei Raumtemperatur und 1 - 2 bar Wasserstoffdruck in Ethanol unter Zusatz von Eisenchlorid um. Dabei erhielt er ein Produktgemisch aus Tetrahydrofurfurylalkohol (THFFOH, Y = 35 %), 1,2-Pentandiol (1,2 PD, Y = 20 %), 1,5-Pentandiol (1,5-PD, Y = 8 %) und 1-Pentanol (1-POH, Y = 11 1%). Adams konnte zeigen, dass Furfural zunächst vollständig unter Verbrauch von einem Äquivalent Wasserstoff zu Furfurylalkohol (FFOH) hydriert wird, welcher anschließend zu den vier oben genannten Produkten reagiert. Ohne Zusatz von Eisenchlorid zeigte der Adams-Katalysator eine äußerst geringe katalytische Aktivität. [19]

Nishimura setzte als Edukt Furfurylalkohol ein. Die Hydrogenolyse wurde bei Raumtemperatur und Atmosphärendruck in Ethanol unter Zusätzen von kleinen Mengen 3 M Salzsäure durchgeführt. Dabei erhielt er ein Produktgemisch bestehend aus Pentan (Y = 0.8 %), 2-Methyltetrahydrofuran (2-MTHF, Y = 14.8 %), 2-Pentanol (2-POH, Y = 5.5 %), 1-Pentanol (Y = 11.6 %), Tetrahydrofurfurylalkohol (Y = 20.6 %), 1,2-Pentandiol (Y = 13.3 %) und 1,5-Pentandiol (Y = 3.3 %). Wurde ohne Zusatz von Salzsäure gearbeitet, kam es zu einem starken Abfall der Katalysatoraktivität während der Reaktion. Eine Erhöhung des Salzsäuregehaltes führte zu einer verstärkten Bildung der Hydrogenolyseprodukte gegenüber Tetrahydrofurfurylalkohol. [20, 21]

Smith und Fuzek verwendeten Essigsäure als Lösungsmittel und erreichten bei 1.4 - 4 bar Wasserstoffdruck eine nahezu quantitative Umsetzung zu 1,2-Pentandiol. [22]

Demgegenüber ist die Umsetzung von Furfural an Platin-Trägerkatalysatoren ebenfalls beschrieben. Furfural wurde 2011 von Xu *et al.* [23] bei 110 - 150 °C und Wasserstoffdrücken von 15 bis 25 bar an Platin-Trägerkatalysatoren umgesetzt. Es wurden durch gemeinsame Fällung präparierte Katalysatoren $Pt/Co_2AlO_4$ und $Pt/Co_3O_4$ sowie ein durch Imprägnierung hergestellter Katalysator $Pt/Al_2O_3$ mit je 1.9 % Platingehalt bei 130 °C und 15 bar Wasserstoffdruck untersucht. Nach 24 h Reaktionszeit traten als Produkte 2-Methylfuran (2-MF) **1,** 2-Methyltetrahydrofuran **2,** 1-Pentanol **3,** 2-Pentanol **4,** Tetrahydrofurfurylalkohol **5,** 1,2-Pentandiol **6,** 1,4-Pentandiol (1,4-PD) 7 und 1,5-Pentandiol **8** auf. Die Ausbeuten sind in Tabelle 1 zusammengefasst. Die höchste Ausbeute an 1,2-Pentandiol erzielte $Pt/Co_3O_4$ mit 14.8 %. $Pt/Co_2AlO_4$ zeichnete sich durch eine außerordentlich hohe katalytische Aktivität im Vergleich zu $Pt/Al_2O_3$ aus. Verantwortlich dafür sind nach Xu *et al.* die hohe Dispersität des Platins auf dem mesoporösen Cobaltaluminatträger sowie die starke Adsorption der C-C-Doppelbindung an Co(III). Furfural wurde stets rasch zu Furfurylalkohol hydriert, welcher nahezu vollständig zu den Produkten **1** bis **8** abreagierte. Es konnte gezeigt werden, dass die Entstehung von 1,2- und 1,5-Pentandiol aus der Hydrogenolyse des Furanringes von Furfurylalkohol und nicht über Tetrahydrofurfurylalkohol als Zwischenschritt verläuft. Die Hydrogenolyse von Tetrahydrofurfurylalkohol an Platinkatalysatoren stellte sich als äußerst schwierig heraus und führte nur zu geringen Umsätzen. Die Bildung von 1,4-Pentandiol **7** führten Xu *et al.* auf die Isomerisierung von 1,2-Pentandiol zurück. Sie spielte vor allen Dingen bei hohen Reaktionstemperaturen eine Rolle und führte zu einer Verringerung der Ausbeute an 1,2-Pentandiol (Tabelle 2). In Schema 1 sind die Reaktionspfade zusammengefasst, die nach Xu *et al.* zu den unterschiedlichen Reaktionsprodukten führen. Hier und im Folgenden

werden dabei die folgenden Abkürzungen verwendet:

Substanzen

1,2-BD: 1,2-Butandiol; 1,2-PD: 1,2-Pentandiol; 1,4-PD: 1,4-Pentandiol; 1,5-PD: 1,5-Pentandiol; 1,5-HD: 1,5-Hexandiol; 1-BOH: 1-Butanol; 1-POH: 1-Pentanol; 2-POH: 2-Pentanol; 2-MF: 2-Methylfuran; 2-MTHF: 2-Methyltetrahydrofuran; CpO: Cyclopentanon; CpOH: Cyclopentanol; DCA: Dicyanamid; IL: Ionische Flüssigkeit; PANI: Polyanilin; FFOH: Furfurylalkohol; THF: Tetrahydrofuran; THFFOH: Tetrahydrofurfurylalkohol.

Physikalische Größen

c: Massenkonzentration; m: Masse; n: Stoffmenge; p: Druck; T: Temperatur; t: Zeit; V: Volumen.

Physikalische Einheiten

°C: Grad Celsius; μl: Mikroliter; μm: Mikrometer; bar: bar; cm: Zentimeter; g: Gramm; h: Stunde; l: Liter; m: Meter; mg: Milligramm; ml: Milliliter; mol: mol; mmol: mmol; N: Normalität [mol/l]; min: Minute

Weitere Abkürzungen

Σ: Bilanz [%]; FID: Flammenionisationsdetektor; GC: Gaschromatographie; GC-MS: Gaschromatographie mit Massenspektrometrie-Kopplung; K: Kalibrierfaktor [mmol/(l Peakfläche)]; Kat: Katalysator; Me: Zweitmetall; rpm: Umdrehungen pro Minute; S: Selektivität [%]; SCILL: Solid catalyst with ionic liquid layer; SILP: Supported ionic liquid phase; X: Umsatz [%]; Y: Ausbeute [%]; AC: Aktivkohle; $AlO_x$: Aluminiumoxid

Tabelle 1: Ausbeuten bei der Umsetzung von Furfural an Pt-Trägerkatalysatoren nach Xu *et al.* [23]. Y(1-4) entspricht der Summe der Ausbeuten an 2-MF, 2-MTHF, 1-POH und 2-POH. Reaktionsbedingungen: T = 130 °C, $p(H_2)$ = 15 bar, t = 24 h.

| Katalysator | Y(1-4) [%] | Y(THFFOH) [%] | Y(1,2-PD) [%] | Y(1,4-PD) [%] | Y(1,5-PD) [%] |
|---|---|---|---|---|---|
| $Pt/Co_2AlO_4$ | 32.6 | 31.1 | 7.1 | 4.5 | 24.6 |
| $Pt/Co_3O_4$ | 20.1 | 21.8 | 14.8 | 2.8 | 30.8 |
| $Pt/Al_2O_3$ | 10.3 | 11.9 | 1.8 | 0.6 | 3.2 |

Tabelle 2: Ausbeuten bei der Umsetzung von Furfural an $Pt/Co_2AlO_4$ bei unterschiedlichen Reaktionstemperaturen nach Xu *et al.* [23]. Y(1-4) entspricht der Summe der Ausbeuten an 2-MF, 2-MTHF, 1- POH und 2-POH. Reaktionsbedingungen: $p(H_2)$ = 15 bar, t = 24 h.

| T [°C] | Y(1-4) [%] | Y(THFFOH) [%] | Y(1,2-PD) [%] | Y(1,4-PD) [%] | Y(1,5-PD) [%] |
|---|---|---|---|---|---|
| 110 | 25.5 | 26.8 | 10.3 | 1.0 | 30.6 |
| 120 | 27.7 | 24.4 | 10.4 | 2.0 | 30.3 |
| 130 | 32.6 | 31.1 | 7.1 | 4.5 | 24.6 |

Die Reaktionspfade bei der Hydrogenolyse von Furfural an Platinkatalysatoren nach Xu *et al.* [11] sind im Folgenden Schema 1 dargestellt. Dabei ist 2-Methylfuran (2-MF) **1,** 2-Methyltetrahydrofuran **2,** 1-Pentanol **3,** 2-Pentanol **4,** Tetrahydrofurfurylalkohol **5,** 1,2 Pentandiol **6,** 1,4-Pentandiol (1,4-PD) **7** und 1,5-Pentandiol **8.**

Schema 1: Reaktionspfade bei der Hydrogenolyse von Furfural an Platinkatalysatoren nach Xu *et al.* [11]

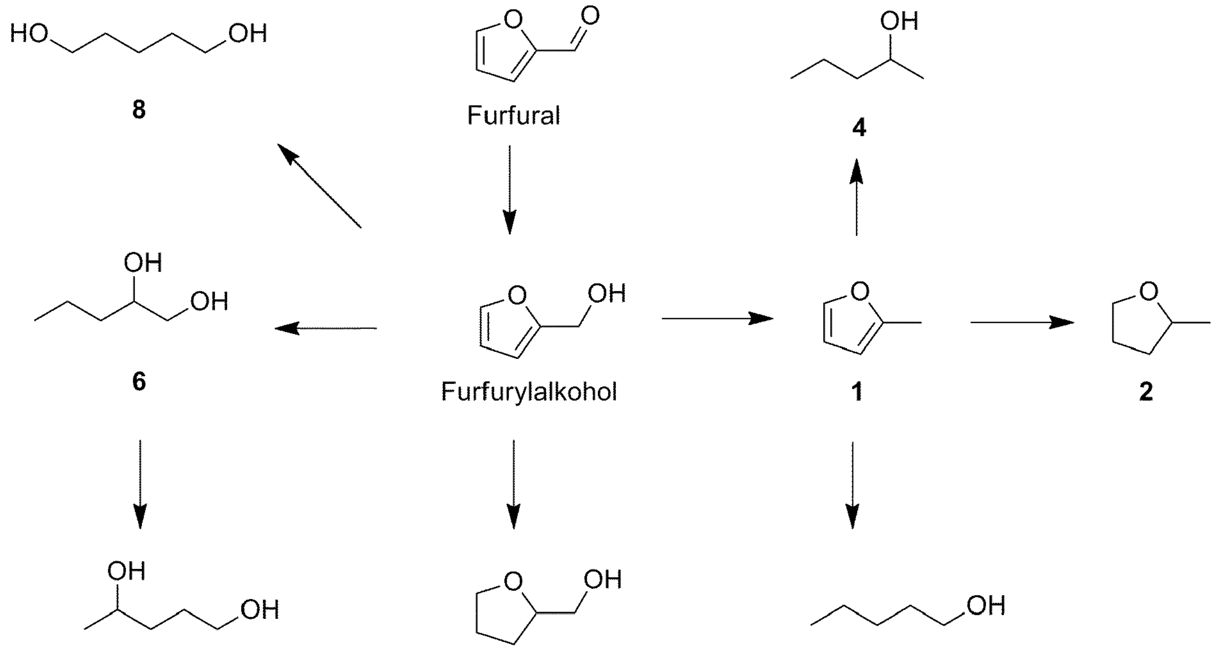

8

Furfural

4

6

Furfurylalkohol

1

2

7

5

3

Die Hydrogenolyse von Furfurylalkohol ist außerdem auch an heterogenen Kupferkatalysatoren beschrieben. Bereits 1931 hat Adkins [24] Furfurylalkohol an einem durch Fällung aus basischer Lösung von Ammoniumdichromat und Kupfernitrat präparierten Kupferchromit-Katalysator umgesetzt. Bei einer Reaktionstemperatur von 175 °C und 100 - 150 bar Wasserstoffdruck entstand als Hauptprodukt mit 40 % Ausbeute 1,2-Pentandiol **6.** Außerdem traten als Nebenprodukte 1,5-Pentandiol **8** (Y = 30 %), Pentanol (Y = 10 %), sowie Tetrahydrofurfurylalkohol **5** und 2-Methyltetrahydrofuran **2** mit unbekannten Ausbeuten auf.

Manly [25] erreichte mit Kupferchromit-Katalysatoren bis zu 4.4 % Ausbeute von 1,2-Pentandiol **6** bei einer Reaktionstemperatur von 175 °C, wobei als Hauptprodukt mit 66.4 % Ausbeute 2-Methylfuran **1** entstand. Als Nebenprodukte traten außerdem 2-Methyltetrahydrofuran **2** und 2-Pentanol **4** auf. 1,5-Pentandiol **8** wurde nicht gebildet. Die Entstehung von 1,2-Pentandiol **6** führte Manly auf die Hydrogenolyse von Furfurylalkohol zurück, der als Zwischenprodukt durch Hydrierung von Furfural entstand. Eine Hydrogenolyse von Tetrahydrofurfurylalkohol zu 1,2-Pentandiol **5** fand unter diesen Reaktionsbedingungen nicht statt. Basierend auf diesen Beobachtungen stellte Manly das folgende Reaktionsschema auf, das alle nachgewiesenen Produkte umfasst.

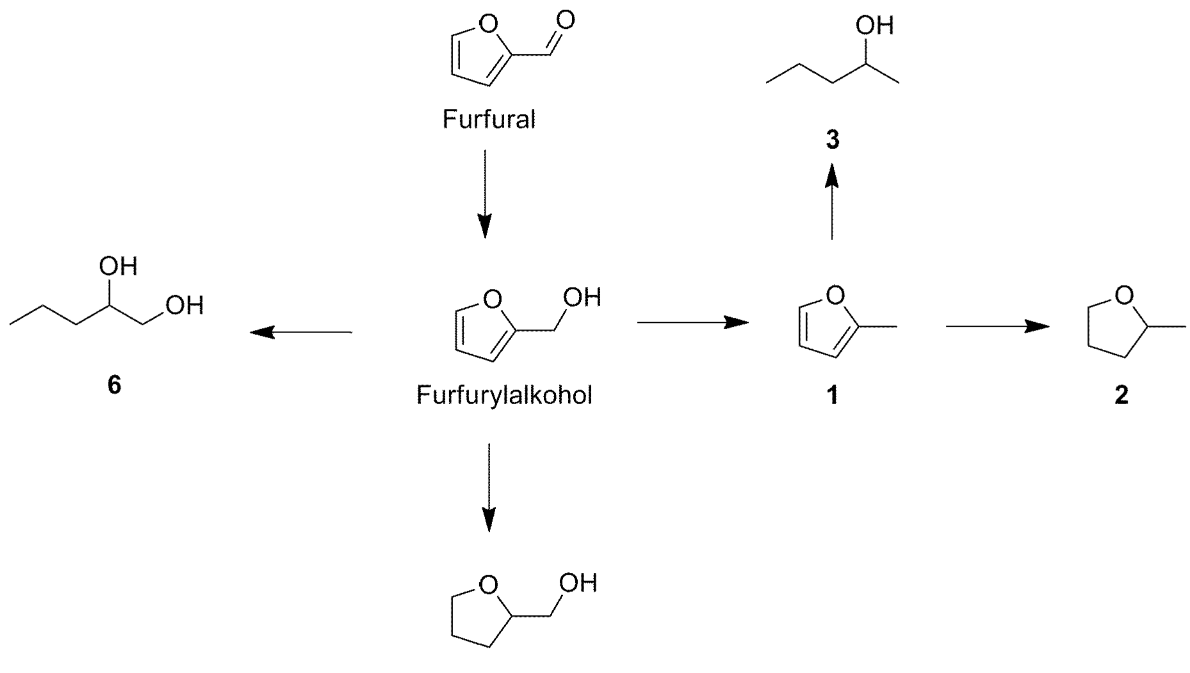

Furfural

3

6

Furfurylalkohol

1

2

5

Der Einsatz von Kupferoxiden zur katalytischen Hydrogenolyse von Furfurylalkohol wurde auch von Hachihama *et al.* beschrieben. [52]

Auch Nickelkatalysatoren fanden Verwendung, um Furfural und Furfurylalkohol mit sehr hoher Selektivität von bis zu 100 % zu Tetrahydrofurfurylalkohol zu hydrieren. Dabei kamen vor allem Nickel-Trägerkatalysatoren, Legierungskatalysatoren und Raney-Nickel zum Einsatz. [20, 26, 27, 28] Typische Reaktionsbedingungen sind Temperaturen von 100 - 180 °C und Wasserstoffdrücke zwischen 20 und 200 bar. Nach Leuck *et al.* kann Furfurylalkohol in saurer wässriger Lösung an Raney-Nickel bei 160 °C und 68 bar zu einem Gemisch aus 1,2- Pentandiol **6,** 1,4-Pentandiol **7,** 1,5-Pentandiol **8,** 1,2,4-Pentantriol und Tetrahydrofurfurylalkohol umgesetzt werden, wobei der Gesamtanteil der Diole im Produktgemisch 40 % beträgt. [29]

Nickelkatalysatoren sind bei der Herstellung von 1,2-Alkandiolen ebenfalls beschrieben, etwa von Wang *et al.* [50] Den Einsatz von Nickelkatalysatoren bei der Herstellung von Tetrahydrofurfurylalkohol beschreiben Zhao *et al.* [51] Den Einsatz von Nickel-AluminiumLegierungen zur Hydrogenolyse von Furanderivaten beschreiben Papa *et al.* in [53]. Kupfer-Chrom-basierte Katalysatoren beschreiben Connor & Adkins. [54]

Schließlich ist im Stand der Technik auch die Hydrierung von Furfural und Furfurylalkohol zu Tetrahydrofurfurylalkohol an unterschiedlichen Rutheniumkatalysatoren beschrieben. [26, 30, 31, 32] Die Reaktion wird in der Regel in Methanol unter milden Reaktionsbedingungen durchgeführt. Bei 40 °C Reaktionstemperatur und 20 bar Wasserstoffdruck wurde mit Ruthenium auf einem Hektoritträger eine Selektivität zu Tetrahydrofurfurylalkohol von über 99 % erreicht. [30] Mit $RuO_2$ wurden bei 120 °C und 50 bar undefinierte, hochmolekulare Nebenprodukte beobachtet. [26] Die Anwendung von Rutheniumkatalysatoren zur Umsetzung von Furfurylalkohol zu 1,2-Pentandiol ist auch von Zhang *et al.* beschrieben [49]. Hier werden verschiedene Rutheniumkatalysatoren eingesetzt, die allerdings auf Trägermaterialien geträgert sind, deren Handhabung schwierig ist und die insbesondere für eine großtechnische Anwendung ungeeignet sind. Zhang *et al.* beschreiben demnach ein Verfahren, welches schwerer handhabbare Träger wie $MnO_2$ oder $AlMgO_4$ einsetzen, die gerade bei großtechnischen Anwendungen Probleme bereiten [49]. Zusätzlich dazu ist $MnO_2$ im Vergleich zu Aluminiumoxid, insbesondere $Al_2O_3$, für großtechnische Verfahren unattraktiv als Trägermaterial. $Al_2O_3$ ist insbesondere aufgrund seiner elektrischen Isolation, seiner mechanischen Festigkeit, seiner hohen Druckfestigkeit, seiner hohen Härte, seiner mittleren Wärmeleitfähigkeit, seiner hohen Korrosions- und Verschleißbeständigkeit, seiner guten Gleiteigenschaften, seiner geringen Dichte sowie der Möglichkeit, es auch bei hohen Temperaturen einzusetzen, ein sehr attraktives Trägermaterial.

Ein anderer Weg zur Synthese von 1,2-Pentandiol ist die Hydrogenolyse von Tetrahydrofurfurylalkohol, welcher durch Hydrierung aus Furfural oder Furfurylalkohol gewonnen werden kann. [20, 26, 27, 28, 30, 31, 32] In der Literatur ist Rhodium das Aktivmetall der Wahl, um Tetrahydrofurfurylalkohol unter moderaten Reaktionsbedingungen zu hydrolysieren. [58] Tomishige *et al.* konnten unter Verwendung von $Rh\text{-}ReO_x/SiO_2$ Katalysatoren 1,5-Pentandiol mit bis zu 77 % Ausbeute erzeugen, ohne dass dabei 1,2-Pentandiol gebildet wurde (Tabelle 3). [33] Die Reaktion wurde in wässriger Lösung bei 120 °C und 80 bar Wasserstoffdruck durchgeführt. Reine Rhodium- oder Rhenium-Trägerkatalysatoren lieferten unter diesen Reaktionsbedingungen nur geringe Umsätze und bildeten bevorzugt 1,2-Pentandiol. So wurde mit $Rh/SiO_2$ bei 5.7 % Umsatz eine Selektivität von 61.7 % zu 1,2-Pentandiol erreicht. Als Nebenprodukte entstanden vor allem 1,5-Pentandiol (S = 18.0 %) und 1-Pentanol (S = 6.2 %). Unter Verwendung von $ReO_x/SiO_2$ betrug die Selektivität zu 1,2-Pentandiol 31.2 % und es entstand kein 1,5-Pentandiol als Nebenprodukt, wobei der Umsatz von Tetrahydrofurfurylalkohol mit weniger als 0.1 % extrem gering war. Auch andere Aktivmetalle wie Ruthenium, Kupfer und Nickel zeigen äußerst geringe Aktivität. Verantwortlich für die vergleichsweise hohe Aktivität von $Rh-ReO_x/SiO_2$ und die hohe Selektivität zu 1,5-Pentandiol ist nach Tomishige die Bildung von $ReO_x$-Clustern auf der Oberfläche der Rh-Partikel. Die $-CH_2OH$-Gruppe wird an diesen Clustern adsorbiert, was den Angriff der an der $Rh-ReO_x$-Grenzfläche gebildeten Hydrid-Ionen auf die benachbarte C-O-Bindung ermöglicht. [34]

Tabelle 3: Umsätze und Selektivitäten bei der Umsetzung von THFFOH an unterschiedlichen Katalysatoren nach Tomishige *et al.* [33]. Reaktionsbedingungen: $p(H_2)$ = 80 bar, 5 % THFFOH in Wasser.

| **Katalysator** | **T [°C]** | **t [h]** | **X [%]** | **S (1,5-PD) [%]** | **S (1,2-PD) [%]** | **S (1-POH) [%]** | **S (sonstige) [%]** |
|---|---|---|---|---|---|---|---|
| $Rh-ReO_x/ SiO_2$ | 120 | 24 | 96.2 | 80.1 | 0.0 | 15.9 | 4.0 |
| $Rh/SiO_2$ | 120 | 4 | 5.7 | 18.0 | 61.7 | 6.2 | 14.1 |
| $ReOx/ SiO_2$ | 120 | 4 | < 0.1 | 0.0 | 31.2 | 5.1 | 63.7 |
| Ru/C | 120 | 4 | 5.0 | 15.1 | 26.1 | 7.8 | 51.0 |
| Kupferchromit | 180 | 4 | 0.6 | 0.0 | 9.4 | 3.0 | 87.6 |
| Raney-Ni | 180 | 4 | 0.2 | 13.1 | 12.7 | 8.9 | 65.2 |

Suzuki *et al.* hydrolysierten Tetrahydrofurfurylalkohol an Rh- und Pd-Katalysatoren unter milden Reaktionsbedingungen, indem sie überkritisches $CO_2$ als Lösungsmittel verwendeten. [35] Unter Verwendung von Rhodium auf dem mesoporösen $SiO_2$-Träger MCM-41 wurde Tetrahydrofurfurylalkohol bei 80 °C und 40 bar Wasserstoffdruck mit 91.2 % Selektivität zu 1,5-Pentandiol umgesetzt, wobei als einziges Nebenprodukt 1-Pentanol entstand. Verantwortlich für die hohe Aktivität und Selektivität des Katalysators ist nach Suzuki *et al.* die Anwesenheit von $Rh_2O_3$-Partikeln in Rh/MCM-41. Wurde der Katalysator vor der Reaktion bei 300 °C reduziert, so dass lediglich Rh (0) vorlag, nahm die Aktivität deutlich ab und die Selektivität veränderte sich zu Gunsten von 1,2-Pentandiol (S = 82.5 %). Weitere Rh- und Pd-Katalysatoren lieferten unter gleichen Reaktionsbedingungen Produktgemische von 1,2-Pentandiol, 1,5-Pentandiol, 1-Pentanol, 2-Pentanol und anderen Nebenprodukten (Tabelle 4). Die beste Ausbeute an 1,2-Pentandiol lieferte Pd/MCM-41 mit 39.1 %.

Tabelle 4: Umsätze und Selektivitäten bei der Umsetzung von THFFOH an Rh- und Pd-Katalysatoren in überkritischem $CO_2$ nach Suzuki *et al.* [35]. Reaktionsbedingungen: T = 80 °C, $p(H_2)$ = 40 bar, [a] Katalysator wurde vor der Reaktion bei 300 °C reduziert.

| **Katalysator** | **X [%]** | **S (1,5-PD) [%]** | **S (1,2-PD) [%]** | **S (1-POH) [%]** | **S(sonstige) [%]** |
|---|---|---|---|---|---|
| Rh/MCM-41 | 80.5 | 91.2 | 0.0 | 8.8 | 0.0 |
| Rh/MCM-41 [a] | 5.0 | 12.1 | 82.5 | 5.4 | 0.0 |
| $Rh/Al_2O_3$ | 60.0 | 21.9 | 51.4 | 19.5 | 7.2 |
| Rh/C | 34.8 | 29.8 | 42.3 | 20.0 | 7.9 |
| $Rh/SiO_2$ | 30.4 | 78.2 | 11.4 | 8.2 | 12.2 |
| Pd/MCM-41 | 50.5 | 12.6 | 77.4 | 10.0 | 0.0 |
| $Pd/Al_2O_3$ | 32.5 | 9.1 | 0.9 | 47.5 | 42.5 |
| Pd/C | 48.9 | 99.2 | 0.3 | 0.5 | 0.0 |

Die wichtigsten Literaturergebnisse zur Hydrogenolyse von Furfurylalkohol oder Tetrahydrofurfurylalkohol zu 1,2-Pentandiol sind in Tabelle 5 zusammengefasst. Die beste Ausbeute wurde mit Pt(IV)-Oxid in Essigsäure als Lösungsmittel erzielt, wobei über die Reaktionsbedingungen keine genauen Angaben vorliegen.

Tabelle 5: Zusammenfassung der Literaturergebnisse für die Hydrogenolyse von Furfurylalkohol bzw. THFFOH zu 1,2-Pentandiol.

| Katalysator | Edukt | Lösemittel | T [°C] | p(H2) [bar] | Y(1,2-PD) [%] |
|---|---|---|---|---|---|
| Pt(IV)-Oxid [18] | Furfural | Ethanol | 25 | 1 - 2 | 20 |
| Pt(IV)-Oxid [20] | FFOH | Ethanol | 25 | 1 | 13.3 |
| Pt(IV)-Oxid [22] | FFOH | Essigsäure | unbekannt | 1.4 - 4 | ca. 100 |
| $Pt/Co_3O_4$ [23] | Furfural | Keines | 130 | 15 | 14.8 |
| Raney-Ni [29] | FFOH | Wasser | 160 | 68 | unbekannt [a)] |
| Kupfer-chromit [24] | FFOH | Keines | 175 | 100-150 | 40 |
| $Rh/Sio_2$ [33] | THFFOH | Wasser | 120 | 80 | 3.5 |
| Pd/MCM-41 [35] | THFFOH | $CO_2$ | 80 | 40 | 39.1 |
| [a)] Die Gesamtausbeute von 1,2-Pentandiol, 1,4-Pentandiol und 1,5-Pentandiol beträgt 40 %. | | | | | |

Als ionische Flüssigkeiten (IL) werden im allgemeinen Salze bezeichnet, die einen Schmelzpunkt unterhalb von 100 °C aufweisen. Die erste 1914 in der Literatur beschriebene, bei Raumtemperatur flüssige IL war Ethylammoniumnitrat. [36] Ionische Flüssigkeiten haben vielversprechende Eigenschaften, die sie insbesondere für die grüne Chemie interessant machen. Sie zeichnen sich unter anderem durch große Stabilität, gute Lösungseigenschaften und ihren vernachlässigbaren Dampfdruck aus. [37] Trotz dieser Vorteile wurde das Interesse der Forschung an ihrem Einsatz als Lösungsmittel oder Katalysatoren erst in den 80er Jahren geweckt. [38, 39] Ein besonders aktuelles Forschungsgebiet ist die Immobilisierung von ionischen Flüssigkeiten an festen Oberflächen. Dabei unterscheidet man zwischen den beiden grundlegenden Konzepten SILP (supported ionic liquid phase) und SCILL (solid catalyst with ionic liquid layer). [40] Bei SILP handelt es sich um eine Variante der homogenen Katalyse, wobei der Katalysator in einer IL gelöst vorliegt, welche an einem festen Träger immobilisiert ist. Im Gegensatz dazu beschreibt SCILL die Beschichtung eines heterogenen Katalysators mit einem dünnen IL-Film. Diese IL-Schicht beeinflusst den Reaktionsverlauf durch die veränderten Löslichkeiten der Reaktanden im Vergleich zum Lösungsmittel. Zusätzlich können auch direkte Wechselwirkungen zwischen IL und Katalysator auftreten, zum Beispiel durch elektronische Ligandeneffekte ähnlich denen von Zweitmetallen [41, 42]. Die Erfinder der vorliegenden Erfindung konnten Selektivitäten von über 99 % bei der Hydrierung von Citral zu Citronellal an Pd-Katalysatoren erzielen, indem der Katalysator mit kleinen Mengen einer ionischen Flüssigkeit beschichtet wurde. Die besten Selektivitäten lieferten dabei ionische Flüssigkeiten, die das Dicyanamid-Anion (DCA) enthielten. [43, 44] Es konnte gezeigt werden, dass DCA an Pd koordiniert und dadurch die Wasserstoffadsorption schwächt, was die Weiterreaktion von Citronellal zu unerwünschten Folgeprodukten verhindert. [42]

Es war deshalb die Aufgabe der vorliegenden Erfindung, ein Verfahren zur Umsetzung von Furfurylalkohol bereitzustellen, welches im Vergleich zu den vergleichbaren Katalysatoren des Standes der Technik eine hohe Selektivität zu 1,2-Pentandiol aufweist und gleichzeitig mit Hilfe eines gut verarbeitbaren und breit verfügbaren Katalysators abläuft. Das Verfahren soll vorteilhafterweise zusätzlich zur hohen Ausbeute an 1,2-Pentandiol, auch zu einer möglichst hohen Gesamtausbeute an Tetrahydrofurfurylalkohol führen. Ein solches Verfahren wäre besonders vorteilhaft, da Tetrahydrofurfurylalkohol auch als Lösungsmittel eingesetzt werden kann und darum, neben 1,2-Pentandiol, ein wichtiges Produkt darstellt. Zusätzlich ist es wünschenswert, dass ein solches Verfahren vollständig abläuft, um zu gewährleisten, dass der giftige Ausgangsstoff Furfurylalkohol vollständig umgesetzt wird.

## Kurzbeschreibung der Erfindung

Es wurde nun überraschend gefunden, dass diese Aufgabe durch das erfindungsgemäße Verfahren gelöst wird. In diesem wird ein auf Aluminiumoxid, insbesondere $Al_2O_3$, oder Aktivkohle (AC) geträgerter Rutheniumkatalysator oder Platin (IV)-Oxid eingesetzt. Das erfindungsgemäße Verfahren führt überraschenderweise mit einer hohen Selektivität zum Produkt 1,2-Pentandiol und gleichzeitig mit hoher Selektivität zu Tetrahydrofurfurylalkohol. Weiterhin zeichnet sich das Verfahren dadurch aus, dass ein fast vollständiger Umsatz erzielt wird.

Die vorliegende Erfindung betrifft in einem ersten Aspekt ein

1.1 Verfahren zur Herstellung von 1,2-Pentandiol, dadurch gekennzeichnet, dass man Furfurylalkohol mit Wasserstoff in Gegenwart eines $Ru/AlO_x$-Katalysatorsystems umsetzt.

Im Sinne der Erfindung wird der Ausdruck "$Ru/AlO_x$-Katalysatorsystem" als Abkürzung für "ein Katalysatorsystem umfassend (I) Ruthenium und (II) einen Träger aus Aluminiumoxid, auf welchem das Ruthenium geträgert ist" verwendet.

1.2. Verfahren nach Punkt 1.1, dadurch gekennzeichnet, dass das Aluminiumoxid $Al_2O_3$ ist.

1.3. Verfahren nach Punkt 1.1 oder 1.2, dadurch gekennzeichnet, dass der Gehalt an Ruthenium 0.01 bis 30 Gew.-% bezogen auf das Gesamtgewicht des Ru/$AlO_x$-Katalysatorsystems beträgt.

1.4. Verfahren nach einem oder mehreren der Punkte 1.1 bis 1.3, dadurch gekennzeichnet, dass das Ru/$AlO_x$-Katalysatorsystem eine BET-Oberfläche von 50 bis 250 $m^2$/g Ru/$AlO_x$-Katalysatorsystem aufweist.

1.5. Verfahren nach einem oder mehreren der Punkte 1.1 bis 1.4, dadurch gekennzeichnet, dass das Ru/$AlO_x$-Katalysatorsystem ein durchschnittliches Porenvolumen von 0.2 bis 0.8 ml/g Ru/$AlO_x$-Katalysatorsystem aufweist.

1.6. Verfahren nach einem oder mehreren der Punkte 1.1 bis 1.5, dadurch gekennzeichnet, dass das Ru/$AlO_x$-Katalysatorsystem das folgende Röntgenbeugungsmuster aufweist:

| Pos. [°2Th.] | Relative Intensität [%] |
|---|---|
| 18.3 | 5 - 10 |
| 18.8 | 12 - 17 |
| 20.33 | 6 - 11 |
| 31.37 | 47 - 53 |
| 32.68 | 25 - 32 |
| 32.825 | 80 - 85 |
| 35.09 | 10 - 15 |
| 36.758 | 53 - 59 |
| 38.97 | 27 - 32 |
| 39.8 | 30 - 35 |
| 40.63 | 8 - 13 |
| 43.359 | 14 - 18 |
| 44.85 | 62 - 67 |
| 45.47 | 45 - 50 |
| 46.54 | 25 - 30 |
| 47.61 | 30 - 35 |
| 50.77 | 8 - 13 |
| 51.5 | 7 - 12 |
| 57.48 | 7 - 11 |
| 59.97 | 15 - 20 |
| 62.38 | 8 - 13 |
| 62.848 | 8 - 13 |
| 64.05 | 10 - 16 |
| 65.7 | 10 - 15 |
| 66.52 | 54 - 59 |
| 67 | 31 - 37 |
| 67.4 | 100 |
| 73.5 | 5 - 10 |

1.7. Verfahren nach einem oder mehreren der Punkte 1.1 bis 1.6, dadurch gekennzeichnet, dass man es bei einer Temperatur von 100 °C - 280 °C durchführt.

1.8. Verfahren nach einem oder mehreren der Punkte 1.1 bis 1.7, dadurch gekennzeichnet, dass man es unter autoklaven Bedingungen durchführt. Dabei bedeutet "autoklave Bedingungen" im Sinne der Erfindung ein Druck von gleich oder

mehr als 10 bar.

1.9. Verfahren nach einem oder mehreren der Punkte 1.1 bis 1.8, dadurch gekennzeichnet, dass man das Verfahren in einem Lösemittel ausgewählt aus der Gruppe bestehend aus Wasser, Ethanol, Tetrahydrofuran und 1,4 - Dioxan durchführt.

1.10. Verfahren nach einem oder mehreren der Punkte 1.1 bis 1.9, dadurch gekennzeichnet, dass man es bei einem pH-Wert von pH 5.3 bis pH 10.5 durchführt.

1.11. Verfahren nach einem oder mehreren der Punkte 1.1 bis 1.10, dadurch gekennzeichnet, dass man es kontinuierlich durchführt.

1.12. Verfahren nach einem oder mehreren der Punkte 1.1 bis 1.11, dadurch gekennzeichnet, dass man es im Batchbetrieb durchführt.

1.13. Verfahren nach einem oder mehreren der Punkte 1.1 bis 1.12, dadurch gekennzeichnet, dass das Ru/$AlO_x$-Katalysatorsystem erhalten wird durch folgende Schritte:

a) Herstellung einer Mischung (i) umfassend eine Rutheniumsalzlösung (ii) und eine wässrige Suspension von Aluminiumoxid (iii);

b) Einstellen der Mischung (i) auf eine Temperatur im Bereich von größer 0°C und kleiner 100 °C und einen pH-Wert von pH 0 - 14;

c) Abtrennen des Ru/$AlO_x$-Katalysatorsystems durch Filtration der Mischung (i);

d) Trocknen des in Schritt c) abgetrennten Ru/$AlO_x$-Katalysatorsystems.

1.14. Verfahren zur Herstellung eines Ru/$AlO_x$-Katalysatorsystems umfassend

a) Herstellung einer Mischung (i) umfassend eine Rutheniumsalzlösung (ii) und eine wässrige Suspension von Aluminiumoxid (iii);

b) Einstellen der Mischung (i) auf eine Temperatur im Bereich von größer 0 °C und kleiner 100°C und einen pH-Wert von pH 0 - 14;

c) Abtrennen des Ru/$AlO_x$-Katalysatorsystems durch Filtration der Mischung (i);

d) Trocknen des in Schritt c) abgetrennten Ru/$AlO_x$-Katalysatorsystems.

1.15 Verfahren nach Punkt 1.14, dadurch gekennzeichnet, dass vor Schritt b) oder zwischen Schritt b) und c), bevorzugt zwischen Schritt b) und c), ein Reduktionsmittel (iv) zu der Mischung (i) zugegeben wird.

1.16 Verfahren nach Punkten 1.14 oder 1.15, dadurch gekennzeichnet, dass in Schritt b) eine Temperatur im Bereich von 55 - 75 °C und ein pH-Wert von 7.5 - 8.5 durch Zugabe einer Base B2 zur Mischung (i) eingestellt wird.

1.17. Ru/$AlO_x$-Katalysatorsystem, was erhalten wird durch ein Verfahren gemäß einem oder mehrerer der Punkte 1.14 bis 1.16.

In einem zweiten Aspekt betrifft die vorliegende Erfindung ein

2.1. Verfahren zur Herstellung von 1,2-Pentandiol, dadurch gekennzeichnet, dass man Furfurylalkohol mit Wasserstoff in Gegenwart eines Ru/AC-Katalysatorsystems umsetzt.

Im Sinne der Erfindung wird der Ausdruck "Ru/AC-Katalysatorsystem" als Abkürzung für "ein Katalysatorsystem umfassend (I) Ruthenium und (II) einen Träger aus Aktivkohle, auf welchem das Ruthenium geträgert ist" verwendet.

2.2. Verfahren nach Punkt 2.1, dadurch gekennzeichnet, dass der Gehalt an Ruthenium 0.01 bis 30 Gew.-% bezogen auf das Gesamtgewicht des Ru/AC-Katalysatorsystems beträgt.

2.3. Verfahren nach Punkt 2.1 oder 2.2, dadurch gekennzeichnet, dass das Katalysatorsystem eine BET-Oberfläche von 300 bis 2000 $m^2$/g Ru/AC-Katalysatorsystem aufweist.

2.4. Verfahren nach einem oder mehreren der Punkte 2.1 bis 2.3, dadurch gekennzeichnet, dass das Ru/AC-Katalysatorsystem ein durchschnittliches Porenvolumen von 0.3 bis 2.0 ml/g Ru/AC-Katalysatorsystem aufweist.

2.5. Verfahren nach einem oder mehreren der Punkte 2.1 bis 2.4, dadurch gekennzeichnet, dass man es bei einer Temperatur von 100 °C - 280 °C durchführt.

2.6. Verfahren nach einem oder mehreren der Punkte 2.1 bis 2.5, dadurch gekennzeichnet, dass man es unter autoklaven Bedingungen durchführt. Dabei bedeutet der Begriff "autoklave Bedingungen" im Sinne der Erfindung ein Druck von gleich oder mehr als 10 bar.

2.7. Verfahren nach einem oder mehreren der Punkte 2.1 bis 2.6, dadurch gekennzeichnet, dass man das Verfahren in einem Lösemittel ausgewählt aus der Gruppe bestehend aus Wasser, Ethanol, Tetrahydrofuran und 1,4 - Dioxan durchführt.

2.8. Verfahren nach einem oder mehreren der Punkte 2.1 bis 2.7, dadurch gekennzeichnet, dass man es bei einem pH-Wert von pH 5.3 bis pH 10.5 durchführt.

2.9. Verfahren nach einem oder mehreren der Punkte 2.1 bis 2.8, dadurch gekennzeichnet, dass man es kontinuierlich durchführt.

2.10. Verfahren nach einem oder mehreren der Punkte 2.1 bis 2.9, dadurch gekennzeichnet, dass man es im Batchbetrieb durchführt.

2.11. Verfahren nach einem oder mehreren der Punkte 2.1 bis 2.10, dadurch gekennzeichnet, dass das Ru/AC-Katalysatorsystem erhalten wird durch folgende Schritte:

a) Herstellung einer Mischung (i) umfassend eine Rutheniumsalzlösung (ii) und eine wässrige Suspension von Aktivkohle (iii);

b) Einstellen der Mischung (i) auf eine Temperatur im Bereich von größer 0 °C und kleiner 100 °C und einen pH-Wert von pH 0 - 14;

c) Abtrennen des Ru/AC-Katalysatorsystems durch Filtration der Mischung (i).

2.12. Verfahren zur Herstellung eines Ru/AC-Katalysatorsystems umfassend

a) Herstellung einer Mischung (i) umfassend eine Rutheniumsalzlösung (ii) und eine wässrige Suspension von Aktivkohle (iii);

b) Einstellen der Mischung (i) auf eine Temperatur im Bereich von größer 0 °C und kleiner 100 °C und einen pH-Wert von pH 0 - 14;

c) Abtrennen des Ru/AC-Katalysatorsystems durch Filtration der Mischung (i).

2.13. Verfahren nach Punkt 2.12, dadurch gekennzeichnet, dass vor Schritt b) oder zwischen Schritt b) und c), bevorzugt zwischen Schritt b) und c), ein Reduktionsmittel (iv) zu der Mischung (i) zugegeben wird.

2.14. Verfahren nach Punkten 2.12 oder 2.13, dadurch gekennzeichnet, dass in Schritt b) eine Temperatur im Bereich von 55 - 75 °C und ein pH-Wert von 7.5 - 8.5 durch Zugabe einer Base B2 zur Mischung (i) eingestellt wird.

2.15. Ru/$AlO_x$-Katalysatorsystem, was erhalten wird durch ein Verfahren gemäß einem oder mehrerer der Punkte 2.12 bis 2.14.

In einem dritten Aspekt betrifft die vorliegende Erfindung ein

3.1. Verfahren zur Herstellung von 1,2-Pentandiol, dadurch gekennzeichnet, dass man Furfurylalkohol mit Wasserstoff in Gegenwart von Pt(IV)-Oxid umsetzt.

3.2. Verfahren nach Punkte 3.1, dadurch gekennzeichnet, dass man es bei einer Temperatur von größer 0 °C und kleiner 100 °C durchführt.

3.3. Verfahren nach Punkt 3.1 oder 3.2, dadurch gekennzeichnet, dass man es bei einem Druck von 1 - 10 bar durchführt.

3.4. Verfahren nach einem oder mehreren der Punkte 3.1 bis 3.3, dadurch gekennzeichnet, dass man das Verfahren in einem Lösemittel ausgewählt aus der Gruppe bestehend aus Wasser, Essigsäure, Ethanol durchführt.

3.5. Verfahren nach einem oder mehreren der Punkte 3.1 bis 3.4, dadurch gekennzeichnet, dass man das Verfahren unter Zusatz von Salzsäure durchführt.

## Detaillierte Beschreibung der Erfindung

Die vorliegende Erfindung betrifft in einem ersten Aspekt ein Verfahren zur Herstellung von 1,2-Pentandiol, welches dadurch gekennzeichnet ist, dass man Furfurylalkohol mit Wasserstoff in Gegenwart eines Katalysatorsystems umfassend

(I) Ruthenium und
(II) einen Träger aus Aluminiumoxid, auf welchem das Ruthenium geträgert ist,

umsetzt.

Im Sinne der Erfindung wird der Ausdruck "Ru/AlO$_x$-Katalysatorsystem" als Abkürzung für "ein Katalysatorsystem umfassend (I) Ruthenium und (II) einen Träger aus Aluminiumoxid, auf welchem das Ruthenium geträgert ist" verwendet.

Ein Vorteil des vorliegenden erfindungsgemäßen Verfahrens zur Herstellung von 1,2-Pentandiol gemäß des ersten Aspekts liegt darin, dass als Träger Aluminiumoxid genutzt wird. Dies ermöglicht es, den Katalysator auch großtechnisch herzustellen und einzusetzen, da sich der Aluminiumoxidträger, insbesondere $Al_2O_3$, hervorragend aus den entsprechenden Vorläuferverbindungen herstellen lässt, so zum Beispiel aus Bayerit und/oder Pseudo-Böhmit. Außerdem ist Aluminiumoxid gut verfügbar und zeichnet sich durch eine gute großtechnische Bearbeitbarkeit und Einsetzbarkeit aus. Diese Vorteile, welche durch Aluminiumoxide wie insbesondere $Al_2O_3$ gewährleistet werden, sind durch andere Trägermaterialien (etwa Manganoxide oder Spinelle wie z. B. $MgAlO_4$ bzw. $MgAl_2O_4$) nicht gewährleistet.

"Aluminiumoxid" im Sinne der vorliegenden Erfindung bedeutet dabei insbesondere $Al_2O_3$. $Al_2O_3$ kann dabei in jeder Modifikation eingesetzt werden. Bevorzugt wird $Al_2O_3$ in einer Modifikation ausgewählt aus theta, gamma oder einer Mischung daraus eingesetzt. Besonders bevorzugt handelt es sich bei "Aluminiumoxid" im Sinne der vorliegenden Erfindung um gamma- $Al_2O_3$. Die Herstellung des erfindungsgemäßen Aluminiumoxids ist dem Fachmann geläufig. Dies kann etwa aus Aluminiumoxidvorläuferverbindungen wie beispielsweise Gibbsit [$Al(OH)_3$], Bayerit [$Al(OH)_3$], Böhmit [AlO(OH)] und/oder Pseudo-Böhmit erfolgen. Diese Aluminiumoxidvorläufer werden mit einer Säure, beispielsweise Salpetersäure, behandelt und anschließend verformt. Die so erhaltenen Formkörper können dann getrocknet werden (dies kann etwa bei Temperaturen von 80 bis 140 °C erfolgen) und anschließend bei Temperaturen von 400 bis 700 °C calciniert werden.

Der Gehalt an Ruthenium im Ru/AlO$_x$-Katalysatorsystem liegt, bezogen auf die Gesamtgewicht des Ru/AlO$_x$-Katalysatorsystems, insbesondere im Bereich 0.01 - 30 Gew.-%, bevorzugt im Bereich 0.01 - 20 Gew.-%, besonders bevorzugt im Bereich 0.5 - 10.5 Gew.-%, ganz besonders bevorzugt im Bereich 5 - 10 Gew.-%, am bevorzugtesten bei 5 Gew.-%. Die Werte werden gemessen nach Trocknung des geträgerten Katalysators bei 80 °C im Vakuum bis zur Gewichtskonstanz. Der Gehalt an Ruthenium bezogen auf das Gesamtgewicht des Ru/AlO$_x$-Katalysatorsystems kann nach DIN51009 bestimmt werden.

Das Ru/AlO$_x$-Katalysatorsystem ist vorzugsweise mesoporös und hat einen Porendurchmesser, der im Bereich von 2 nm bis 50 nm liegt, bestimmt gemäß DIN ISO 9277.

Die BET-Gesamtoberfläche des Ru/AlO$_x$-Katalysatorsystems kann zwischen 50 und 250 $m^2/g$ Ru/AlO$_x$-Katalysatorsystem, insbesondere zwischen 100 und 150 $m^2/g$ Ru/AlO$_x$-Katalysatorsystem liegen. Die BET-Gesamtoberfläche wird dabei nach DIN ISO 9277 bestimmt.

Das Porenvolumen des Ru/AlO$_x$-Katalysatorsystems beträgt 0.2 bis 0.8 ml/g Ru/AlO$_x$-Katalysatorsystem, bevorzugt 0.3 bis 0.6 ml/g Ru/AlO$_x$-Katalysatorsystem, besonders bevorzugt 0.4 ml/g Ru/AlO$_x$-Katalysatorsystem. Das Porenvolumen wird dabei nach DIN ISO 9277 bestimmt.

Das Ru/AlO$_x$-Katalysatorsystem weist bevorzugt das folgende Röntgenbeugungsmuster auf.

| Pos. [°2Th.] | Relative Intensität [%] |
|---|---|
| 18.3 | 5 - 10 |

(fortgesetzt)

| Pos. [°2Th.] | Relative Intensität [%] |
|---|---|
| 18.8 | 12 - 17 |
| 20.33 | 6 - 11 |
| 31.37 | 47 - 53 |
| 32.68 | 25 - 32 |
| 32.825 | 80 - 85 |
| 35.09 | 10 - 15 |
| 36.758 | 53 - 59 |
| 38.97 | 27 - 32 |
| 39.8 | 30 - 35 |
| 40.63 | 8 - 13 |
| 43.359 | 14 - 18 |
| 44.85 | 62 - 67 |
| 45.47 | 45 - 50 |
| 46.54 | 25 - 30 |
| 47.61 | 30 - 35 |
| 50.77 | 8 - 13 |
| 51.5 | 7 - 12 |
| 57.48 | 7 - 11 |
| 59.97 | 15 - 20 |
| 62.38 | 8 - 13 |
| 62.848 | 8 - 13 |
| 64.05 | 10 - 16 |
| 65.7 | 10 - 15 |
| 66.52 | 54 - 59 |
| 67 | 31 - 37 |
| 67.4 | 100 |
| 73.5 | 5 - 10 |

Das Ru/$AlO_x$-Katalysatorsystem weist noch bevorzugter das folgende Röntgenbeugungsmuster auf.

| Pos. [°2Th.] | Relative Intensität [%] |
|---|---|
| 18.3 | 8.69 |
| 18.8 | 13.92 |
| 20.33 | 8.8 |
| 31.37 | 51.95 |
| 32.68 | 28.16 |
| 32.825 | 82.43 |
| 35.09 | 12.34 |

(fortgesetzt)

| Pos. [°2Th.] | Relative Intensität [%] |
|---|---|
| 36.758 | 56.2 |
| 38.97 | 30.89 |
| 39.8 | 32.12 |
| 40.63 | 10.43 |
| 43.359 | 16.21 |
| 44.85 | 64.42 |
| 45.47 | 47.23 |
| 46.54 | 28.33 |
| 47.61 | 32.26 |
| 50.77 | 11.05 |
| 51.5 | 9.98 |
| 57.48 | 8.68 |
| 59.97 | 17.46 |
| 62.38 | 11.05 |
| 62.848 | 10.72 |
| 64.05 | 13.91 |
| 65.7 | 12.04 |
| 66.52 | 56.88 |
| 67 | 34.05 |
| 67.4 | 100 |
| 73.5 | 6.88 |

Die Korngrößenverteilung des Ru/$AlO_x$-Katalysatorsystems liegt typischerweise bei d10 = 2 - 8 $\mu$m; d50 = 20 - 40 $\mu$m; d90 = 50 - 80 $\mu$m.

Dabei bedeutet "d10 = 2 - 8 $\mu$m", dass 10 % des Ru/$AlO_x$-Katalysatorsystems eine Körngröße von 2 - 8 $\mu$m oder kleiner aufweist.
Dabei bedeutet "d50 = 20 - 40 $\mu$m", dass 50 % des Ru/$AlO_x$-Katalysatorsystems eine Körngröße von 20 - 40 $\mu$m oder kleiner aufweist.
Dabei bedeutet "d90 = 50 - 80 $\mu$m", dass 90 % des Ru/$AlO_x$-Katalysatorsystems eine Körngröße von 50 - 80 $\mu$m oder kleiner aufweist.

Das erfindungsgemäße Verfahren gemäß des ersten Aspekts der Erfindung läuft üblicherweise wie folgt ab:

Im erfindungsgemäßen Verfahren zur Herstellung von 1,2-Pentandiol gemäß des ersten Aspekts der Erfindung ist die Temperatur grundsätzlich nicht beschränkt, so kann beispielsweise eine Temperatur von größer oder gleich 100 °C eingestellt werden. Bevorzugt wird das erfindungsgemäße Verfahren zur Herstellung von 1,2-Pentandiol gemäß des ersten Aspekts der Erfindung bei einer Temperatur von 100 °C bis 280 °C, besonders bevorzugt bei einer Temperatur von 150 bis 260°C durchgeführt. Als ganz besonders vorteilhaft hat es sich erwiesen, und ganz besonders bevorzugt ist es deshalb, das erfindungsgemäße Verfahren zur Herstellung von 1,2-Pentandiol gemäß des ersten Aspekts der Erfindung bei einer Temperatur von 200 °C bis 240 °C durchzuführen.

Das erfindungsgemäße Verfahren zur Herstellung von 1,2-Pentandiol gemäß des ersten Aspekts der Erfindung wird bevorzugt unter autoklaven Bedingungen durchgeführt. "Autoklave Bedingungen" beschreiben im Sinne der Erfindung Drücke von gleich oder mehr als 10 bar, bevorzugt Drücke im Bereich von 10 bis 200 bar, besonders bevorzugt Drücke im Bereich von 50 bis 150 bar, am bevorzugtesten einen Druck von 100 bar.

Das erfindungsgemäße Verfahren zur Herstellung von 1,2-Pentandiol gemäß des ersten Aspekts der vorliegenden Erfindung kann in jedem beliebigen Lösemittel durchgeführt werden. Insbesondere werden in diesem Zusammenhang Lösemittel ausgewählt aus der Gruppe bestehend aus Wasser, Ethanol, Tetrahydrofuran, 1,4-Dioxan eingesetzt. Besonders bevorzugt wird als Lösemittel Wasser eingesetzt.

Das erfindungsgemäße Verfahren gemäß des ersten Aspekts der vorliegenden Erfindung kann im Batchbetrieb, aber auch kontinuierlich durchgeführt werden.

Im erfindungsgemäßen Verfahren zur Herstellung von 1,2-Pentandiol gemäß des ersten Aspekts der vorliegenden Erfindung kann als Wasserstoff jedes Gas verwendet werden, welches freien Wasserstoff aufweist und frei von Zusätzen ist, die das Verfahren unterbinden könnten, wie zum Beispiel Kohlenmonoxid.

Die Menge an im erfindungsgemäßen Verfahren zur Herstellung von 1,2-Pentandiol gemäß des ersten Aspekts der vorliegenden Erfindung eingesetzten Ru/$AlO_x$-Katalysatorsystems ist nicht besonders beschränkt und liegt insbesondere im Bereich von 0.01 - 30 Gew.-%, bevorzugt 0.5 - 30 Gew.-%, besonders bevorzugt 1 - 20 Gew.-%, ganz besonders bevorzugt 1 - 10 Gew-% jeweils bezogen auf das Gewicht des eingesetzten Furfurylalkohols.

Das Ru/$AlO_x$-Katalysatorsystem kann neben Ruthenium weitere Metalle ausgewählt aus der Gruppe bestehend aus Zink, Nickel, Platin und Eisen, bevorzugt Zink und Eisen, besonders bevorzugt Eisen umfassen.
Es ist jedoch bevorzugt, dass das Ru/$AlO_x$-Katalysatorsystem neben Ruthenium keine weiteren Metalle aufweist. "Kein weiteres Metall" bedeutet im Sinne der Erfindung, dass der Anteil eventuell weiterer neben Ruthenium auf dem Träger aus Aluminiumoxid geträgerter Metalle berechnet auf die Trockenmenge an Katalysatorsystem zwischen 0 Gew.-% und 5 Gew.% liegt, bevorzugt zwischen 0 Gew.-% und 2.5 Gew.-%, bevorzugter zwischen 0 Gew.-% und 1 Gew.-%, besonders bevorzugt zwischen 0 Gew.-% und 0.1 Gew.-%, ganz besonders bevorzugt zwischen 0 Gew.-% und 0.01 Gew.-%, am bevorzugtesten zwischen 0 Gew.-% und 0.001 Gew.-% liegt (mittels der Methode nach DIN51009 bestimmbar).

Überraschenderweise wurde außerdem gefunden, dass sich besonders gute Selektivitäten zum 1,2-Pentandiol ergeben, wenn das erfindungsgemäße Verfahren zur Herstellung von 1,2-Pentandiol gemäß des ersten Aspekts der vorliegenden Erfindung bei einem pH-Wert von 5.3 bis 10.3, bevorzugter von 6.2 bis 10.2, am bevorzugtesten bei einem pH-Wert von 7.6 durchgeführt wird. Dies kann zum Beispiel dadurch erreicht werden, in dem vor der Reaktion eine Base, beispielsweise $Na_2CO_3$, zugegeben wird. Die Menge der zum Erreichen des jeweiligen pH-Wertes notwendigen Base kann vom Fachmann routinemäßig bestimmt werden.

Der Ausdruck "das erfindungsgemäße Verfahren zur Herstellung von 1,2-Pentandiol gemäß des ersten Aspekts der vorliegenden Erfindung wird bei einem pH-Wert von 5.3 bis 10.3, bevorzugter von 6.2 bis 10.2, am bevorzugtesten bei einem pH-Wert von 7.6 durchgeführt" bedeutet im Falle dass, wenn das erfindungsgemäße Verfahren zur Herstellung von 1,2-Pentandiol gemäß des ersten Aspekts der vorliegenden Erfindung unter autoklaven Bedingungen durchgeführt wird, dass vor der Reaktion soviel Base B1 zugegeben wird, dass der pH-Wert von 5.3 bis 10.3, bevorzugter von 6.2 bis 10.2, am bevorzugtesten von 7.6 direkt nach Beendigung der Reaktion in der Reaktionslösung gemessen werden kann. "Direkt nach Beendigung der Reaktion" bedeutet im Sinne der Erfindung der Zeitpunkt, zu welchem sich der Anteil an 1,2-Pentandiol nicht mehr ändert.

Als Base B1 eignet sich grundsätzlich jede alkalisch reagierende Substanz, mit der der gewünschte pH eingestellt werden kann, insbesondere ist die Base ausgewählt aus der Gruppe bestehend aus Alkalimetallacetate, Erdalkalimetallacetate, Alkalimetallcarbonate, Erdalkalimetallcarbonate, Alkalimetallhydrogencarbonate, Erdalkalimetallhydrogencarbonate, Alkalimetallhydroxide, Erdalkalimetallhydroxide, bevorzugt ist die Base ausgewählt aus der Gruppe bestehend aus Alkalimetallacetate, Erdalkalimetallacetate, Alkalimetallcarbonate, Erdalkalimetallcarbonate, noch bevorzugter ist die Base ausgewählt aus der Gruppe bestehend aus Alkalimetallacetate, Alkalimetallcarbonate, besonders bevorzugt ist die Base ausgewählt aus der Gruppe bestehend aus Natriumcarbonat und Natriumacetat, und am bevorzugtesten ist die Base Natriumcarbonat.

Das erfindungsgemäße Verfahren zur Herstellung von 1,2-Pentandiol gemäß des ersten Aspekts der vorliegenden Erfindung kann auch unter Zusatz weiterer organischer Verbindungen durchgeführt werden, wobei die organischen Verbindungen ausgewählt sind aus der Gruppe bestehend aus Natriumdicyanamid, 1-Butyl-3-methylimidazoliumdicyanamid, N-Butyl-3-methylpyridinium-dicyanamid, 1-Butyl-1-methylpyrrolidiniumdicyanamid, bevorzugt 1-Butyl-3-methylimidazolium-dicyanamid. Auch unter Zusatz solcher organischer Verbindungen zeigt das erfindungsgemäße Verfahren gemäß des ersten Aspekts der Erfindung immer noch überraschend gute Ausbeuten. Es hat sich jedoch gezeigt, dass das erfindungsgemäße Verfahren gemäß des ersten Aspekts der Erfindung die besten Ausbeuten liefert, wenn kein solcher Zusatz erfolgt. Das erfindungsgemäße Verfahren gemäß des ersten Aspekts der vorliegenden Erfindung wird bevorzugt deshalb ohne den Zusatz von organischen Verbindungen ausgewählt sind aus der Gruppe bestehend aus Natriumdicyanamid, 1-Butyl-3-methylimidazolium-dicyanamid, N-Butyl-3-methylpyridinium-dicyanamid, 1-Butyl-1-methylpyrrolidinium-dicyanamid durchgeführt.

Die Durchmischung der Reaktanden (Furfurylalkohol, $H_2$), die etwa durch eine gewisse Rührgeschwindigkeit erreicht werden kann, kann in jeder dem Fachmann geläufigen Weise erfolgen.

Das erfindungsgemäße Verfahren ist insbesondere für die Umsetzung von Furfurylalkohol zu 1,2-Pentandiol geeignet.

Das Ru/$AlO_x$-Katalysatorsystem wird durch Trägern des Rutheniums auf dem Aluminiumoxidträger hergestellt. Das Ruthenium kann auf den Aluminiumoxidträger durch Imprägnierung, Beschichtung, Abscheidung durch Kopräzipitation oder andere geeignete Prozesse wie spray deposition geträgert werden. Bevorzugt wird das Ru/$AlO_x$-Katalysa-

torsystem hergestellt, in dem der Aluminiumoxidträger mit Ruthenium imprägniert wird. Dies geschieht, in dem der Aluminiumoxidträger mit einer Rutheniumsalzlösung in Kontakt gebracht wird und das Ruthenium auf dem Aluminiumoxidträger durch Besprühen oder mittels pH-gesteuerter Kopräzipitation abgeschieden wird.

In einer bevorzugten Vorgehensweise wird das Ru/$AlO_x$-Katalysatorsystem durch ein Verfahren zur Herstellung eines Ru/$AlO_x$-Katalysatorsystems erhalten.

Das Verfahren zur Herstellung eines Ru/$AlO_x$-Katalysatorsystems umfasst

a) Herstellung einer Mischung (i) umfassend eine Rutheniumsalzlösung (ii) und eine wässrige Suspension von Aluminiumoxid (iii);

b) Einstellen der Mischung (i) auf eine Temperatur im Bereich von größer 0°C und kleiner 100°C und einen pH-Wert von pH 0 - 14;

c) Abtrennen des Ru/$AlO_x$-Katalysatorsystems durch Filtration der Mischung (i);

d) Trocknen des in Schritt c) abgetrennten Ru/$AlO_x$-Katalysatorsystems.

Die vorliegende Erfindung betrifft auch ein Ru/$AlO_x$-Katalysatorsystem erhältlich nach dem Verfahren zur Herstellung eines Ru/$AlO_x$-Katalysatorsystems.

Im Schritt a) des Verfahren zur Herstellung eines Ru/$AlO_x$-Katalysatorsystems wird zunächst eine Mischung (i) umfassend eine Rutheniumsalzlösung (ii) und eine wässriger Suspension von Aluminiumoxid (iii) hergestellt. Dies kann durch jede dem Fachmann geläufige Art und Weise geschehen, geschieht typischerweise jedoch, in dem eine Rutheniumsalzlösung (ii) und eine wässrige Suspension von Aluminiumoxid (iii) gemischt werden.

Die Rutheniumsalzlösung (i) im Sinne der Erfindung ist jede rutheniumhaltige, bevorzugt wässrige, Lösung eines Rutheniumsalzes. Das Rutheniumsalz im Sinne der Erfindung wird insbesondere ausgewählt aus der Gruppe bestehend aus Rutheniumcarbonat [$Ru(CO_3)_3$]; Rutheniumcarboxylate wie z.B. Ruthenium (II, III) $\mu$-oxoacetat [$(CH_3CO_2)_7Ru_3O$-$3H_2O$]; Rutheniumcarbonyle; Rutheniumhalide wie z.B. Rutheniumbromid ($RuBr_3$), Rutheniumchlorid ($RuCl_3$), Rutheniumchloridhydrat ($RuCl_3$-$xH_2O$), Rutheniumiodid ($RuI_3$); Rutheniumnitrate wie z.B. $Ru(NO_3)_3$-$xH_2O$; Rutheniumoxide wie z. B. $RuO_2$ und Ruthenium(IV)oxidhydrat ($RuO_2$-$xH_2O$); Rutheniumnitrosylnitrate wie z. B. Rutheniumnitrosylnitrat [$Ru(NO)(NO_3)_x(OH)_y$, wobei x = 1, 2, 3; y = 0, 1, 2; und x+y = 3]; Rutheniumchlorkomplexe; Rutheniumaminkomplexe, Rutheniumnitritkomplexe. In einer bevorzugten Ausführungsform ist das Rutheniumsalz Rutheniumnitrosylnitrat [$Ru(NO)(NO_3)_x(OH)_y$, wobei x = 1, 2, 3; y = 0, 1, 2; und x+y = 3], besonders bevorzugt ist Rutheniumnitrosylnitrat $Ru(NO)(NO_3)_3$.

Als wässrige Suspension von Aluminiumoxid (iii) eignet sich jede Suspension, die Aluminiumoxid, bevorzugt $Al_2O_3$, noch bevorzugter $\gamma$-$Al_2O_3$, aufweist.

In Schritt b) des Verfahrens zur Herstellung eines Ru/$AlO_x$-Katalysatorsystems wird die Mischung (i) auf eine Temperatur im Bereich von größer 0 °C und kleiner 100 °C und einen pH-Wert von pH 0 - 14 eingestellt.
Dies umfasst jegliche Vorgehensweise, durch welche eine Mischung (i) umfassend eine Rutheniumsalzlösung und eine wässriger Suspension von Aluminiumoxid mit einer Temperatur im Bereich von größer 0 °C und kleiner 100 °C und einen pH-Wert von pH 0 - 14 erhalten wird. Dazu kann zum Beispiel auch schon die Rutheniumsalzlösung (ii) und/oder die wässrige Suspension von Aluminiumoxid (iii) auf die entsprechende Temperatur und/oder den entsprechenden pH-Wert eingestellt werden, bevor die Rutheniumsalzlösung (ii) und die wässrige Suspension von Aluminiumoxid (iii) gemischt werden, so dass die resultierende Mischung (i) ohne weiteres Zutun die Temperatur im gewünschten Bereich und den pH-Wert im gewünschten Bereich aufweist. Alternativ kann auch erst die Rutheniumsalzlösung (ii) und eine wässrige Suspension von Aluminiumoxid (iii) gemischt werden und dann eine Temperatur im Bereich von größer 0 °C und kleiner 100 °C und einen pH-Wert von pH 0 - 14 in der Mischung (i) eingestellt werden.

"Temperatur im Bereich von größer 0 °C und kleiner 100 °C" bedeutet im Sinne der Erfindung bevorzugt eine Temperatur von 50 °C bis kleiner 100 °C, bevorzugt 55 °C bis 75 °C, noch bevorzugter 60 °C.

In der erfindungsgemäßen Ausführungsform, in welcher erst die Rutheniumsalzlösung (ii) und eine wässrige Suspension von Aluminiumoxid (iii) gemischt werden und dann eine Temperatur im Bereich von größer 0 °C und kleiner 100 °C und einen pH-Wert von pH 0 - 14 in der Mischung (i) eingestellt werden, erfolgt die Einstellung auf eine Temperatur im Bereich von größer 0 °C und kleiner 100 °C bevorzugt durch Erhitzen über einen Zeitraum von 30 min bis 300 min, bevorzugt 90 min bis 240 min, bevorzugter 120 min bis 200 min, besonders bevorzugt innerhalb von 180 min. Bevorzugt wird in dieser Ausführungsform außerdem ein alkalischer pH-Wert von größer pH 7.0 bis pH 14.0, bevorzugt von größer pH 7.0 bis pH 10.0, bevorzugter von pH 7.5 bis pH 8.5 und besonders bevorzugt von pH 8.0 eingestellt.

Ein saurer pH-Wert kann mit jeder organischen oder anorganischen Säure eingestellt werden, insbesondere Halogenwasserstoffen, bevorzugt HCl, Schwefelsäure, Salpetersäure, schweflige Säure, salpetrige Säure.

Ein alkalischer pH-Wert kann durch Zugabe einer entsprechenden Menge an Base B2 erreicht werden. Die

Base B2 kann dabei als Feststoff oder als Lösung zu der Mischung (i), der Rutheniumsalzlösung (ii) oder der wässrigen Suspension von Aluminiumoxid (iii), bevorzugt zur Mischung (i) zugegeben werden. Dem Fachmann ist geläufig, welche Menge der Base B2 er zugeben muss, um den gewünschten pH-Wert einzustellen.

Als Base B2 im Sinne der vorliegenden Erfindung kann jede organische oder anorganische Base eingesetzt werden. Insbesondere ist die Base B2 ausgewählt aus der Gruppe bestehend aus Erdalkalimetallhydroxiden, Alkalimetallhydroxiden, Erdalkalimetallcarbonaten, Alkalimetallcarbonaten, Erdalkalimetallhydrogencarbonate, Alkalimetallhydrogencarbonaten, Erdalkalimetallacetaten, Alkalimetallacetaten. Bevorzugt ist die Base B2 ausgewählt aus der Gruppe bestehend aus Lithiumhydroxid, Natriumhydroxid, Kaliumhydroxid, Lithiumcarbonat, Natriumcarbonat, Kaliumcarbonat, Lithiumhydrogencarbonat, Natriumhydrogencarbonat, Kaliumhydrogencarbonat, Lithiumacetat, Natriumacetat, Kaliumacetat. Besonders bevorzugt ist die Base B2 ausgewählt aus der Gruppe bestehend aus Natriumcarbonat, Lithiumhydroxid, Lithiumcarbonat. Gesonders bevorzugt ist die Base B2 Natriumcarbonat.

In einer bevorzugten Ausführungsform ist das Verfahren zur Herstellung eines Ru/$AlO_x$-Katalysatorsystems dadurch gekennzeichnet, dass ein Reduktionsmittel (iv) zum Einsatz kommt. Dies kann dadurch realisiert werden, dass das Reduktionsmittel (iv) vor Schritt b) oder zwischen Schritt b) und c), bevorzugt zwischen Schritt b) und c) zu der Mischung (i) gegeben wird.

Als Reduktionsmittel (iv) eignet sich jeder Stoff, der Ruthenium zu reduzieren in der Lage ist. Das Reduktionsmittel ist insbesondere ausgewählt aus der Gruppe bestehend aus Hydrazin, Borhydride der Alkalimetalle, Borhydride der Erdalkalimetalle, Formiate der Alkalimetalle, Formiate der Erdalkalimetalle, Hypophosphite der Alkalimetalle, Hypophosphite der Erdalkalimetalle, Wasserstoff, Ameisensäure, Formaldehyd. Bevorzugt ist das Reduktionsmittel ausgewählt aus der Gruppe bestehend aus Hydrazin, Natriumformiat, Natriumborhydrid, Natriumhypophosphit, Wasserstoff, Ameisensäure, Formaldehyd. Besonders bevorzugt wird als Reduktionsmittel Formaldehyd, ganz besonders bevorzugt als wässrige Lösung, eingesetzt.

Bevorzugt wird das Reduktionsmittel zwischen Schritt b) und c) zu der Mischung (i) gegeben. In dieser bevorzugten Ausführungsform des Verfahren zur Herstellung eines Ru/$AlO_x$-Katalysatorsystems ist es besonders bevorzugt, dass die Mischung nach Zugabe des Reduktionsmittels noch 30 - 90 min, ganz besonders bevorzugt 60 min bei der Temperatur gerührt wird.

Der bei Schritt a) und b) vorhandene Druck ist nicht beschränkt, insbesondere werden diese Schritte bei Normaldruck ausgeführt (1 bar).

Im Schritt c) des Verfahrens zur Herstellung eines Ru/$AlO_x$-Katalysatorsystems wird die Mischung (i), die nach Schritt b) des Verfahrens zur Herstellung eines Ru/$AlO_x$-Katalysatorsystems und optionaler Zugabe des Reduktionsmittels nach Schritt b) erhalten wird, filtriert, so dass das Ru/$AlO_x$-Katalysatorsystem erhalten wird. Die Filtration findet üblicherweise über alle dem Fachmann bekannten Filtrationsmethoden statt, insbesondere Methoden wie Abdekantieren, Filtrieren, Zentrifugieren, Vakuumfiltration, Druckfiltration. Dadurch kann das Ru/$AlO_x$-Katalysatorsystems aus der Mischung (i) isoliert werden.

Im Schritt d) des Verfahrens zur Herstellung eines Ru/$AlO_x$-Katalysatorsystems wird das im Schritt c) erhaltene Ru/$AlO_x$-Katalysatorsystem getrocknet. Dies findet üblicherweise im Vakuum und einer Temperatur von unter 120 °C, bevorzugt von unter 100 °C statt, besonders bevorzugt bei 80 °C statt. "Vakuum" im Sinne der Erfindung bedeutet Drücke unterhalb 1 bar, bevorzugt unterhalb 0.8 bar, bevorzugter unterhalb 0.1 bar, besonders bevorzugt unterhalb 0.02 bar, am bevorzugtesten unterhalb von 0.005 bar.

Der Gehalt an Ruthenium im Ru/$AlO_x$-Katalysatorsystem nach Schritt d) des Verfahrens zur Herstellung eines Ru/$AlO_x$-Katalysatorsystems kann dadurch gesteuert werden, dass man im Schritt a) die entsprechende Menge Rutheniumsalz mit der entsprechenden Menge Aluminiumoxid umsetzt. Dies liegt im Vermögen des Fachmanns. Beispielsweise kann ein Ru/$AlO_x$-Katalysatorsystem mit einem entsprechenden Rutheniumgehalt von 5 Gew.-% dadurch hergestellt werden, dass in Schritt a) 5 g Ruthenium in Form von 15,68 g Rutheniumnitrosylnitrat $Ru(NO)(NO_3)_3$ zu einer wässrigen Suspension von 95 g Aluminiumoxid zugegeben wird. Das Reduktionsmittel wird, wenn es zugegeben wird, insbesondere in 2 fachem molaren Überschuss, bevorzugt in äquimolarer Menge bezogen auf die molare Menge an Ruthenium in der Komponente (ii) zugegeben.

In einer ganz besonders bevorzugten Ausführungsform des Verfahrens zur Herstellung eines Ru/$AlO_x$-Katalysatorsystems umfasst dies

a) Herstellung einer Mischung (i) umfassend eine Rutheniumsalzlösung (ii) und eine wässrige Suspension von Aluminiumoxid (iii);

b1) Einstellen eines pH-Wert von pH 7.5 - 8.5 unter Zugabe einer Base B2;

b2) Einstellen der Mischung (i) auf eine Temperatur im Bereich von 55 - 75 °C durch Erhitzen über einen Zeitraum von 120 min bis 200 min, besonders bevorzugt von 180 min;

b3) Zugabe eines Reduktionsmittels, bevorzugt Formaldehyd, zur Mischung (i);

c) Abtrennen des Ru/$AlO_x$-Katalysatorsystems durch Filtration der Mischung (i);

d) Trocknen des in Schritt c) abgetrennten Ru/$AlO_x$-Katalysatorsystems.

Die vorliegende Erfindung betrifft in einem zweiten Aspekt ein Verfahren zur Herstellung von 1,2-Pentandiol, welches dadurch gekennzeichnet ist, dass man Furfurylalkohol mit Wasserstoff in Gegenwart eines Ru/AC-Katalysatorsystems umsetzt.

Im Sinne der Erfindung wird der Ausdruck "Ru/AC-Katalysatorsystem" als Abkürzung für "ein Katalysatorsystem umfassend (I) Ruthenium und (II) einen Träger aus Aktivkohle, auf welchem das Ruthenium geträgert ist" verwendet.

Der Gehalt an Ruthenium im Ru/AC-Katalysatorsystem liegt, bezogen auf die Gesamtgewicht des Ru/AC-Katalysatorsystems, insbesondere im Bereich 0.01 - 30 Gew.-%, bevorzugt im Bereich 0.01 - 20 Gew.-%, besonders bevorzugt im Bereich 0.5 - 10.5 Gew.-%, ganz besonders bevorzugt im Bereich 5 - 10 Gew.-%, am bevorzugtesten bei 5 Gew.-%. Die Werte werden gemessen nach Trocknung des Ru/AC-Katalysatorsystems bei 80 °C im Vakuum bis zur Gewichtskonstanz. Der Gehalt an Ruthenium bezogen auf das Gesamtgewicht des Ru/AC-Katalysatorsystems kann nach DIN51009 bestimmt werden.

Es wurde überraschend festgestellt, dass mit dem Ru/AC-Katalysatorsystem im erfindungsgemäßen Verfahren zur Herstellung von 1,2-Pentandiol das Produkt 1,2-Pentandiol in einer überraschend hohen Selektivität hergestellt werden kann im Vergleich zum Stand der Technik, wie zum Beispiel von Zhang *et al.* beschrieben [49].

Die BET-Gesamtoberfläche des Ru/AC-Katalysatorsystems liegt zwischen 300 und 2000 $m^2$/g Ru/AC-Katalysatorsystem, insbesondere zwischen 500 und 1500 $m^2$/g Ru/AC-Katalysatorsystem, bevorzugt zwischen 800 und 1100 $m^2$/g Ru/AC-Katalysatorsystem. Die BET-Gesamtoberfläche wird dabei nach DIN ISO 9277 bestimmt.

Das Porenvolumen des Ru/AC-Katalysatorsystems beträgt 0.3 bis 2.0 ml/g Ru/AC-Katalysatorsystem, bevorzugt 0.5 bis 1.8 ml/g Ru/AC-Katalysatorsystem, besonders bevorzugt 0.6 bis 1.5 ml/g Ru/AC-Katalysatorsystem. Das Porenvolumen wird dabei nach DIN ISO 9277 bestimmt.

Die Korngrößenverteilung des Ru/AC-Katalysatorsystems liegt typischerweise bei d10 = 2 - 10 $\mu$m; d50 = 15 - 30 $\mu$m; d90 = 50 - 100 $\mu$m.

Dabei bedeutet "d10 = 2 - 10 $\mu$m", dass 10 % des Ru/AC-Katalysatorsystems eine Körngröße von 2 - 10 $\mu$m oder kleiner aufweist.

Dabei bedeutet "d50 = 15 - 30 $\mu$m", dass 50 % des Ru/AC-Katalysatorsystems eine Körngröße von 15 - 30 $\mu$m oder kleiner aufweist.

Dabei bedeutet "d90 = 50 - 100 $\mu$m", dass 90 % des Ru/AC-Katalysatorsystems eine Körngröße von 50 - 100 $\mu$m oder kleiner aufweist.

Im erfindungsgemäßen Verfahren zur Herstellung von 1,2-Pentandiol gemäß des zweiten Aspekts der Erfindung ist die Temperatur grundsätzlich nicht beschränkt, so kann beispielsweise eine Temperatur von größer oder gleich 100 °C eingestellt werden. Bevorzugt wird das erfindungsgemäße Verfahren zur Herstellung von 1,2-Pentandiol gemäß des zweiten Aspekts der Erfindung bei einer Temperatur von 100 °C bis 280 °C, besonders bevorzugt bei einer Temperatur von 150 bis 260°C durchgeführt. Als ganz besonders vorteilhaft hat es sich erwiesen und ganz besonders bevorzugt ist es deshalb,das erfindungsgemäße Verfahren zur Herstellung von 1,2-Pentandiol gemäß des zweiten Aspekts der Erfindung bei einer Temperatur von 200 °C bis 240 °C durchzuführen.

Das erfindungsgemäße Verfahren zur Herstellung von 1,2-Pentandiol gemäß des zweiten Aspekts der Erfindung wird bevorzugt unter autoklaven Bedingungen durchgeführt. "Autoklave Bedingungen" beschreiben im Sinne der Erfindung Drücke von gleich oder mehr als 10 bar, bevorzugt Drücke im Bereich von 10 bis 200 bar, besonders bevorzugt Drücke im Bereich von 50 bis 150 bar, am bevorzugtesten einen Druck von 100 bar.

Das erfindungsgemäße Verfahren zur Herstellung von 1,2-Pentandiol gemäß des zweiten Aspekts der vorliegenden Erfindung kann in jedem beliebigen Lösemittel durchgeführt werden. Insbesondere werden in diesem Zusammenhang Lösemittel ausgewählt aus der Gruppe bestehend aus Wasser, Ethanol, Tetrahydrofuran, 1,4-Dioxan eingesetzt. Besonders bevorzugt wird als Lösemittel Wasser eingesetzt.

Das erfindungsgemäße Verfahren gemäß des zweiten Aspekts der vorliegenden Erfindung kann im Batchbetrieb, aber auch kontinuierlich durchgeführt werden.

Im erfindungsgemäßen Verfahren zur Herstellung von 1,2-Pentandiol gemäß des zweiten Aspekts der vorliegenden Erfindung kann als Wasserstoff jedes Gas verwendet werden, welches freien Wasserstoff aufweist und frei von Zusätzen ist, die das Verfahren unterbinden könnten, wie zum Beispiel Kohlenmonoxid.

Die Menge an im erfindungsgemäßen Verfahren zur Herstellung von 1,2-Pentandiol gemäß des zweiten Aspekts der vorliegenden Erfindung eingesetzten Ru/AC-Katalysatorsystems ist nicht besonders beschränkt und liegt insbesondere im Bereich von 0.01 - 30 Gew.-%, bevorzugt 0.5 - 30 Gew.-%, besonders bevorzugt 1 - 20 Gew.-%, ganz besonders bevorzugt 1 - 10 Gew-% jeweils bezogen auf das Gewicht des eingesetzten Furfurylalkohols.

Überraschenderweise wurde außerdem gefunden, dass sich besonders gute Selektivitäten zum 1,2-Pentandiol ergeben, wenn das erfindungsgemäße Verfahren zur Herstellung von 1,2-Pentandiol gemäß des zweiten Aspekts der vorliegenden Erfindung bei einem pH-Wert von 5.3 bis 10.3, bevorzugter von 6.2 bis 10.2, am bevorzugtesten bei einem pH-Wert von 7.6 durchgeführt wird. Dies kann zum Beispiel dadurch erreicht werden, in dem vor der Reaktion eine Base B1 zugegeben wird. Die Menge der zum Erreichen des jeweiligen pH-Wertes notwendigen Base kann vom Fachmann routinemäßig bestimmt werden.

Der Ausdruck "das erfindungsgemäße Verfahren zur Herstellung von 1 ,2-Pentandiol gemäß des zweiten Aspekts der vorliegenden Erfindung wird bei einem pH-Wert von 5.3 bis 10.3, bevorzugter von 6.2 bis 10.2, am bevorzugtesten bei einem pH-Wert von 7.6 durchgeführt" bedeutet im Falle dass, wenn das erfindungsgemäße Verfahren zur Herstellung von 1,2-Pentandiol gemäß des zweiten Aspekts der vorliegenden Erfindung unter autoklaven Bedingungen durchgeführt wird, dass vor der Reaktion soviel Base B1 zugegeben wird, dass der pH-Wert von 5.3 bis 10.3, bevorzugter von 6.2 bis 10.2, am bevorzugtesten von 7.6 direkt nach Beendigung der Reaktion in der Reaktionslösung gemessen werden kann. "Direkt nach Beendigung der Reaktion" bedeutet im Sinne der Erfindung den Zeitpunkt, zu welchem sich der Anteil an 1,2-Pentandiol nicht mehr ändert.

Die Base B1 ist oben definiert.

Das Ru/AC-Katalysatorsystem wird durch Trägern des Rutheniums auf dem Aktivkohleträger hergestellt. Das Ruthenium kann auf den Aktivkohleträger durch Imprägnierung, Beschichtung, Abscheidung durch Kopräzipitation, oder andere geeignete Prozesse wie *spray deposition* geträgert werden. Bevorzugt wird das Ru/AC-Katalysatorsystem hergestellt, in dem der Aktivkohleträger mit Ruthenium imprägniert wird. Dies geschieht, in dem der Aktivkohleträger mit einer Rutheniumsalzlösung in Kontakt gebracht wird und das Ruthenium auf dem Aktivkohleträger durch Besprühen oder mittels pH-gesteuerter Kopräzipitation abgeschieden wird.

In einer bevorzugten Vorgehensweise wird das Ru/AC-Katalysatorsystem durch ein Verfahren zur Herstellung eines Ru/AC-Katalysatorsystems erhalten.

Das Verfahren zur Herstellung eines Ru/AC-Katalysatorsystems umfasst

a) Herstellung einer Mischung (i) umfassend eine Rutheniumsalzlösung (ii) und eine wässrige Suspension von Aktivkohle (iii);

b) Einstellen der Mischung (i) auf eine Temperatur im Bereich von größer 0°C und kleiner 100°C und einen pH-Wert von pH 0 - 14;

c) Abtrennen des Ru/AC-Katalysatorsystems durch Filtration der Mischung (i).

Die vorliegende Erfindung betrifft auch ein Ru/AC-Katalysatorsystem erhältlich nach dem Verfahren zur Herstellung eines Ru/AC-Katalysatorsystems.

Im Schritt a) des Verfahren zur Herstellung eines Ru/AC-Katalysatorsystems wird zunächst eine Mischung (i) umfassend eine Rutheniumsalzlösung (ii) und eine wässriger Suspension von Aktivkohle (iii) hergestellt. Dies kann durch jede dem Fachmann geläufige Art und Weise geschehen, geschieht typischerweise jedoch, in dem eine Rutheniumsalzlösung (ii) und eine wässrige Suspension von Aktivkohle (iii) gemischt werden.

Die Rutheniumsalzlösung (i) im Sinne der Erfindung ist jede rutheniumhaltige, bevorzugt wässrige, Lösung eines Rutheniumsalzes. Das Rutheniumsalz im Sinne der Erfindung wird insbesondere ausgewählt aus der Gruppe bestehend aus Rutheniumcarbonat $[Ru(CO_3)_3]$; Rutheniumcarboxylate wie z. B. Ruthenium (II, III) μ-oxoacetat $[(CH_3CO_2)_7Ru_3O\text{-}3H_2O]$; Rutheniumcarbonyle; Rutheniumhalide wie z. B. Rutheniumbromid ($RuBr_3$), Rutheniumchlorid ($RuCl_3$), Rutheniumchloridhydrat ($RuCl_3\text{-}xH_2O$), Rutheniumiodid ($RuI_3$); Rutheniumnitrate wie z. B. $Ru(NO_3)_3\text{-}xH_2O$; Rutheniumoxide wie z. B. $RuO_2$ und Ruthenium(IV)oxidhydrat ($RuO_2\text{-}xH_2O$); Rutheniumnitrosylnitrate wie z. B. Rutheniumnitrosylnitrat $[Ru(NO)(NO_3)_x(OH)_y$, wobei x = 1, 2, 3; y = 0, 1, 2; und x+y = 3]; Rutheniumchlorkomplexe; Rutheniumaminkomplexe, Rutheniumnitritkomplexe. In einer bevorzugten Ausführungsform ist das Rutheniumsalz Rutheniumnitrosylnitrat $[Ru(NO)(NO_3)_x(OH)_y$, wobei x = 1, 2, 3; y = 0, 1, 2; und x+y = 3], besonders bevorzugt ist Rutheniumnitrosylnitrat $Ru(NO)(NO_3)_3$.

Als wässrige Suspension von Aktivkohle (ii) eignet sich jede Suspension, die Aktivkohle, aufweist. "Aktivkohle" bezeichnet im Sinne der Erfindung den dem Fachmann geläufigen Begriff und bezieht sich auf ein amorphes, bevorzugt zu mehr als 90 % kohlenstoffhaltiges Material mit einer hochporösen Struktur, einer innere Oberfläche von bevorzugt 300-2000 $M^2$/g Kohle und einer Dichte im Bereich von bevorzugt 0.2 - 0.6 g/$cm^3$. Als Aktivkohle kommt im Sinne der Erfindung bevorzugt chemisch aktivierte Kohlenstoff, physikalisch aktivierte Kohlenstoff, Ruß, carbon black, carbon nanotubes, Aerogele in Frage, bevorzugt carbon black.

In Schritt b) des Verfahrens zur Herstellung eines Ru/AC-Katalysatorsystems wird die Mischung (i) auf eine Temperatur im Bereich von größer 0 °C und kleiner 100 °C und einen pH-Wert von pH 0 - 14 eingestellt.

Dies umfasst jegliche Vorgehensweise, durch welche eine Mischung (i) umfassend eine Rutheniumsalzlösung und eine

wässriger Suspension von Aktivkohle mit einer Temperatur im Bereich von größer 0 °C und kleiner 100 °C und einen pH-Wert von pH 0 - 14 erhalten wird. Dazu kann zum Beispiel auch schon die Rutheniumsalzlösung (ii) und/oder die wässrige Suspension von Aktivkohle (iii) auf die entsprechende Temperatur und/oder den entsprechenden pH-Wert eingestellt werden bevor die Rutheniumsalzlösung (ii) und die wässrige Suspension von Aktivkohle (iii) gemischt werden, so dass dadurch die Mischung (i) mit der gewünschten Temperatur und dem gewünschten pH-Wert erhalten wird. Alternativ kann auch erst die Rutheniumsalzlösung (ii) und eine wässrige Suspension von Aktivkohle (iii) gemischt werden und dann eine Temperatur im Bereich von größer 0 °C und kleiner 100 °C und einen pH-Wert von pH 0 - 14 in der Mischung (i) eingestellt werden.

"Temperatur im Bereich von größer 0 °C und kleiner 100 °C" bedeutet im Sinne der Erfindung bevorzugt eine Temperatur von 50 °C bis kleiner 100 °C, bevorzugt 55°C bis 75 °C, noch bevorzugter 60 °C.

In der erfindungsgemäßen Ausführungsform, in welcher erst die Rutheniumsalzlösung (ii) und eine wässrige Suspension von Aktivkohle (iii) gemischt werden und dann eine Temperatur im Bereich von größer 0°C und kleiner 100°C und einen pH-Wert von pH 0 - 14 in der Mischung (i) eingestellt werden, erfolgt die Einstellung auf eine Temperatur im Bereich von größer 0 °C und kleiner 100 °C bevorzugt durch Erhitzen über einen Zeitraum von 30 min bis 300 min, bevorzugt 90 min bis 240 min, bevorzugter 120 min bis 200 min, besonders bevorzugt innerhalb von 180 min. Bevorzugt wird in dieser Ausführungsform außerdem ein alkalischer pH-Wert von größer pH 7.0 bis pH 14.0, bevorzugt von größer pH 7.0 bis pH 10.0, bevorzugter von pH 7.5 bis pH 8.5 und besonders bevorzugt von pH 8.0 eingestellt.

Ein saurer pH-Wert kann mit jeder organischen oder anorganischen Säure eingestellt werden, insbesondere Halogenwasserstoffen, bevorzugt HCl, Schwefelsäure, Salpetersäure, schweflige Säure, salpetrige Säure.

Ein alkalischer pH Wert kann durch Zugabe einer entsprechenden Menge an Base B2 zu den jeweiligen Komponenten (i), (ii), (iii) erreicht werden. Die Base kann dabei als Feststoff oder als Lösung zugegeben werden. Dem Fachmann ist geläufig, welche Menge der Base B2 er zugeben muss, um den gewünschten pH-Wert einzustellen.

In einer bevorzugten Ausführungsform des Verfahrens zur Herstellung eines Ru/AC-Katalysatorsystems wird in Schritt a) ein alkalischer pH-Wert der Mischung (i) eingestellt, noch bevorzugter ein pH-Wert im Bereich von größer pH 7.0 bis pH 14.0, bevorzugt von größer pH 7.0 bis pH 10.0, bevorzugter von pH 7.5 bis pH 8.5 und besonders bevorzugt von pH 8.0.

Als Base B2 im Sinne der vorliegenden Erfindung kann jede organische oder anorganische Base eingesetzt werden. Insbesondere ist die Base B2 ausgewählt aus der Gruppe bestehend aus Erdalkalimetallhydroxiden, Alkalimetallhydroxiden, Erdalkalimetallcarbonaten, Alkalimetallcarbonaten, Erdalkalimetallhydrogencarbonate, Alkalimetallhydrogencarbonaten, Erdalkalimetallacetaten, Alkalimetallacetaten. Bevorzugt ist die Base B2 ausgewählt aus der Gruppe bestehend aus Lithiumhydroxid, Natriumhydroxid, Kaliumhydroxid, Lithiumcarbonat, Natriumcarbonat, Kaliumcarbonat, Lithiumhydrogencarbonat, Natriumhydrogencarbonat, Kaliumhydrogencarbonat, Lithiumacetat, Natriumacetat, Kaliumacetat. Besonders bevorzugt ist die Base B2 ausgewählt aus der Gruppe bestehend aus Natriumcarbonat, Lithiumhydroxid, Lithiumcarbonat. Ganz besonders bevorzugt ist die Base B2 Natriumcarbonat.

In einer bevorzugten Ausführungsform ist das Verfahren zur Herstellung eines Ru/AC-Katalysatorsystems dadurch gekennzeichnet, dass ein Reduktionsmittel (iv) zum Einsatz kommt. Dies kann dadurch realisiert werden, dass das Reduktionsmittel (iv) vor Schritt b) oder zwischen Schritt b) und c), bevorzugt zwischen Schritt b) und c) zu der Mischung (i) gegeben wird.

Als Reduktionsmittel (iv) eignet sich jeder Stoff, der Ruthenium zu reduzieren in der Lage ist. Das Reduktionsmittel ist insbesondere ausgewählt aus der Gruppe bestehend aus Hydrazin, Borhydride der Alkalimetalle, Borhydride der Erdalkalimetalle, Formiate der Alkalimetalle, Formiate der Erdalkalimetalle, Hypophosphite der Alkalimetalle, Hypophosphite der Erdalkalimetalle, Wasserstoff, Ameisensäure, Formaldehyd. Bevorzugt ist das Reduktionsmittel ausgewählt aus der Gruppe bestehend aus Hydrazin, Natriumformiat, Natriumborhydrid, Natriumhypophosphit, Wasserstoff, Ameisensäure, Formaldehyd. Besonders bevorzugt wird als Reduktionsmittel Formaldehyd, noch bevorzugter als wässrige Lösung, eingesetzt.

Bevorzugt wird das Reduktionsmittel zwischen Schritt b) und c) zu der Mischung (i) gegeben. In dieser bevorzugten Ausführungsform des Verfahren zur Herstellung eines Ru/AC-Katalysatorsystems ist es besonders bevorzugt, dass die Mischung nach Zugabe des Reduktionsmittels noch 30 - 90 min, ganz besonders bevorzugt 60 min bei der Temperatur gerührt wird.

Der bei Schritt a) und b) vorhandene Druck ist nicht beschränkt, insbesondere werden diese Schritte bei Normaldruck ausgeführt (1 bar).

Im Schritt c) des Verfahrens zur Herstellung eines Ru/AC-Katalysatorsystems wird die Mischung (i), die nach Schritt b) des Verfahrens zur Herstellung eines Ru/AC-Katalysatorsystems und optionaler Zugabe des Reduktionsmittels nach Schritt b) erhalten wird, filtriert, so dass das Ru/AC-Katalysatorsystem erhalten wird. Die Filtration findet üblicherweise über alle dem Fachmann bekannten Filtrationsmethoden statt, insbesondere Methoden wie Abdekantieren, Filtrieren, Zentrifugieren, Vakuumfiltration, Druckfiltration. Dadurch kann das Ru/AC-Katalysatorsystems aus der Mischung (i) isoliert werden.

Der Gehalt an Ruthenium im Ru/AC-Katalysatorsystem nach Schritt d) des Verfahrens zur Herstellung eines

Ru/AC-Katalysatorsystems kann dadurch gesteuert werden, dass man im Schritt a) die entsprechende Menge Rutheniumsalz mit der entsprechenden Menge Aktivkohle umsetzt. Dies liegt im Vermögen des Fachmanns. Beispielsweise kann ein Ru/AC-Katalysatorsystem mit einem entsprechenden Rutheniumgehalt von 5 Gew.-% dadurch hergestellt werden, dass in Schritt a) 5 g Ruthenium in Form von 15,68 g Rutheniumnitrosylnitrat $Ru(NO)(NO_3)_3$ zu einer wässrigen Suspension von 95 g Aktivkohle zugegeben wird. Das Reduktionsmittel wird, wenn es zugegeben wird, insbesondere in 2 fachem molaren Überschuss, bevorzugt in äquimolarer Menge bezogen auf die molare Menge an Ruthenium in der Komponente (ii) zugegeben.

In einer ganz besonders bevorzugten Ausführungsform des Verfahrens zur Herstellung eines Ru/AC-Katalysatorsystems umfasst dies

a) Herstellung einer Mischung (i) umfassend eine Rutheniumsalzlösung (ii) und eine wässrige Suspension von Aktivkohle (iii);

b1) Einstellen eines pH-Wert von pH 7.5 - 8.5 unter Zugabe einer Base B2;

b2) Einstellen der Mischung (i) auf eine Temperatur im Bereich von 55 - 75°C durch Erhitzen über einen Zeitraum von 120 min bis 200 min, besonders bevorzugt von 180 min;

b3) Zugabe eines Reduktionsmittels, bevorzugt Formaldehyd, zur Mischung (i);

c) Abtrennen des Ru/AC-Katalysatorsystems durch Filtration der Mischung (i).

Die vorliegende Erfindung betrifft in einem dritten Aspekt ein Verfahren zur Herstellung von 1,2-Pentandiol, welches dadurch gekennzeichnet ist, dass man Furfurylalkohol mit Wasserstoff in Gegenwart eines Pt(IV)-Oxid Katalysators umsetzt.

"Pt(IV)-Oxid-Katalysator" im Sinne der vorliegenden Erfindung bedeutet ein Adams-Typ-Katalysator und bezieht sich auf Pt(IV)-Oxid.

Es wurde überraschend festgestellt, dass mit dem Pt(IV)-Oxid-Katalysator im erfindungsgemäßen Verfahren zur Herstellung von 1,2-Pentandiol das Produkt 1,2-Pentandiol mit einer überraschend hohen Selektivität hergestellt werden kann.

Das erfindungsgemäße Verfahren gemäß des dritten Aspekts der Erfindung läuft üblicherweise wie folgt ab:

Im erfindungsgemäßen Verfahren zur Herstellung von 1,2-Pentandiol gemäß des dritten Aspekts der Erfindung ist die Temperatur grundsätzlich nicht beschränkt. Vorzugsweise wird das Verfahren jedoch schon bei einer Temperatur im Bereich von größer 0 °C bis unter 100 °C durchgeführt, bevorzugter im Bereich 5 °C bis 60 °C, noch bevorzugter im Bereich 20 °C bis 55 °C.

Der im erfindungsgemäße Verfahren zur Herstellung von 1,2-Pentandiol gemäß des dritten Aspekts der Erfindung ist der Druck grundsätzlich nicht beschränkt und beträgt insbesondere 1 - 10 bar, bevorzugt 1 - 5 bar, besonders bevorzugt 2 bar.

Das erfindungsgemäße Verfahren gemäß des dritten Aspekts der vorliegenden Erfindung kann in jedem beliebigen Lösemittel durchgeführt werden. Insbesondere werden in diesem Zusammenhang Lösemittel ausgewählt aus der Gruppe bestehend aus Essigsäure, Wasser und Ethanol eingesetzt. Besonders gute Selektivitäten zu 1,2-Pentandiol wurden beobachtet, wenn als Lösungsmittel Ethanol eingesetzt wurde. Demnach wird im erfindungsgemäßen Verfahren gemäß des ersten Aspekts der vorliegenden Erfindung besonders bevorzugt als Lösemittel Ethanol gewählt.

Die nachfolgenden Beispiele sollen die Erfindung illustrieren und sind nicht so zu verstehen, als würden sie die Erfindung in irgendeiner Form beschränken.

## Beispielteil

### 1. Verwendete Chemikalien

Folgende Chemikalien wurden verwendet:

Furfurylalkohol (*Sigma Aldrich*, ≥ 98 %)

Lösungsmittel:

1,4-Dioxan (*Merck,* ≥ 99 %), Essigsäure (*Sigma Aldrich,* ≥ 99.5 %), Ethanol (*Roth,* ≥ 99.8 %), THF (*Sigma Aldrich,* ≥ 99.9 %), Wasser (*VWR,* HiPerSolv CHROMANORM).

Säuren und Basen:

Natriumacetat (*F. Klasovsky,* ACS-Qualität), Natriumcarbonat (*Sigma Aldrich,* ≥ 99.5 %), 2 N Salzsäure (*Roth*).

Metallvorläuferverbindungen:

Kupfer(II)-nitrat-trihydrat (*Merck,* ≥ 99.5 %), Nickel(II)-nitrat-hexahydrat *(Aldrich,* 99.999 %), Tetraaminplatin(II)-nitrat (*Alfa Aesar,* 99.99 %), Zinknitrat-hexahydrat (*Sigma Aldrich,* 98 %), Zinn(II)-chlorid (*Alfa Aesar,* ≥ 99%).

Zur Kalibrierung von GC und GC-MS:

1,2-Butandiol (*Fluka,* ≥ 98 %), Cyclopentanol *(Aldrich,* 99 %), Cyclopentanon (*Sigma Aldrich,* ≥ 99 %), 1,2-Hexandiol *(Aldrich,* 98 %), 2-Methylfuran (*Aldrich,* 99 %), 1-Pentanol (*Sigma Aldrich,* ≥ 99 %), 1,2-Pentaniol *(Aldrich,* 96 %), 1,5-Pentandiol (*Fluka,* ≥ 97 %), Tetrahydrofurfurylalkohol (*Fluka,* ≥ 98 %).

Ionische Flüssigkeiten:

1-Butyl-3-methylimidazoliumdicyanamid (*Merck*), N-Butyl-3-methylpyridiniumdicyanamid (*Merck*), 1-Butyl-1-methylpyrrolidiniumdicyan-amid (*Merck*).

Gase:

Argon (*Linde,* ≥ 99.999%), Wasserstoff (*Linde,* ≥ 99.999 %).

Sonstige:

Natriumdicyanamid *(Aldrich,* 96 %)

Katalysatoren:

Tabelle 6: Getestete Katalysatoren für die Hydrogenolyse von Furfurylalkohol.

| **Katalysatorbezeichnung** | **Hersteller** | **Zusammensetzung** |
|---|---|---|
| Al1 | Eigene Herstellung (Abschnitt 2.11) | 5 % $Ru/Al_2O_3$ |
| Al2 | Eigene Herstellung (Abschnitt 2.12) | 5 % Ru/AC |
| Al3 | Eigene Herstellung (Abschnitt 2.12) | 5 % Ru/AC |
| RR506 | Südzucker | 1% $Ru/Al_2O_3$ |
| Y40753-BS | KataLeuna | 1% $Ru/Al_2O_3$ |
| FS384 | Eigene Herstellung | 1 % Ru auf porösen Glas |
| Pt-JA-024 | Eigene Herstellung | Pt/Polyanilin |
| Pt-JA-026 | Eigene Herstellung | 10 % Pt/C |
| Pt-JA-023 | Eigene Herstellung | Pt(IV)-Oxid |
| Pt-JA-021 | Eigene Herstellung | Pt(IV)-Oxid |
| Heraeus | Heraeus | Pt(IV)-Oxid |
| Sigma Aldrich | Sigma Aldrich | Pt(IV)-Oxid |

**2. Allgemeine Versuchsvorschriften**

Die Katalysatoren wurden in Form von feinkörnigem Pulver verwendet. Falls sie als Formkörper vorlagen, wurden sie zunächst mit einem Mörser zerrieben und gesiebt, wobei lediglich die feinste Fraktion mit Korngrößen ≤ 200 μm zur Hydrogenolyse eingesetzt wurde.

*Allgemeiner Versuchsaufbau*

Der Rührautoklav (*Parr Instruments*) besteht aus rostfreiem Stahl und besitzt 300 ml Fassungsvermögen. Über einen Druckminderer ist ein 500 ml Gastank an den Reaktor angeschlossen, welcher über ein Gaszuleitungssystem sowohl mit Argon (≤ 80 bar) als auch Wasserstoff (≤ 200 bar) beschickt werden kann. Ein Abgasventil dient zur Entspannung des Reaktors nach Versuchsende. Zur Durchmischung wird ein Gaseintragsrührer mit einer maximalen Rührgeschwindigkeit von 1600 rpm verwendet. Zum Aufheizen der Reaktionsmischung dient ein thermostatgesteuerter Heizmantel, mit welchem bis zu 280 °C eingestellt werden können. Über einen Entnahmehahn können während der Reaktion Proben entnommen werden. Desweiteren ist zum Einfüllen des Edukts ein Dosiertank an den Reaktor angeschlossen, welcher ebenfalls über den Gastank mit Wasserstoff und Argon beschickt werden kann. Der Druck wird über ein Manometer und die Temperatur in Gas- und Flüssigphase getrennt über Thermoelemente gemessen. Druck- und Temperaturverläufe werden digital gespeichert und können computergesteuert nachvollzogen werden.

2.1 Allgemeine Versuchsvorschrift zur Umsetzung von Furfurylalkohol mit Wasserstoff in Gegenwart eines Ru/$AlO_x$-Katalysators

Im Autoklaven wird eine wässrige Suspension aus Lösungsmittel und Katalysator, sowie eventuelle Zusatzstoffe wie Base oder ionische Flüssigkeit vorgelegt. Die Reaktionsapparatur wird verschlossen und es wird eine Rührgeschwindigkeit von 1000 rpm eingestellt. Der Reaktor wird dreimal mit 10 bar Argon, sowie einmal mit 10 bar Wasserstoff gespült. Anschließend wird der Wasserstoffdruck so eingestellt, dass der erwartete Druck bei Erreichen der Reaktionstemperatur 10 - 20 bar unter dem Reaktionsdruck liegt. Innerhalb von 30 - 60 Minuten wird auf Reaktionstemperatur aufgeheizt. Sobald diese erreicht ist, wird über den Dosiertank Furfurylalkohol oder eine Lösung von Furfurylalkohol rasch eindosiert. Über den Gastank wird permanent Wasserstoff nachgeliefert und der Reaktionsdruck konstant gehalten. Falls während der Reaktion Proben entnommen werden sollen, wird die Entnahmeleitung vorher mit 1 - 2 ml Reaktionsmischung gespült und dann 0.5 - 1 ml Probe entnommen. Wenn der Wasserstoffdruck im Gastank nicht mehr weiter absinkt, wird der Heizmantel entfernt und der Reaktor an der Luft auf Raumtemperatur abgekühlt. Nach der Entnahme des Reaktionsgemisches wird der Reaktor gründlich gesäubert und bei 150 °C unter 30 bar Argon für 60 Minuten ausgeheizt.
Die Zusammensetzungen der im Autoklaven und im Dosiertank vorgelegten Flüssigphasen für verschiedene Eduktkonzentrationen c(FFOH) und unterschiedliche Lösungsmittel sind in Tabelle 7 aufgelistet. Das Reaktionsvolumen bei Raumtemperatur beträgt stets 100 ml.

Tabelle 7: Zusammensetzung der vorgelegten Flüssigphasen im Autoklaven und Dosiertank.

| **c(FFOH) [g / 100 ml Reaktionslösung]** | **Lösemittel** | **Zusammensetzung Autoklav** | **Zusammensetzung Dosiertank** |
|---|---|---|---|
| 7.46 | Wasser | 86.8 ml Wasser | 7.46 g FFOH, 6.6 ml $H_2O$ |
| 20 | Wasser | 75.0 ml Wasser | 20.0 g FFOH, 7.0 ml $H_2O$ |
| 40 | Wasser | 64.6 ml Wasser | 40.0 g FFOH |
| 40 | Ethanol | 64.0 ml Ethanol | 40.0 g FFOH |
| 40 | THF | 63.8 ml THF | 40.0 g FFOH |
| 40 | 1.4-Dioxan | 64.0 ml 1,4-Dioxan | 40.0 g FFOH |

2.2 Allgemeine Versuchsvorschrift zur Umsetzung von Furfurylalkohol mit Wasserstoff in Gegenwart eines Pt(IV)-Oxid-Katalysator (Pt-JA-023)

Eine Lösung von 20 g FFOH in 81 ml Ethanol werden zusammen mit 0.5 g Katalysator Pt-JA-023 im Autoklaven vorgelegt. Die Reaktionsapparatur wird verschlossen und es wird eine Rührgeschwindigkeit von 1000 rpm eingestellt. Der Reaktor wird dreimal mit 10 bar Argon, sowie einmal mit 10 bar Wasserstoff gespült. Es werden 20 bar Wasserstoffdruck im Reaktor eingestellt und durch permanentes Nachliefern von Wasserstoff aus dem Gastank konstant gehalten. Nach 10 Minuten wird auf 50 °C und nach weiteren 30 Minuten auf 75 °C erhitzt. Weitere 45 Minuten später werden schließlich der Reaktionsdruck auf 60 bar und die Reaktionstemperatur auf 180 °C erhöht. Nach insgesamt 100 Minuten Reaktionszeit wird der Heizmantel entfernt und die Reaktionsmischung an der Luft auf Raumtemperatur abgekühlt. Der Reaktorinhalt wird entnommen und die Reaktionsapparatur gründlich gesäubert.

2.3 Allgemeine Versuchsvorschrift zur Umsetzung von Furfurylalkohol mit Wasserstoff in Gegenwart eines Pt(IV)-Oxid-Katalysator (Heraeus) in Ethanol

Im Autoklaven wird eine Suspension von 0.5 g Katalysator Pt(IV)-Oxid-Katalysator von Heraeus in 71 ml Ethanol vorgelegt. Die Reaktionsapparatur wird verschlossen und es wird eine Rührgeschwindigkeit von 1000 rpm eingestellt. Der Reaktor wird dreimal mit 10 bar Argon, sowie einmal mit 10 bar Wasserstoff gespült. Nachdem über den Gastank 6 bar Wasserstoffdruck im Reaktor eingestellt wurden, wird auf 75 °C erhitzt. Anschließend werden über den Gastank 10 bar Wasserstoffdruck eingestellt und für 30 Minuten gerührt. Der Heizmantel wird entfernt und der Reaktor so lange an der Luft gekühlt, bis die Suspension Raumtemperatur erreicht hat. Über das Abgasventil wird auf 1.5 bar entspannt. Dann wird über den Dosiertank eine Lösung von 10 g FFOH in 20 ml Ethanol rasch eindosiert. Der Wasserstoffdruck wird auf 2 bar eingestellt und durch permanentes Nachliefern von Wasserstoff aus dem Gastank konstant gehalten. 170 Minuten nach Reaktionsbeginn wird die Reaktionstemperatur auf 50 °C erhöht. Nach insgesamt 300 Minuten Reaktionszeit wird schließlich der Heizmantel entfernt und der Reaktor an der Luft auf Raumtemperatur abgekühlt. Der Reaktorinhalt wird entnommen und die Reaktionsapparatur gründlich gesäubert.

2.4 Allgemeine Versuchsvorschrift zur Umsetzung von Furfurylalkohol mit Wasserstoff in Gegenwart eines Pt(IV)-Oxid-Katalysator (Heraeus) in Essigsäure

Im Autoklaven wird eine Suspension von 0.5 g Katalysator in 71 ml Essigsäure vorgelegt. Die Reaktionsapparatur wird verschlossen und es wird eine Rührgeschwindigkeit von 1000 rpm eingestellt. Der Reaktor wird dreimal mit 10 bar Argon, sowie einmal mit 10 bar Wasserstoff gespült. Nachdem über den Gastank 8 bar Wasserstoffdruck im Reaktor eingestellt wurden, wird auf 75 °C erhitzt. Anschließend werden über den Gastank 10 bar Wasserstoffdruck eingestellt und für 30 Minuten gerührt. Der Heizmantel wird entfernt und der Reaktor so lange an der Luft gekühlt, bis die Suspension Raumtemperatur erreicht hat. Über das Abgasventil wird auf 1.5 bar entspannt. Dann wird über den Dosiertank eine Lösung von 10 g FFOH in 20 ml Essigsäure rasch eindosiert und bei einer Reaktionstemperatur von 50 °C der Druck durch Nachliefern von Wasserstoff aus dem Gastank auf 2 bar konstant gehalten. 30 Minuten nach Reaktionsbeginn wird die Temperatur auf 75 °C erhöht. Nach insgesamt 150 Minuten Reaktionszeit wird der Heizmantel entfernt und der Reaktor an der Luft abgekühlt. Der Reaktorinhalt wird entnommen und die Reaktionsapparatur gründlich gesäubert.

2.5 Allgemeine Versuchsvorschrift zur Umsetzung von Furfurylalkohol mit Wasserstoff in Gegenwart eines Pt(IV)-Oxid-Katalysator (Pt-JA-021;Sigma Aldrich) in Ethanol ohne Zusatz von Salzsäure

Im Autoklaven wird eine Suspension von 0.5 g Katalysator Pt-JA-021 bzw. 0.5 g Pt(IV)-Oxid Katalysator von Sigma Aldrich in 71 ml Ethanol vorgelegt. Die Reaktionsapparatur wird verschlossen und es wird eine Rührgeschwindigkeit von 1000 rpm eingestellt. Der Reaktor wird dreimal mit 10 bar Argon, sowie einmal mit 10 bar Wasserstoff gespült. Nachdem über den Gastank 6 bar Wasserstoffdruck im Reaktor eingestellt wurden, wird auf 75 °C erhitzt. Anschließend werden über den Gastank 10 bar Wasserstoffdruck eingestellt und für 30 Minuten gerührt. Falls die Reaktion bei Raumtemperatur durchgeführt werden soll, wird der Heizmantel entfernt und der Reaktor so lange an der Luft gekühlt, bis die Suspension Raumtemperatur erreicht hat. Über das Abgasventil wird auf 1.5 bar entspannt. Dann wird über den Dosiertank eine Lösung von 10 g FFOH in 20 ml Lösungsmittel rasch eindosiert. Der Reaktionsdruck wird eingestellt und durch permanentes Nachliefern von Wasserstoff aus dem Gastank konstant gehalten. Wenn der Druck im Gastank nicht mehr weiter abfällt, wird der Heizmantel entfernt und der Reaktor an der Luft auf Raumtemperatur abgekühlt. Der Reaktorinhalt wird entnommen und die Reaktionsapparatur gründlich gesäubert.

2.6 Allgemeine Versuchsvorschrift zur Umsetzung von Furfurylalkohol mit Wasserstoff in Gegenwart eines Pt(IV)-Oxid-Katalysator (Pt-JA-021) in Ethanol unter Zusatz von Salzsäure

Bei Versuchsdurchführung verläuft analog zur Durchführung ohne Zusatz von Salzsäure (Abschnitt 2.5). Anstatt die Suspension aus Lösungsmittel und Katalysator direkt im Reaktor vorzulegen, wird sie zusammen mit 1 ml 2 N Salzsäure in einen Tefloneinsatz gefüllt, welcher in den Reaktor eingebaut wird.

2.7 Allgemeine Versuchsvorschrift zur Umsetzung von Furfurylalkohol mit Wasserstoff in Gegenwart eines Pt(IV)-Oxid-Katalysator (Sigma Aldrich) in Ethanol unter Zusatz von Salzsäure

In einem Tefloneinsatz wird eine Suspension von 0.5 g Katalysator, 10 g FFOH, 91 ml Ethanol und 1 ml 2 N Salzsäure vorgelegt. Die Reaktionsapparatur wird verschlossen und es wird eine Rührgeschwindigkeit von 1000 rpm eingestellt. Der Reaktor wird dreimal mit 10 bar Argon, sowie einmal mit 10 bar Wasserstoff gespült. Über das Abgasventil

wird so lange entspannt, bis der gewünschte Reaktionsdruck erreicht ist. Durch permanentes Nachliefern von Wasserstoff aus dem Gastank wird der Druck während der Reaktion konstant gehalten. Wenn der Druck im Gastank nicht mehr weiter abfällt, wird der Heizmantel entfernt und der Reaktor an der Luft auf Raumtemperatur abgekühlt. Der Reaktorinhalt wird entnommen und die Reaktionsapparatur gründlich gesäubert.

2.8 Allgemeine Versuchsvorschrift bei Einsatz von Furan, THF und THFFOH als Edukt

Im Autoklaven wird eine Suspension aus 0.5 g 5% $Ru/Al_2O_3$ (Al1) und eine Lösung des Edukts in 100 ml Wasser vorgelegt. Die Reaktionsapparatur wird verschlossen und es wird eine Rührgeschwindigkeit von 1000 rpm eingestellt. Der Reaktor wird dreimal mit 10 bar Argon, sowie einmal mit 10 bar Wasserstoff gespült. Anschließend wird der Wasserstoffdruck so eingestellt, dass der erwartete Druck bei Erreichen der Reaktionstemperatur 10 - 20 bar unter dem Reaktionsdruck liegt. Innerhalb von 30 - 60 Minuten wird auf Reaktionstemperatur aufgeheizt. Nach Ablauf der Reaktionszeit wird der Heizmantel entfernt und der Reaktor an der Luft auf Raumtemperatur abgekühlt. Nach der Entnahme des Reaktionsgemisches wird der Reaktor gründlich gesäubert und bei 150 °C unter 30 bar Argon für 60 Minuten ausgeheizt.

2.9 Allgemeine Versuchsvorschrift zur Umsetzung von Furfurylalkohol mit Wasserstoff in Gegenwart eines Pt(IV)-Oxid-Katalysator - Versuch in Glasapparatur

Für die Hydrogenolyse mit Pt(IV)-Oxid-Katalysator (Sigma Aldrich) bei Atmosphärendruck wird eine einfache Glasapparatur verwendet.

*Versuchsaufbau*

In einem 250 ml Dreihalskolben kann die Reaktionsmischung mit Hilfe eines Magnetrührers durchmischt werden. Durch einen Rückflusskühler ist gewährleistet, dass möglichst wenig Lösungsmittel während der Reaktion freigesetzt wird. Die Temperatur kann mit einem Thermometer abgelesen werden, welches in die Reaktionslösung eintaucht. Über einen Schwebekörper-Durchflussmesser kann wahlweise Stickstoff oder Wasserstoff in die Reaktionslösung mit variabler Strömungsgeschwindigkeit eingeleitet werden.

*Versuchsdurchführung*

Eine Suspension bestehend aus 10 g FFOH, 91 ml Ethanol, 1 ml 2 N Salzsäure und 0.5 g Pt(IV)-Oxid (Sigma Aldrich) wird im Dreihalskolben vorgelegt. Unter Rühren wird 10 Minuten lang mit Stickstoff gespült. Anschließend wird Wasserstoff mit einem Volumenstrom von 270 $cm^3$ pro Minute zugeführt und 270 Minuten bei Raumtemperatur gerührt.

2.10 Allgemeine Versuchsvorschrift zur Umsetzung von Furfurylalkohol mit Wasserstoff in Gegenwart eines Pt(IV)-Oxid-Katalysator - Versuche im Multibatch

Die Versuche zum Vergleich der Katalysator-Performance unter Zusatz unterschiedlicher ionischer Flüssigkeiten wurden in einer Multibatchanlage durchgeführt.

*Versuchsaufbau*

Die Multibatchanlage umfasst fünf Reaktoren aus rostfreiem Stahl mit jeweils 40 ml Fassungsvermögen. Die Durchmischung wird mit Hilfe eines fünffachen Magnetrührwerkes gewährleistet. Jedem Reaktor ist ein 75 ml Gastank aus rostfreiem Stahl vorgeschaltet, welcher über das Gaszuleitungssystem mit Argon oder Wasserstoff $\leq$ 100 bar beschickt werden kann. Über eine thermostatgesteuerte Batterie von Heizblöcken können die einzelnen Reaktoren unabhängig voneinander beheizt werden. Die Steuerung und Kontrolle der Reaktionstemperatur erfolgte computergesteuert.

*Versuchsdurchführung*

Im Reaktor wird eine Suspension aus 4 g FFOH, 6.1 ml Wasser, 100 mg des 5 % $Ru/Al_2O_3$ Katalysators Al1 und 5 $\mu$l ionische Flüssigkeit (bzw. 2.5 mg Natriumdicyanamid) vorgelegt. Der Reaktor wird verschlossen und an die Anlage angeschlossen. Nachdem dreimal mit 30 bar Argon und einmal mit 50 bar Wasserstoff gespült wurde, werden 85 bar Wasserstoff beaufschlagt und auf 180 °C aufgeheizt. Der Druck erreicht beim Aufheizen zwischenzeitlich bis zu 90 bar und fällt zu Reaktionsende auf etwa 75 bar ab. Der Versuch wird 100 Minuten nach Erreichen der Reaktionstemperatur abgebrochen. Dazu wird die Druckleitung des Reaktors von der Anlage getrennt und der Reaktor im Wasserbad auf Raumtemperatur abgekühlt. Es wird durch vorsichtiges Öffnen des Nadelventils auf Umgebungsdruck entspannt und die Reaktionsmischung aus dem Reaktor entnommen.

2.11 Herstellung des 5 % $Ru/Al_2O_3$-Katalysators Al1

15,7 g Rutheniumnitrosylnitrat in 500 ml entionisiertem Wasser wurden zu einer Suspension von 95 g gamma-Aluminiumoxid in 1500 ml entionisiertem Wasser getropft. Der pH-Wert der Suspension wurde mit Zugabe von $Na_2CO_3$ auf pH 8.0 eingestellt. Die Suspension wurde innerhalb von 3 Stunden auf 60 °C erhitzt und anschließend bei 60 °C für eine weitere Stunde gerührt. Danach wurde eine äquimolare Menge (äquimolar bezogen auf Ruthenium) Formaldehyd als wässrige Lösung versetzt und nochmals eine Stunde gerührt. Der Katalysator wurde durch Filtration abgetrennt und im Vakuum bei 80 °C bis zur Gewichtskonstanz getrocknet. Der Rutheniumgehalt des Katalysators entsprach 5 Gew.-% an Ruthenium bezogen auf den gesamten $Ru/Al_2O_3$-Katalysator Al1. Der Gehalt an Ruthenium kann per AAS/ICP nachgeprüft werden (DIN51009).

Der erhaltene Katalysator wurde mittels Röntgenpulverdiffraktometrie untersucht. Röntgenpulverdiffraktometrie ist ein zerstörungsfreies analytisches Verfahren zur Bestimmung von kristallinen Formen (auch Phasen) in Pulvern oder Feststoffen.

Das Prinzip der Röntgenpulverdiffraktometrie ist das folgende: Trifft ein Röntgstrahl auf ein Atom, so wird er an dessen Elektronen gestreut. Liegen die Atome, wie in kristallinen Materialen, periodisch angeordnet vor, so tritt unter einem bestimmten Winkel (2θ) zwischen der Ausfalls- und Einfallsrichtung des Röntgenstrahls Interferenz ein. Diese Interferenzbedingung ist abhängig von der verwendeten Wellenlänge λ, der Röntgenstrahlung und dem Abstand d der reflektierenden Netzebenen im Gitter und wird durch die Bragg'sche Gleichung beschrieben:

$$2d\sin\theta = n\lambda$$

Die Intensität des reflektierten Röntgenstrahls wird als Funktion des Bragg'schen Streuwinkels θ gemessen. Üblicherweise wird die Intensität als Funktion von 2θ im Diffraktogramm dargestellt.

Aufgrund der regelmäßigen Anordnung der Atome auf Netzebenen in Kristallen werden in kristallinem Material diskrete Reflexe bei charakteristischen Netzebenenabständen detektiert.
Die Winkellage der Reflexe wird nur durch die Geometrie der Elementarzelle der kristallinen Phase bestimmt. Das relative Intensitätsverhältnis der beobachteten Reflexe wird dagegen durch das unterschiedliche Streuverhalten der Atome in Abhängigkeit von der Elektronendichte (Ordnungszahl) und die Lage der einzelnen Atome in der Elementarzelle moduliert.

Die Halbwertsbreite der Reflexe lässt auf die Kristallgröße der untersuchten Phase schließen.

**Probenpräparation**

Die Probe wurde wie folgt präpariert: Eine Teilmenge des angelieferten Probenmaterials (typischerweise zwischen 0,5 g und 2 g) wurde in einen 16mm-Probenträger mittels, backloading' präpariert. Dabei wird der Probenträger von der Rückseite mit der Probe befüllt um die Vorzugsorientierung zu minimieren.

Die so präparierte Probe wurde in dem Gerät PANalytical X'Pert MPD Pro unter folgenden Messparametern analysiert:

| **Parameter:** | |
|---|---|
| XRD-Gerät: | PANalytical Theta/Theta Diffraktometer |
| Röntgenröhre: | LFF-Cu-Röntgenröhre, Cu Kα, λ=0,1542 nm |
| Anregung: | 40 mA, 40 KV |
| Detektor: | X'Celerator |
| Probenhalter: | Ø 16 mm |
| Rotation: | Ja / 1 Umdrehung/s |
| 2-Theta Messbereich: | 5° - 100° |
| Step (°2Θ) | 0,017° |
| Time per step: | 40s |
| n (siehe Bragg'sche Gleichung) = 1 | |

Dabei wurden die folgenden Geräte benutzt:

**Geräte:**

PANalytical X'Pert MPD Pro
Prüfmethode: XRPD (X-ray powder diffraction)
Prüfvorschrift: SOP ROE-002

Auswertung mittels aktueller Version von PANalytical software HighScore Plus und aktueller Version von der ICDD-Datenbank mit kristallinen Referenzphasen

Es wurden die folgenden Peaks gefunden:

| Pos. [°2Th.] | Relative Intensität [%] |
|---|---|
| 18.3 | 8.69 |
| 18.8 | 13.92 |
| 20.33 | 8.8 |
| 31.37 | 51.95 |
| 32.68 | 28.16 |
| 32.825 | 82.43 |
| 35.09 | 12.34 |
| 36.758 | 56.2 |
| 38.97 | 30.89 |
| 39.8 | 32.12 |
| 40.63 | 10.43 |
| 43.359 | 16.21 |
| 44.85 | 64.42 |
| 45.47 | 47.23 |
| 46.54 | 28.33 |
| 47.61 | 32.26 |
| 50.77 | 11.05 |
| 51.5 | 9.98 |
| 57.48 | 8.68 |
| 59.97 | 17.46 |
| 62.38 | 11.05 |
| 62.848 | 10.72 |
| 64.05 | 13.91 |
| 65.7 | 12.04 |
| 66.52 | 56.88 |
| 67 | 34.05 |
| 67.4 | 100 |
| 73.5 | 6.88 |

Der 5 % Ru/$Al_2O_3$-Katalysators Al1 ist mesoporös und hat einen Porendurchmesser, der im Bereich von 2 nm bis 50 nm liegt, bestimmt gemäß DIN ISO 9277.

Die BET-Gesamtoberfläche des 5 % Ru/$Al_2O_3$-Katalysators Al1 liegt zwischen 100 und 150 $m^2$/g Katalysator. Die BET-Gesamtoberfläche wird dabei nach DIN ISO 9277 bestimmt.

Das Porenvolumen des 5 % Ru/$Al_2O_3$-Katalysators Al1 liegt bei 0.4 ml/g Katalysator. Das Porenvolumen wird

dabei nach DIN ISO 9277 bestimmt.

2.12 Herstellung des 5 % Ru/AC-Katalysators Al2 sowie des 5% Ru/C Katalysators Al3

Der 5 % Ru/AC-Katalysators Al2 sowie des 5 % Ru/C Katalysators Al3 wurden analog wie für den Katalysator Al1 beschrieben hergestellt (Abschnitt 2.11), mit dem Unterschied, dass anstatt $Al_2O_3$ die entsprechende Menge Aktivkohle als Träger eingesetzt wurde und keine Trocknung stattfindet im Anschluss an die Filtration bevor der Katalysator benutzt wird. Der Gehalt an Ruthenium kann per AAS/ICP nachgeprüft werden nach Trocknung bei 80 °C im Vakuum bis zur Gewichtskonstanz (DIN51009).

2.13 Herstellung der [5:2] Ru-Zn, [5:2] Ru-Fe, [1:2] Ru-Cu und [1:2] Ru-Ni Katalysatoren auf $Al_2O_3$

Die Präparation der Ru-Zn, Ru-Fe, Ru-Cu und Ru-Ni Katalysatoren auf Aluminiumoxid erfolgt durch Imprägnierung nach der incipient wetness Methode. Dazu werden 0.9 ml einer Lösung der Metallvorläuferverbindung in Wasser auf 1 g des 5% Ru/$Al_2O_3$-Katalysators Al1 getropft. Der feuchte Katalysator wird über Nacht bei 85 °C im Trockenschrank getrocknet. Die Kalzinierung erfolgt im Formierofen bei 130 $cm^3$/min Luftstrom, wobei 1 h lang auf 200 °C aufgeheizt und diese Temperatur 1 h lang konstant gehalten wird. Das Abkühlen auf Raumtemperatur erfolgt an der Luft bei gleichbleibendem Volumenstrom. Die Reduktion wird bei 270 $cm^3$/min Wasserstoffstrom durch einstündiges Aufheizen auf 250 °C und anschließendes Konstanthalten der Temperatur für 1 h durchgeführt. Der Formierreaktor wird an der Luft unter Wasserstoffstrom auf 100 °C und anschließend unter Stickstoffstrom auf Raumtemperatur abgekühlt. Die eingesetzten Mengen der einzelnen Metallvorläuferverbindungen sind in Tabelle 8 aufgeführt.

Tabelle 8: Eingesetzte Mengen der Metallvorläuferverbindungen zur Präparation bimetallischer Katalysatoren.

| **Zweitmetall** | **Vorläuferverbindung** | **Menge** |
|---|---|---|
| Zn | Zink(II)-nitrathexahydrat | 59.5 mg |
| Fe | Eisen(III)-nitratnonahydrat | 77.2 mg |
| Cu | Kupfer(II)-nitrattrihydrat | 239.2 mg |
| Ni | Nickel(II)-nitrathexahydrat | 287.9 mg |

2.14 Herstellung des [1:2]Ru-Pt Katalysators auf $Al_2O_3$

Der Ru-Pt/$Al_2O_3$ Katalysator wird durch Imprägnierung aus überstehender Lösung der Metallvorläuferverbindung hergestellt. Dazu wird eine Lösung von 383 mg Tetraaminplatin(II)-nitrat in 2.7 ml Wasser zu 1 g des 5% Ru/$Al_2O_3$ Katalysators Al1 getropft. Die Suspension wird 2 Tage lang in einem geschlossenen Gefäß aufbewahrt. Nach dem Abdekantieren der überstehenden Lösung wird der feuchte Katalysator über Nacht bei 85 °C im Trockenschrank getrocknet. Kalzinierung und Reduktion erfolgen analog zu den anderen bimetallischen Katalysatoren.

2.15 Herstellung des [17:2] Ru-Sn Katalysators auf $Al_2O_3$

Der Ru-Sn/$Al_2O_3$ Katalysator wird in situ erzeugt, indem im Autoklaven zusammen mit der Suspension des Katalysators 10 mg Sn(II)-chlorid vorgelegt werden. Die Versuchsdurchführung erfolgt gemäß der allgemeinen Vorschrift unter Abschnitt 2.1.

**3. Analytik**

Von jedem durchgeführten Hydrogenolyseversuch wurde nach Beendigung der Reaktion eine Ausbauprobe entnommen. Diese wurde filtriert und das Filtrat 1 : 10 mit Wasser verdünnt. Bei Einsatz hoher FFOH-Anfangskonzentrationen ≥ 200 g/l wurde stattdessen 1 : 20 verdünnt. Die verdünnten Proben wurden per GC und GC-MS analysiert, um die Reaktionsprodukte zu identifizieren sowie Umsatz und Selektivitäten zu bestimmen.

Zur Identifikation der Reaktionsprodukte wurde eine *GCMS-QP2010 SE* mit Gaschromatograph *GC-20120 Plus* der Firma *SHIMADZU* verwendet. Als stationäre Phase diente eine DB-Wax-Säule mit den in Tabelle 9 aufgeführten Spezifikationen.

Tabelle 9: Spezifikationen der verwendeten Säule und Analysemethode bei GC- und GC-MS-Untersuchungen.

| Parameter | Wert |
|---|---|
| Länge | 30 m |
| Durchmesser | 0.25 mm |
| Filmdicke | 0.25 μm |
| Starttemperatur | 50 °C |
| Heizrate | 10 °C / min |
| Endtemperatur | 220 °C |
| Hold-Time | 8 min |
| Eluent | Helium |
| Eluentstrom | 1 ml / min |

Zur Bestimmung von Umsätzen und Selektivitäten wurde ein Gaschromatograph HP 6890 *GC System* der Firma *Hewlett Packard* verwendet. Säulen- und Methodenparameter sind identisch mit den in Tabelle 9 aufgeführten Parametern für die GC-MS-Untersuchungen. Zur Signaldetektion kam ein Flammenionisationsdetektor zum Einsatz.

Zur Identifikation der detektierten Signale wurden wässrige Lösungen von Furfurylalkohol und den wichtigsten erwarteten Reaktionsprodukten untersucht und zur eindeutigen Zuordnung mit GC-MS-Ergebnissen abgeglichen. Zur quantitativen Kalibrierung wurden wässrige Maßlösungen der jeweiligen Verbindung im Konzentrationsbereich von 0.5 g/l bis 10 g/l verwendet. Bei Verbindungen, für die keine quantitative Kalibrierung durchgeführt wurde, wurden Kalibrierfaktoren von 0.05 mmol/l pro Peakfläche angenommen. In Tabelle 10 sind die gemessenen Retentionszeiten und Kalibrierfaktoren zusammengefasst.

Tabelle 10: Retentionszeiten und Kalibrierfaktoren

| Verbindung | Retentionszeit [min] | Kalibrierfaktor [mmol/l pro Peakfläche] |
|---|---|---|
| THF | 3.011 | nicht bestimmt |
| 2-Methyltetrahydrofuran | 3.087 | nicht bestimmt |
| 2-Methylfuran | 3.155 | nicht bestimmt |
| 2-Pentanol | 5.198 | nicht bestimmt |
| 1-Butanol | 5.438 | 0.0645 |
| Cyclopentanon | 5.990 | 0.0664 |
| 1-Pentanol | 6.667 | nicht bestimmt |
| Cyclopentanol | 7.330 | 0.0598 |
| Cyclopentenon | 8.034 | nicht bestimmt |
| Tetrahydrofurfurylalkohol | 9.815 | 0.0468 |
| Furfurylalkohol | 11.478 | 0.0565 |
| 1,2-Butandiol | 11.722 | 0.0649 |
| 2*S*,4*R*-Pentandiol | 11.838 | nicht bestimmt |
| 1,2-Pentandiol | 12.819 | 0.0504 |
| 1,4-Pentandiol | 13.885 | nicht bestimmt |
| 1,5-Hexandiol | 14.939 | nicht bestimmt |
| 1,5-Pentandiol | 15.378 | 0.0455 |
| 4-Hydroxy-2-cyclopentenon | 16.376 | nicht bestimmt |

## 4. Ergebnisse

Auswertung von Katalysatorsystemen hinsichtlich des Umsatzes und der Selektivität von Furfurylalkohol zu 1,2-Pentandiol

Es wurden unterschiedliche Katalysatorsysteme in Hinblick auf Umsatz von Furfurylalkohol und Selektivität zu 1,2-Pentandiol untersucht. Zum einen kamen Ruthenium und Platin-Trägerkatalysatoren zum Einsatz, welche zum Erzielen guter Aktivität und Selektivität hohe Drücke und Temperaturen benötigen. Für den selektivsten dieser Katalysatoren wurden weitere Experimente unter Variation der Reaktionsbedingungen und unter Zusatz diverser Zweitmetalle und ionischer Flüssigkeiten durchgeführt. Zum anderen wurden Platin(IV)-Oxid-Katalysatoren untersucht, die bereits bei wesentlich milderen Bedingungen in organischer Lösung aktiv sind. Umsätze und Selektivitäten wurden mit Hilfe von GC-Analysen der Ausbauproben bestimmt und beziehen sich auf die Stoffmengen in der flüssigen Phase, welche mit Hilfe der experimentell bestimmten Kalibrierfaktoren (Tabelle 10) aus den Peakflächen berechnet wurden. Die Versuchsergebnisse sind im Anhang in tabellarischer Form zusammengefasst.

Katalysatorscreening

In 100 ml einer wässrigen Lösung von Furfurylalkohol wurden die in Tabelle 11 gezeigten 6 Ru-Katalysatoren sowie 2 Pt-Katalysatoren bei 200 °C und 100 bar Wasserstoffdruck im Batchreaktor getestet. Dabei wurden je 0.5 g Katalysator und die genannten Zusätze eingesetzt eingesetzt. Die Reaktion wurde abgebrochen, sobald ein vollständiger Umsatz von Furfurylalkohol erreicht war. In Tabelle 11 sind die Reaktionszeiten für vollständigen Umsatz und die Selektivitäten zu den wichtigsten Reaktionsprodukten für die 8 untersuchten Katalysatoren zusammengefasst.

Dabei ist festzustellen, dass alle in Tabelle 11 gezeigten $Al_2O_3$-geträgerten Rutheniumkatalysatoren mit einer überraschend hohen Selektivität zum Produkt 1,2-Pentandiol reagierten. Die Selektivität unter den gewählten Bedingungen war in jedem Fall höher als etwa die von Zhang *et al.* für $Ru/AlMgO_4$ beschrieben [49]. Die beste Selektivität zu 1,2-Pentandiol lieferte 5 % $Ru/Al_2O_3$ (Al1) mit 32 %. Wie aus Tabelle 12 ersichtlich, wird eine hohe Selektivität auch ohne Zusatz von $Na_2CO_3$ beobachtet. Somit erreichten die Rutheniumkatalysatoren in der Regel noch bessere Selektivitäten als die Platinkatalysatoren. Zusätzlich dazu wurden auch hohe Ausbeuten an THFFOH beobachtet. Diese konnten bei einem insgesamt vollständigen Umsatz des Ausgangsprodukts Furfurylalkohol erreicht werden, und stellt somit ein überraschend vorteilhaftes Ergebnis im Vergleich zu den Ergebnissen des Standes der Technik, insbesondere [49], dar.

Weiterhin überraschend war, dass für kohlenstoffgeträgerte Rutheniumkatalysatoren die Selektivität für 1,2-Pentandiol bei 20 bzw. 21 % lag, somit um das hundertfache höher als für den von Zhang *et al.* beschriebenen vergleichbaren Katalysator [49].

Sogar mit 1 % Ru/Glas (FS384) konnte eine Selektivität von 14 % beobachtet werden. Zudem zeichneten sich die Rutheniumkatalysatoren durch höhere Aktivität aus, wobei auch hier 1 % Ru/Glas die Ausnahme darstellte. Mit allen getesteten Katalysatoren war die Bildung von 1,2-Pentandiol (1,2-PD) gegenüber 1,5-Pentandiol (1,5-PD) stark begünstigt. Das Hauptprodukt stellte Tetrahydrofurfurylalkohol (THFFOH) dar, welches je nach eingesetztem Katalysator in 1.5- bis 3-fachem Stoffmengenverhältnis gegenüber 1,2-Pentandiol entstand. Außerdem traten als Nebenprodukte 1-Pentanol (1-POH), 1,2-Butandiol (1,2-BD), 1,4-Pentandiol (1,4-PD), Cyclopentanon (CpO), Cyclopentanol (CpOH), 2-Methyltetrahydrofuran (2-MTHF), 1-Butanol (1-BOH), vermutlich 1,5-Hexandiol (1,5-HD) sowie weitere unbekannte Verbindungen auf.

Tabelle 11: Reaktionszeiten und Selektivitäten *bei vollständigem Umsatz* von Furfurylalkohol in wässriger Lösung. Reaktionsbedingungen: m(Kat) = 0.5 g, T = 200 °C, $p(H_2)$ = 100 bar, Rührgeschwindigkeit = 1000 rpm, [a] c(FFOH) = 40 g / 100 ml, [b] c(FFOH) = 7.46 g / 100 ml, [c] 100 mg $Na_2CO_3$, [d] 300 µl ges. $Na_2CO_3$-Lösung.

| Katalysator | t [min] | S (1,2-PD) [%] | S (1,5-PD) [%] | S (THFFOH) [%] | S (1,2-BD) [%] | S (CpOH) [%] |
|---|---|---|---|---|---|---|
| 5% $Ru/Al_2O_3$ (Al1) [a, c] | 60 | 32 | 1 | 59 | 2 | 1 |
| 5 % Ru/C (Al2) [b] | 45 | 21 | 1 | 60 | 10 | 0 |
| 5 % Ru/C (Al3) [b] | 45 | 20 | 1 | 49 | 7 | 0 |

(fortgesetzt)

| Katalysator | t [min] | S (1,2-PD) [%] | S (1,5-PD) [%] | S (THFF OH) [%] | S (1,2-BD) [%] | S (CpOH) [%] |
|---|---|---|---|---|---|---|
| 1 % $Ru/Al_2O_3$ (RR506) [b, c] | 40 | 26 | 1 | 60 | 1 | 1 |
| 1 % $Ru/Al_2O_3$ (Y40753-BS) [b, c] | 60 | 30 | 2 | 61 | 2 | 1 |
| 10% Ru/Glas (FS384) [a, c] | 200 | 14 | 1 | 32 | 1 | 10 |
| Pt/PANI (Pt-JA-024) [b, d] | 240 | 14 | 2 | 22 | 0 | 3 |
| 10 % Pt/C (Pt-JA-026) [b, c] | 180 | 20 | 8 | 34 | 0 | 2 |

Es wurde versucht, ein umfassendes Reaktionsschema (Schema 3) aufzustellen, das die Entstehung der identifizierten Reaktionsprodukte aufzeigt. Zu diesem Zweck wurde mit Erkenntnissen aus der Literatur verglichen, sowie Experimente zur Absicherung einzelner Reaktionspfade durchgeführt.

Von Xu *et al.* [23] wurde ein Reaktionsschema (Schema 1) zur Umsetzung von Furfural an Platin-Trägerkatalysatoren erarbeitet, welches mit den Ergebnissen dieser Arbeit für Platin- und Ruthenium-Trägerkatalysatoren in Einklang steht und die Entstehung vieler der nachgewiesenen Produkte erklärt. Demnach reagiert Furfurylalkohol über folgende Reaktionspfade:

1. Die Spaltung einer C-O-Bindung des Furanringes führt zur Bildung von 1,2-Pentandiol **6** oder 1,5-Pentandiol **8**. 1,2-Pentandiol kann dabei zu 1,4-Pentandiol **7** isomerisieren.
2. Durch Abspaltung der Hydroxylgruppe entsteht 2-Methylfuran **1.** Dieses kann durch Hydrierung des Furanringes zu 2-Methyltetrahydrofuran **2** oder durch Hydrogenolyse der C-O-Bindung zu 1-Pentanol **4** reagieren.
3. Durch Hydrierung des Furanringes entsteht Tetrahydrofurfurylalkohol **5.**

Die Hydrogenolyse von Tetrahydrofurfurylalkohol zu 1,2- oder 1,5-Pentandiol war bei den von Xu *et al.* verwendeten Reaktionsbedingungen von $p(H_2)$ = 15 bar und T = 130 °C vernachlässigbar. Nach Tomishige *et al.* wurden mit Ru/C bei T = 120 °C und $p(H_2)$ = 80 bar selbst nach 4 h Reaktionszeit lediglich 5 % Umsatz an THFFOH erzielt. [33] Da in dieser Arbeit jedoch mit deutlich höheren Temperaturen und Wasserstoffdrücken gearbeitet wurde, wurde ein Experiment mit THFFOH als Edukt durchgeführt, um herauszufinden, ob die Hydrogenolyse von THFFOH auch bei schärferen Reaktionsbedingungen vernachlässigbar ist. Dazu wurde der aktivste Katalysator, 5 % $Ru/Al_2O_3$ (Al1), bei einem Wasserstoffdruck von 100 bar und einer Reaktionstemperatur von 240 °C verwendet. Es zeigte sich, dass nach 15 Minuten Reaktionszeit nur 24 % des THFFOH umgesetzt wurden. Als Hauptprodukt wurde 1-BOH mit 36 % Selektivität gebildet. 1,2-Pentandiol und 1,5-Pentandiol entstanden nur in geringen Mengen mit Selektivitäten von 6 bzw. 3 %. Daraus ist zu schließen, dass 1,2- und 1,5-Pentandiol fast ausschließlich durch Hydrogenolyse von FFOH entstehen, während die Hydrogenolyse von THFFOH nur eine untergeordnete Rolle spielt. Die Bildung von 1-Pentanol aus 2-Methylfuran verläuft nach Xu *et al.* analog zur Entstehung von 1,2-Pentandiol aus FFOH durch Spaltung des Furanringes an der gleichen C-O-Bindung. Die Entstehung von 1-Butanol **10** verläuft vermutlich über Furan **9** als Zwischenprodukt. Aus der Literatur ist bekannt, dass Furan durch Decarboxylierung von Furfurylalkohol bei hohen Temperaturen unter Abspaltung von Wasserstoff und Kohlenmonoxid gebildet werden kann, beispielsweise unter Verwendung von Kupferkatalysatoren. [45] Um nachzuprüfen, ob die Bildung von 1-Butanol tatsächlich durch Hydrogenolyse von Furan zu erklären ist, wurde Furan in wässriger Lösung an 5 % $Ru/Al_2O_3$ (Al1) bei 175 °C und $p(H_2)$ = 100 bar umgesetzt. Nach 30 Minuten Reaktionszeit entstand bei 100 % Umsatz mit 15 % Selektivität 1-Butanol. Das Hauptprodukt war mit 54 % Selektivität Tetrahydrofuran (THF). Um festzustellen, ob dieses ein Zwischenprodukt darstellt, wurde THF in wässriger Lösung am gleichen Katalysator bei 240 °C und $p(H_2)$ = 100 bar umgesetzt. Nach 15 Minuten wurde nur ein geringer Umsatz von 14 % beobachtet und die Ausbeute an 1-Butanol betrug 3 %. Die Beobachtung, dass THF selbst bei 240 °C relativ stabil ist, während Furan bereits bei 175 °C mit einer fünfmal so hohen Ausbeute zu 1-Butanol reagiert, lässt darauf schließen, dass 1-Butanol hauptsächlich durch Hydrogenolyse von Furan entsteht. Diese Erkenntnis folgt dem beobachteten Trend, dass die gesättigten Ringe (THFFOH, 2-MTHF, THF) gegenüber einer Spaltung der C-O-Bindung an den verwendeten Ru- und Pt-Trägerkatalysatoren verhältnismäßig unreaktiv sind.

Cyclopentanon **13** und Cyclopentanol **14** traten als Nebenprodukte mit Selektivitäten von bis zu 17 bzw. 10 % auf. In der Literatur ist die Umsetzung von Furfural in wässriger Lösung an Ru- und Pt-Katalysatoren zu diesen beiden Produkten unter ähnlichen Reaktionsbedingungen bekannt. [46] Hronec und Fulajtarova erhielten mit Ru/C bei 175 °C Reaktionstemperatur und 80 bar Wasserstoffdruck ein Produktgemisch, in dem Cyclopentanon als Hauptprodukt (Y = 57.33 %) und Cyclopentanol als Nebenprodukt (Y = 9.50 %) auftraten. Die Anwesenheit von Wasser spielt bei dieser Reaktion eine entscheidende Rolle. Dies ist vermutlich darauf zurückzuführen, dass als Zwischenschritt eine Isomerisierung von Furfurylalkohol zu 4-Hydroxy-2-cyclopentenon **11** stattfindet. Aus der Literatur ist bekannt, dass diese Reaktion beim Erhitzen einiger wässrigen Lösung von Furfurylalkohol auf 200 °C ohne Einsatz eines Katalysators zu beobachten ist. [47] Um dies zu überprüfen, wurde ein Versuch ohne Katalysator unter üblichen Reaktionsbedingungen von 200 °C und 100 bar Wasserstoffdruck durchgeführt. Dabei wurden nach 120 Minuten Reaktionszeit die Produkte 4-Hydroxy-2-cyclopentenon **11,** 2-Cyclopentenon **12** und Cyclopentanon **13** via GC-MS nachgewiesen. Diese Beobachtung legt die Vermutung nahe, dass Furfurylalkohol unter diesen Bedingungen zunächst zu 4-Hydroxy-2-cyclopentenon isomerisiert und anschließend sukzessiv zu Cyclopentenon und Cyclopentanon reduziert wird. In Anwesenheit eines Hydrierkatalysators wie Ru oder Pt findet schließlich die Reduktion von Cyclopentanon zu Cyclopentanol statt. Da dieser Reaktionspfad als ersten Schritt eine Isomerisierung aufweist, welche unabhängig vom eingesetzten Katalysator auftritt, sollte er umso mehr an Bedeutung gewinnen, je geringer die Aktivität des Katalysators ist. Tatsächlich wies Pt/PANI (Pt-JA-024) - der Katalysator mit der geringsten Aktivität - mit 20 % die größte gemeinsame Selektivität zu Cyclopentanon (S = 17 %) und Cyclopentanol (S = 3 %) auf.

Häufig wurde beobachtet, dass die Gesamtstoffmenge aller nachgewiesenen Produkte nicht mit der Stoffmenge des eingesetzten Edukts übereinstimmte. Um dieser Tatsache Rechnung zu tragen, wurde die Bilanz $\Sigma$ eingeführt. Sie wird berechnet, indem die Summe der Stoffmengen aller nachgewiesenen Stoffe (einschließlich Edukt) in der Ausbauprobe durch die Stoffmenge des eingesetzten Edukts geteilt wird. Vor allem bei langen Reaktionszeiten oder hohen Temperaturen wurden häufig Werte für $\Sigma$ berechnet, die deutlich unter 100 % lagen. Dies lässt darauf schließen, dass Reaktionsprodukte entstehen, die nicht über die angewendete gaschromatographische Analyse nachweisbar sind. Vermutlich handelt es sich dabei um Polyfurfurylalkohol, welcher in wässriger Lösung aus Furfurylalkohol entsteht. [48] Sowohl die Polymerisation von Furfurylalkohol als auch die Isomerisierung zu 4-Hydroxy-2-cyclopentenon finden bevorzugt unter sauren Bedingungen statt. Dies ist problematisch, da während der Reaktion in allen Experimenten ein Abfallen des pH-Wertes beobachtet wurde, insbesondere bei langen Reaktionszeiten. So sank beim Leerversuch ohne Katalysator der pH-Wert nach 120 Minuten Reaktionszeit von 7 auf 2.4 ab. Die Polymerisation gewinnt, da sie ohne Hydrierkatalysator auftritt, umso mehr an Bedeutung, je weniger aktiv der verwendete Katalysator ist.

Die Entstehung von 1,2-Butandiol kann anhand des aufgestellten Reaktionsschemas nicht erklärt werden kann. Eine weitere Verbindung, die in einigen Versuchen mit kleinen Ausbeuten auftrat, wurde via GC-MS als 1,5-Hexandiol identifiziert. Es ist jedoch fragwürdig, ob diese Zuordnung korrekt ist, da bisher kein Mechanismus für die Einführung des sechsten C-Atoms unter diesen Bedingungen bekannt ist.

Schema 3: Reaktionsschema für die Umsetzung von Furfurylalkohol an Ru- und Pt-Trägerkatalysatoren.

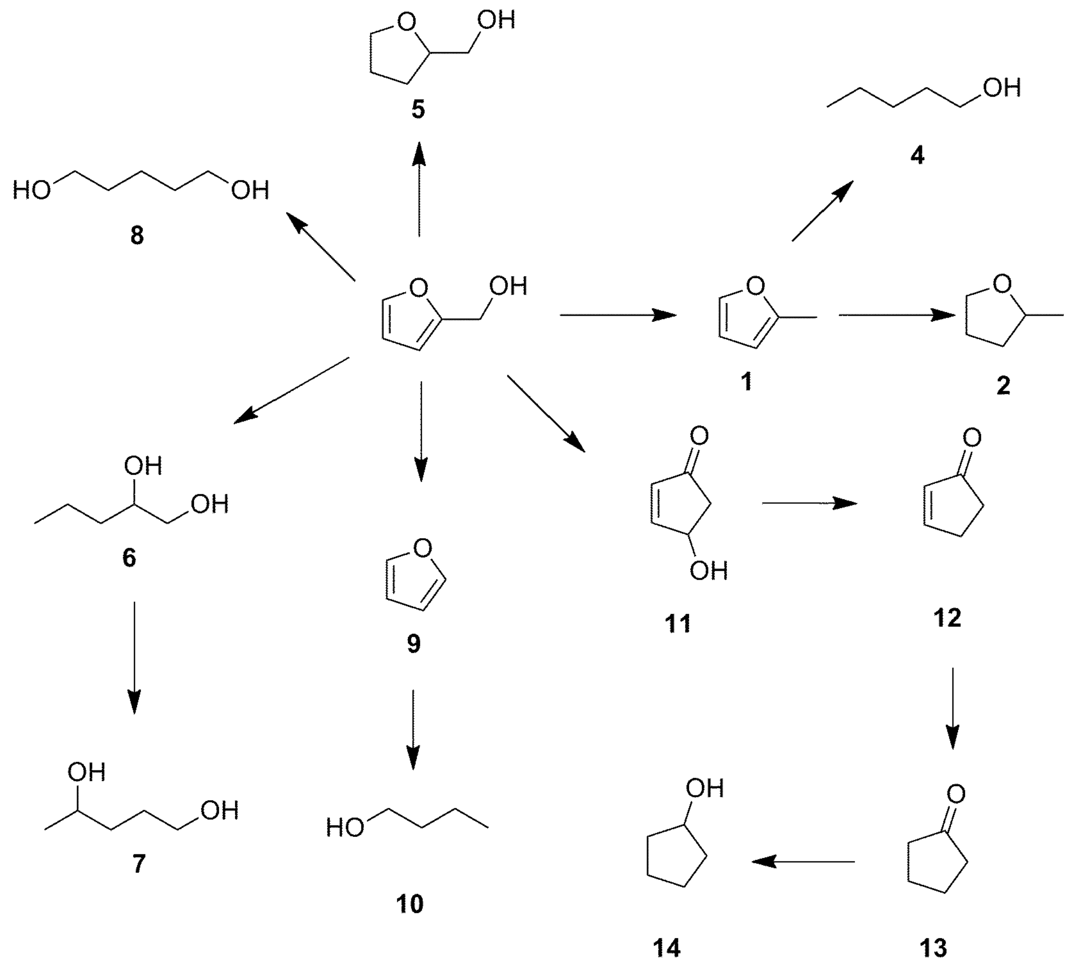

5 4 8 1 2 6 11 12 9 7 10 14 13

2. **Beispiele 1 - 3, 3***: Herstellung von 1,2-Pentandiol mit einem $Ru/Al_2O_3$-Katalysator (erfindungsgemäß)

Die Umsetzung wurde in einem Rührautoklav (*Parr Instruments)* bestehend aus rostfreiem Stahl mit 300 ml Fassungsvermögen durchgeführt. Über einen Druckminderer ist ein 500 ml Gastank an den Reaktor angeschlossen, welcher über ein Gaszuleitungssystem sowohl mit Argon (≤ 80 bar) als auch Wasserstoff (≤ 200 bar) beschickt werden kann. Ein Abgasventil dient zur Entspannung des Reaktors nach Versuchsende. Zur Durchmischung wird ein Gaseintragsrührer mit einer maximalen Rührgeschwindigkeit von 1600 rpm verwendet. Zum Aufheizen der Reaktionsmischung dient ein thermostatgesteuerter Heizmantel, mit welchem bis zu 280 °C eingestellt werden können. Über einen Entnahmehahn können während der Reaktion Proben entnommen werden. Des Weiteren ist zum Einfüllen des Edukts ein Dosiertank an den Reaktor angeschlossen, welcher ebenfalls über den Gastank mit Wasserstoff und Argon beschickt werden kann. Der Druck wird über ein Manometer und die Temperatur in Gas- und Flüssigphase getrennt über Thermoelemente gemessen. Druck- und Temperaturverläufe werden digital gespeichert und gesteuert.

Im Autoklaven wurden 86.8 ml Wasser und 0.5 g des 5% $Ru/Al_2O_3$ - Katalysators Al1 vorgelegt. Die Reaktionsapparatur wurde verschlossen und es wurde eine Rührgeschwindigkeit von 1000 rpm eingestellt. Der Reaktor wurde dreimal mit 10 bar Argon, sowie einmal mit 10 bar Wasserstoff gespült. Anschließend wurde der Wasserstoffdruck so eingestellt, dass der erwartete Druck bei Erreichen der Reaktionstemperatur 10 - 20 bar unter dem Reaktionsdruck von 100 bar liegen würde. Innerhalb von 30 - 60 Minuten wurde auf Reaktionstemperatur aufgeheizt. Die Reaktionstemperatur betrug im Falle von Beispiel 1 150°C, im Falle von Beispiel 2 200°C, im Falle von Beispiel 3 240°C, im Falle von Beispiel 3* 260°C. Sobald diese erreicht war, wurde über den Dosiertank 7.46 g Furfurylalkohol in 6.6 ml Wasser rasch eindosiert. Über den Gastank wurde permanent Wasserstoff nachgeliefert und der Reaktionsdruck konstant gehalten. Zur Probenentnahme wurde die Entnahmeleitung vorher mit 1 - 2 ml Reaktionsmischung gespült und dann 0.5 - 1 ml Probe entnommen. Wenn der Wasserstoffdruck im Gastank nicht mehr weiter absank, wurde der Heizmantel entfernt und der Reaktor an der Luft auf Raumtemperatur abgekühlt. Nach der Entnahme des Reaktionsgemisches wurde der Reaktor gründlich gesäubert und bei 150 °C unter 30 bar Argon für 60 Minuten ausgeheizt.

Das Produktgemisch wurde analysiert und es wurden die folgenden Selektivitäten bei den verschiedenen Temperaturen festgestellt (siehe Tabelle 12).

Tabelle 12: Selektivitäten *bei vollständigem Umsatz* von Furfurylalkohol in wässriger Lösung bei unterschiedlichen Temperaturen mit 5 % $Ru/Al_2O_3$. Reaktionsbedingungen: m(Kat) = 0.5 g, $p(H_2)$ = 100 bar, Rührgeschwindigkeit = 1000 rpm, c(FFOH) = 7.46 g / 100 ml, * 300 mg $Na_2CO_3$.

| **Beispiel** | **T [°C]** | **t [min]** | **S(1,2-PD) [%]** | **S(THFFOH) [%]** | **S(1,2-PD) / S(THFFOH)** | **S(CpOH) [%]** | **Σ [%]** |
|---|---|---|---|---|---|---|---|
| 1 | 150 | 60 | 14 | 73 | 0.19 | 0 | 88 |
| 2 | 200 | 30 | 26 | 53 | 0.49 | 0 | 90 |
| 3 | 240 | 15 | 27 | 25 | 1.08 | 4 | 74 |
| 3* | 260 | 25 | 13 | 9 | 1.44 | 14 | 54 |

Wie aus der Tabelle ersichtlich, liegt das Verhältnis von 1,2-Pentandiol zu 1,5-Tetrahdrofurfurylalkohol überraschenderweise schon bei einer Temperatur von 150 °C bei 0.19 und damit über dem im Stand der Technik beschriebenen Verhältnis. Noch viel überraschender war, dass schon nach spätestens 60 min ein Vollumsatz erreicht wurde, wobei die Selektivität zu 1,2-Pentandiol bei 150 °C schon bei 14 % liegt, bei 200 °C und 240 °C sogar bei 26 bzw. 27 %. Diese optimale Temperatur scheint unter diesen Reaktionsbedingungen demnach bei 200 - 240 °C zu liegen.

**<u>Beispiele 4 - 8 (erfindungsgemäß)</u>:**

Es wurde nun überraschenderweise gefunden, dass die Selektivität weiter erhöht werden konnte, wenn die Hydrogenolyse unter Zusatz kleiner Mengen gesättigter $Na_2CO_3$-Lösung oder festem $Na_2CO_3$ durchgeführt wurde.

Im Einzelnen wurden im Autoklaven 86.8 ml Wasser, 0.5 g des 5 % $Ru/Al_2O_3$ - Katalysators Al1 sowie die in Tabelle 13 angegebene Menge $Na_2CO_3$ (0 mg, 10 mg, 30 mg, 60 mg, 300 mg) vorgelegt. Die Reaktionsapparatur wurde verschlossen und es wurde eine Rührgeschwindigkeit von 1000 rpm eingestellt. Der Reaktor wurde dreimal mit 10 bar Argon, sowie einmal mit 10 bar Wasserstoff gespült. Anschließend wurde der Wasserstoffdruck so eingestellt, dass der erwartete Druck bei Erreichen der Reaktionstemperatur 10 - 20 bar unter dem Reaktionsdruck von 100 bar liegen würde. Innerhalb von 30 - 60 Minuten wurde auf Reaktionstemperatur aufgeheizt. Die Reaktionstemperatur betrug 240 °C. Sobald diese erreicht war, wurde über den Dosiertank 7.46 g Furfurylalkohol in 6.6 ml Wasser rasch eindosiert. Über den Gastank wurde permanent Wasserstoff nachgeliefert und der Reaktionsdruck konstant gehalten. Zur Probenentnahme wurde die Entnahmeleitung vorher mit 1 - 2 ml Reaktionsmischung gespült und dann 0.5 - 1 ml Probe entnommen. Wenn der Wasserstoffdruck im Gastank nicht mehr weiter absank, das war im Falle der Beispiele 4 - 7 nach 15 Minuten der Fall, im Falle des Beispiels 8 nach 25 Minuten der Fall, wurde der Heizmantel entfernt und der Reaktor an der Luft auf Raumtemperatur abgekühlt. Nach der Entnahme des Reaktionsgemisches wurde der Reaktor gründlich gesäubert und bei 150 °C unter 30 bar Argon für 60 Minuten ausgeheizt.

Das Produktgemisch wurde analysiert und es wurden die folgenden Selektivitäten bei den verschiedenen Temperaturen festgestellt:

Tabelle 13: Selektivitäten *bei vollständigem Umsatz* von Furfurylalkohol in wässriger Lösung unter Zusatz unterschiedlicher Mengen gesättigter $Na_2CO_3$-Lösung mit 5 % $Ru/Al_2O_3$ (Al1). Reaktionsbedingungen: m(Kat) = 0.5 g, T = 240 °C, $p(H_2)$ = 100 bar, Rührgeschwindigkeit = 1000 rpm, c(FFOH) = 7.46 g / 100 ml, t = 15 Minuten bzw. bei Beispiel 8 25 min.

| **Beispiel** | **m($Na_2CO_3$) [mg]** | **pH (nach Reaktion)** | **S(1,2-PD) [%]** | **S(CpOH) [%]** | **Σ [%]** |
|---|---|---|---|---|---|
| 4 | 0 | 5.3 | 27 | 4 | 74 |
| 5 | 10 | 6.2 | 32 | 7 | 87 |
| 6 | 30 | 7.6 | 35 | 5 | 91 |
| 7 | 60 | 10.2 | 30 | 4 | 80 |

(fortgesetzt)

| Beispiel | m($Na_2CO_3$) [mg] | pH (nach Reaktion) | S(1,2-PD) [%] | S(CpOH) [%] | Σ [%] |
|---|---|---|---|---|---|
| 8 | 300 | 10.5 | 25 | 3 | 67 |

Wie aus der Tabelle ersichtlich, nimmt die Selektivität zu 1,2-Pentandiol zunächst mit der Menge der eingesetzten Base zu. Bei Zusatz von 150 µl gesättigter $Na_2CO_3$-Lösung (entspricht 30 mg $Na_2CO_3$) lag die Selektivität mit 35 % um 8 % höher als ohne Zusatz von Base. Der nach der Reaktion gemessene pH-Wert im Reaktionsaustrag ist dabei mit 7.6 leicht basisch. Werden 300 µl $Na_2CO_3$-Lösung (entspricht 60 mg $Na_2CO_3$) verwendet, nimmt die Selektivität zu 1,2-Pentandiol wieder ab. Bei Einsatz von 300 mg $Na_2CO_3$ in fester Form ist die Selektivität mit 25 % schließlich niedriger als wenn ohne Zusatz von Base gearbeitet wird. Dieser pH-Effekt war völlig überraschend. Vor allem war es hinsichtlich des Standes der Technik überraschend, dass die Selektivität in einem bestimmten pH-Bereich einen solch deutlichen Anstieg aufwies [47], [48].

**Beispiele 9 und 10:**

Zur Bestätigung dieses Effekts wurde ein weiterer entsprechender Versuch durchgeführt, in welchem Natriumacetat als Base eingesetzt wurde (Beispiele 9 und 10). Im Einzelnen wurden im Autoklaven 64.4 ml Wasser, 1.0 g des 5 % $Ru/Al_2O_3$ - Katalysators Al1 sowie 100 mg $Na_2CO_3$ (Beispiel 9) bzw. 5 g Natriumacetat (Beispiel 10) vorgelegt. Die Reaktionsapparatur wurde verschlossen und es wurde eine Rührgeschwindigkeit von 1000 rpm eingestellt. Der Reaktor wurde dreimal mit 10 bar Argon, sowie einmal mit 10 bar Wasserstoff gespült. Anschließend wurde der Wasserstoffdruck so eingestellt, dass der erwartete Druck bei Erreichen der Reaktionstemperatur 10 - 20 bar unter dem Reaktionsdruck von 100 bar liegen würde. Innerhalb von 30 - 60 Minuten wurde auf Reaktionstemperatur aufgeheizt. Die Reaktionstemperatur betrug 200 °C. Sobald diese erreicht war, wurde über den Dosiertank 40 g Furfurylalkohol rasch eindosiert. Über den Gastank wurde permanent Wasserstoff nachgeliefert und der Reaktionsdruck konstant gehalten. Zur Probenentnahme wurde die Entnahmeleitung vorher mit 1 - 2 ml Reaktionsmischung gespült und dann 0.5 - 1 ml Probe entnommen. Wenn der Wasserstoffdruck im Gastank nicht mehr weiter absank, das war nach 30 Minuten der Fall, wurde der Heizmantel entfernt und der Reaktor an der Luft auf Raumtemperatur abgekühlt. Nach der Entnahme des Reaktionsgemisches wurde der Reaktor gründlich gesäubert und bei 150 °C unter 30 bar Argon für 60 Minuten ausgeheizt.

Das Produktgemisch wurde analysiert und es wurden die folgenden Selektivitäten bei den verschiedenen Temperaturen festgestellt (Tabelle 14).

Tabelle 14: Selektivitäten *bei vollständigem Umsatz* von Furfurylalkohol in wässriger Lösung unter Zusatz verschiedener Basen mit 5 % $Ru/Al_2O_3$ (Al1). Reaktionsbedingungen: m(Kat) = 1.0 g, T = 200 °C, p($H_2$) = 100 bar, Rührgeschwindigkeit = 1000 rpm, c(FFOH) = 40.0 g / 100 ml, t = 30 Minuten (Beispiel 9) bzw. 25 min (Beispiel 10).

| Beispiel | Base | pH (nach Reaktion) | S(1,2-PD) [%] | S(CpOH) [%] | Σ [%] |
|---|---|---|---|---|---|
| 9 | 100 mg $Na_2CO_3$ | 7.9 | 34 | 1 | ≥ 100 |
| 10 | 5 g NaOAc | 7.0 | 30 | 1 | ≥ 100 |

Da es sich bei Natriumacetat um eine deutlich schwächere Base handelt, mussten große Mengen verwendet werden, um einen neutralen pH-Wert nach der Reaktion zu erzielen. Tabelle 14 zeigt einen Vergleich der Versuchsergebnisse, die bei Einsatz der beiden Basen unter Reaktionsbedingungen von T = 200 °C und p($H_2$) = 100 bar erhalten wurden. Bei Verwendung von 100 mg $Na_2CO_3$ wurde eine Selektivität von 34 % erreicht, die mit 5 g Natriumacetat erreichte Selektivität von 30 % ist zurückzuführen auf die Bildung unbekannter Nebenprodukte, die die Ausbeute an 1,2-Pentandiol verringern. Dennoch ist auch die mit Natriumacetat beobachtete Selektivität mit 30 % höher als jene der Blindprobe (Beispiel 4 bzw. Beispiel 8).

Es konnte demnach gezeigt werden, dass der überraschende positive Effekt auch bei Verwendung von anderen Basen als lediglich mit $Na_2CO_3$ erreicht werden kann und es sich um einen pH-Effekt handelt.

**Beispiele 11 und 12:**

Um den Einfluss der Eduktkonzentration auf die Selektivitätsverteilung zu untersuchen, wurde die Hydrolyse

mit zwei unterschiedlichen Konzentrationen von Furfurylalkohol in Wasser bei 200 °C unter 100 bar Wasserstoffdruck bis zum vollständigen Umsatz des Edukts durchgeführt. Im Einzelnen wurden in Beispiel 11 im Autoklaven 75.0 ml Wasser, 0.5 g des 5% $Ru/Al_2O_3$ - Katalysators Al1 sowie 100 mg $Na_2CO_3$ vorgelegt. In Beispiel 12 wurden im Autoklaven 64.4 ml Wasser, 0.5 g des $Ru/Al_2O_3$ - Katalysators sowie 100 mg $Na_2CO_3$ vorgelegt. Die Reaktionsapparatur wurde verschlossen und es wurde eine Rührgeschwindigkeit von 1000 rpm eingestellt. Der Reaktor wurde dreimal mit 10 bar Argon, sowie einmal mit 10 bar Wasserstoff gespült. Anschließend wurde der Wasserstoffdruck so eingestellt, dass der erwartete Druck bei Erreichen der Reaktionstemperatur 10 - 20 bar unter dem Reaktionsdruck von 100 bar liegen würde. Innerhalb von 30 - 60 Minuten wurde auf Reaktionstemperatur aufgeheizt. Die Reaktionstemperatur betrug 200 °C. Sobald diese erreicht war, wurden über den Dosiertank im Falle des Beispiels 11 20.0 g Furfurylalkohol in 7.0 ml Wasser, im Falle des Beispiels 12 40 g Furfurylalkohol rasch eindosiert. Über den Gastank wurde permanent Wasserstoff nachgeliefert und der Reaktionsdruck konstant gehalten. Zur Probenentnahme wurde die Entnahmeleitung vorher mit 1 - 2 ml Reaktionsmischung gespült und dann 0.5 - 1 ml Probe entnommen. Wenn der Wasserstoffdruck im Gastank nicht mehr weiter absank, das war im Falle des Beispiels 11 nach 25 Minuten, im Falle des Beispiels 12 nach 60 Minuten der Fall, wurde der Heizmantel entfernt und der Reaktor an der Luft auf Raumtemperatur abgekühlt. Nach der Entnahme des Reaktionsgemisches wurde der Reaktor gründlich gesäubert und bei 150 °C unter 30 bar Argon für 60 Minuten ausgeheizt.

Das Produktgemisch wurde analysiert und es wurden die folgenden Selektivitäten bei den verschiedenen Temperaturen festgestellt:

Die Versuchsergebnisse sind in Tabelle 15 aufgelistet. Die Unterschiede in der Produktverteilung bei Konzentrationen von 20 und 40 g FFOH in 100 mL Reaktionslösung waren dabei äußerst gering. Offenbar hat die Eduktkonzentration innerhalb dieses Konzentrationsbereiches also keinen nennenswerten Einfluss auf die Produktselektivitäten. Da eine hohe Eduktkonzentration für industrielle Anwendungen erstrebenswert ist, wurde in weiteren Versuchen mit diesem Katalysator bei einer Konzentration von 40 g FFOH in 100 ml Reaktionslösung gearbeitet.

Tabelle 15: Selektivitäten *bei vollständigem Umsatz* von Furfurylalkohol in wässriger Lösung mit 5 % $Ru/Al_2O_3$ (Al1) unter Variation der Eduktkonzentration. Reaktionsbedingungen: m(Kat) = 0.5 g, T = 200 °C, $p(H_2)$ = 100 bar, Rührgeschwindigkeit = 1000 rpm, 100 mg $Na_2CO_3$, [a]t = 25 Minuten, [b] t = 60 Minuten.

| **Beispiel** | **c (FFOH) [g / 100 ml]** | **S(1,2-PD) [%]** | **S(THFFOH) [%]** | **Σ [%]** |
|---|---|---|---|---|
| 11 | 20 [a] | 32 | 60 | ≥ 100 |
| 12 | 40 [b] | 32 | 57 | ≥ 100 |

**Beispiele 13 bis 15:**

Um den Einfluss der Katalysatormenge auf die Selektivitätsverteilung zu untersuchen, wurde die Hydrolyse mit unterschiedlichen Mengen an 5 % $Ru/Al_2O_3$ - Katalysators Al1 bei 200 °C unter 100 bar Wasserstoffdruck bis zum vollständigen Umsatz des Edukts durchgeführt. Im Einzelnen wurden im Autoklaven 64.4 ml Wasser, 100 mg $Na_2CO_3$ sowie 0.25 g (Beispiel 13), 0.5 g (Beispiel 14) bzw. 1.0 g (Beispiel 15) des Katalysators 5 % $Ru/Al_2O_3$ vorgelegt. Die Reaktionsapparatur wurde verschlossen und es wurde eine Rührgeschwindigkeit von 1000 rpm eingestellt. Der Reaktor wurde dreimal mit 10 bar Argon, sowie einmal mit 10 bar Wasserstoff gespült. Anschließend wurde der Wasserstoffdruck so eingestellt, dass der erwartete Druck bei Erreichen der Reaktionstemperatur 10 - 20 bar unter dem Reaktionsdruck von 100 bar liegen würde. Innerhalb von 30 - 60 Minuten wurde auf Reaktionstemperatur aufgeheizt. Die Reaktionstemperatur betrug 200 °C. Sobald diese erreicht war, wurden über den Dosiertank 40 g Furfurylalkohol rasch eindosiert. Über den Gastank wurde permanent Wasserstoff nachgeliefert und der Reaktionsdruck konstant gehalten. Zur Probenentnahme wurde die Entnahmeleitung vorher mit 1 - 2 ml Reaktionsmischung gespült und dann 0.5 - 1 ml Probe entnommen. Wenn der Wasserstoffdruck im Gastank nicht mehr weiter absank, das war im Falle des Beispiels 13 nach 105 Minuten, im Falle des Beispiels 13 nach 60 Minuten der Fall und im Falle des Beispiels 15 nach 30 Minuten der Fall, wurde der Heizmantel entfernt und der Reaktor an der Luft auf Raumtemperatur abgekühlt. Nach der Entnahme des Reaktionsgemisches wurde der Reaktor gründlich gesäubert und bei 150 °C unter 30 bar Argon für 60 Minuten ausgeheizt.

Das Produktgemisch wurde analysiert und es wurden die folgenden Selektivitäten bei den verschiedenen Temperaturen festgestellt:

Die Versuchsergebnisse sind in Tabelle 16 zusammengefasst.

Tabelle 16: Selektivitäten *bei vollständigem Umsatz* von Furfurylalkohol in wässriger Lösung mit 5 % $Ru/Al_2O_3$ (Al1) unter Variation der Katalysatormasse. Reaktionsbedingungen: T = 200 °C, $p(H_2)$ = 100 bar, Rührgeschwindigkeit = 1000 rpm, c(FFOH) = 40 g / 100 ml, 100 mg $Na_2CO_3$.

| Beispiel | m(Kat) [g] | t [min] | S(1,2-PD) [%] | S(THFFOH) [%] | S(CpOH) [%] | Σ [%] |
|---|---|---|---|---|---|---|
| 13 | 0.25 | 105 | 23 | 46 | 3 | 79 |
| 14 | 0.5 | 60 | 32 | 57 | 1 | ≥ 100 |
| 15 | 1.0 | 30 | 34 | 56 | 0 | ≥ 100 |

Eine Verringerung der Katalysatormasse führte zu kleineren Selektivitäten von 1,2-Pentandiol. Während sie bei Einsatz von 1 g Katalysator 34 % betrug, lag sie unter Verwendung von 0.25 g Katalysator bei nur 23 %. Weitere Trends bei Verringerung der Katalysatormasse waren eine vermehrte Entstehung von Cyclopentanol, sowie eine Abnahme der Bilanz Σ auf unter 100 % bei Einsatz von 0.25 g Katalysator, welche auf die Bildung von Polymerisationsprodukten hinweist. Diese Ergebnisse ergeben somit überraschenderweise, dass eine Katalysatormenge von größer 0.25 g, bevorzugt größer 0.5 g, besonders bevorzugt von 0.5 g bis 1.0 g an 5 % $Ru/Al_2O_3$ zur vorteilhaften Selektivität von 1,2-Pentandiol führt.

**Beispiele 16 bis 18:**

Um den Einfluss des Wasserstoffdrucks auf die Produktverteilung bei der Hydrogenolyse von Furfurylalkohol zu untersuchen, wurde die Reaktion bei 200 °C mit Drücken zwischen 50 und 150 bar durchgeführt.

Im Einzelnen wurden im Autoklaven 64.4 ml Wasser, 100 mg $Na_2CO_3$ sowie 1.0 g des Katalysators 5% $Ru/Al_2O_3$ - Katalysators Al1 vorgelegt. Die Reaktionsapparatur wurde verschlossen und es wurde eine Rührgeschwindigkeit von 1000 rpm eingestellt. Der Reaktor wurde dreimal mit 10 bar Argon, sowie einmal mit 10 bar Wasserstoff gespült. Anschließend wurde der Wasserstoffdruck so eingestellt, dass der erwartete Druck bei Erreichen der Reaktionstemperatur 10 - 20 bar unter dem jeweiligen Reaktionsdruck von 50 bar, 100 bar bzw. 150 bar liegen würde. Innerhalb von 30 - 60 Minuten wurde auf Reaktionstemperatur aufgeheizt. Die Reaktionstemperatur betrug 200 °C. Sobald diese erreicht war, wurden über den Dosiertank 40 g Furfurylalkohol rasch eindosiert. Über den Gastank wurde permanent Wasserstoff nachgeliefert und der Reaktionsdruck konstant gehalten. Zur Probenentnahme wurde die Entnahmeleitung vorher mit 1 - 2 ml Reaktionsmischung gespült und dann 0.5 - 1 ml Probe entnommen. Wenn der Wasserstoffdruck im Gastank nicht mehr weiter absank, das war im Falle des Beispiels 16 nach 140 Minuten, im Falle des Beispiels 17 nach 30 Minuten der Fall und im Falle des Beispiels 18 nach 25 Minuten der Fall, wurde der Heizmantel entfernt und der Reaktor an der Luft auf Raumtemperatur abgekühlt. Nach der Entnahme des Reaktionsgemisches wurde der Reaktor gründlich gesäubert und bei 150 °C unter 30 bar Argon für 60 Minuten ausgeheizt.

Das Produktgemisch wurde analysiert und es wurden die folgenden Selektivitäten bei den verschiedenen Temperaturen festgestellt: Die Versuchsergebnisse sind in Tabelle 17 zusammengefasst.

Tabelle 17: Selektivitäten bei der Hydrogenolyse von Furfurylalkohol in wässriger Lösung mit 5 % $Ru/Al_2O_3$ (Al1) unter Variation des Wasserstoffdrucks. Reaktionsbedingungen: m(Kat) = 1.0 g, T = 200 °C, Rührgeschwindigkeit = 1000 rpm, c(FFOH) = 40 g / 100 ml, 100 mg $Na_2CO_3$, [a] X = 100 %, [b] X = 99 %.

| Beispiel | $p(H_2)$ [bar] | t [min] | S(1,2-PD) [%] | S(THFFOH) [%] | S(1,2-PD) / S(THFFOH) | S(CpOH) [%] | Σ [%] |
|---|---|---|---|---|---|---|---|
| 16 | 50 [a] | 140 | 34 | 38 | 0.89 | 6 | ≥ 100 |
| 17 | 100 [a] | 30 | 34 | 56 | 0.61 | 0 | ≥ 100 |
| 18 | 150 [b] | 25 | 31 | 60 | 0.52 | 0 | ≥ 100 |

Es zeigte sich überraschenderweise, dass die hohe Selektivität zu 1,2-Pentandiol über einem weiten Druckbereich beobachtet werden konnte. Dennoch wurde eine Abhängigkeit der Katalysatoraktivität vom Wasserstoffdruck beobachtet: Während bei $p(H_2)$ = 100 bar nach 30 Minuten vollständiger Umsatz von Furfurylalkohol erreicht wurde, war bei $p(H_2)$ = 50 bar der Umsatz erst nach 140 Minuten nahezu vollständig. Dieser Aktivitätsverlust hat wie erwartet eine erhöhte Selektivität zu Cyclopentanol zur Folge. So entstand bei 50 bar Wasserstoffdruck Cyclopentanol mit einer Selektivität von 7 %, während es bei $p(H_2)$ = 150 bar nur im Spurenbereich nachgewiesen wurde. Das Selektivitätsverhältnis S(1,2-PD) / S(THFFOH) nimmt mit fallendem Druck zu.
Dies führt dazu, dass bei $p(H_2)$ = 50 bar trotz der vermehrten Bildung von Cyclopentanol und weiteren Nebenprodukten die Selektivität zu 1,2-Pentandiol mit 34 % so hoch ist wie bei $p(H_2)$ = 100 bar.

**Beispiele 19 bis 22:**

Um den Einfluss des Lösemittels abzuschätzen, wurde die Reaktion in drei verschiedenen organischen Lösungsmitteln bei 200 °C und 100 bar Wasserstoffdruck bis zum vollständigen Umsatz von Furfurylalkohol durchgeführt.

Im Einzelnen wurden im Autoklaven 100 mg $Na_2CO_3$, 1.0 g des Katalysators 5 % $Ru/Al_2O_3$-Katalysators Al1 sowie 64.0 ml Ethanol (Beispiel 20), 63.8 ml Tetrahdrofuran (THF; Beispiel 21), 64 ml Dioxan (Beispiel 22) oder 64.6 ml Wasser mit 100 mg $Na_2CO_3$ (Beispiel 19) vorgelegt. Die Reaktionsapparatur wurde verschlossen und es wurde eine Rührgeschwindigkeit von 1000 rpm eingestellt. Der Reaktor wurde dreimal mit 10 bar Argon, sowie einmal mit 10 bar Wasserstoff gespült. Anschließend wurde der Wasserstoffdruck so eingestellt, dass der erwartete Druck bei Erreichen der Reaktionstemperatur 10 - 20 bar unter dem jeweiligen Reaktionsdruck von 100 bar liegen würde. Innerhalb von 30 - 60 Minuten wurde auf Reaktionstemperatur aufgeheizt. Die Reaktionstemperatur betrug 200 °C. Sobald diese erreicht war, wurden über den Dosiertank 40 g Furfurylalkohol rasch eindosiert. Über den Gastank wurde permanent Wasserstoff nachgeliefert und der Reaktionsdruck konstant gehalten. Zur Probenentnahme wurde die Entnahmeleitung vorher mit 1 - 2 ml Reaktionsmischung gespült und dann 0.5 - 1 ml Probe entnommen. Wenn der Wasserstoffdruck im Gastank nicht mehr weiter absank, das war im Falle der Beispiele 19 und 21 nach 30 Minuten, im Falle des Beispiels 20 nach 60 Minuten der Fall und im Falle des Beispiels 22 nach 45 Minuten der Fall, wurde der Heizmantel entfernt und der Reaktor an der Luft auf Raumtemperatur abgekühlt. Nach der Entnahme des Reaktionsgemisches wurde der Reaktor gründlich gesäubert und bei 150 °C unter 30 bar Argon für 60 Minuten ausgeheizt.

Das Produktgemisch wurde analysiert und es wurden die folgenden Selektivitäten bei den verschiedenen Temperaturen festgestellt. Die Versuchsergebnisse sind in Tabelle 18 zusammengefasst.

Tabelle 18: Selektivitäten *bei vollständigem Umsatz* von Furfurylalkohol mit 5 % $Ru/Al_2O_3$ (Al1) unter Variation des Lösungsmittels. Reaktionsbedingungen: m(Kat) = 1.0 g, T = 200 °C, $p(H_2)$ = 100 bar, Rührgeschwindigkeit = 1000 rpm, c(FFOH) = 40 g / 100 ml, [a] 100 mg $Na_2CO_3$.

| Beispiel | Lösemittel | t [min] | S(1,2-PD) [%] | S(THFFOH) [%] | S(CpOH) [%] | Σ [%] |
|---|---|---|---|---|---|---|
| 19 | Wasser [a] | 30 | 34 | 56 | 0 | ≥ 100 |
| 20 | Ethanol | 60 | 25 | 61 | 0 | 91 |
| 21 | THF | 30 | 25 | 69 | 0 | ≥ 100 |
| 22 | 1.4-Dioxan | 45 | 20 | 64 | 0 | 88 |

Obwohl auch die Selektivitäten bei Verwendung der genannten organischen Lösemittel überraschend gut waren, wurde festgestellt, dass bei Verwendung des Lösemittels Wasser die höchste Selektivität erhalten werden konnte.

**Beispiele 23 und 24:**

Um den Einfluss der Diffusion zu untersuchen, wurde die Hydrogenolyse bei 200 °C und 100 bar Wasserstoffdruck bis zum vollständigen Umsatz von Furfurylalkohol bei 2 verschiedenen Rührgeschwindigkeiten durchgeführt.
Im Einzelnen wurden im Autoklaven 64.6 ml Wasser und 1.0 g des Katalysators 5 % $Ru/Al_2O_3$ - Katalysators Al1 mit 100 mg $Na_2CO_3$ vorgelegt. Die Reaktionsapparatur wurde verschlossen und es wurde eine Rührgeschwindigkeit von 1000 rpm (Beispiel 23) oder 1600 rpm (Beispiel 24) eingestellt. Der Reaktor wurde dreimal mit 10 bar Argon, sowie einmal mit 10 bar Wasserstoff gespült. Anschließend wurde der Wasserstoffdruck so eingestellt, dass der erwartete Druck bei Erreichen der Reaktionstemperatur 10 - 20 bar unter dem jeweiligen Reaktionsdruck von 100 bar liegen würde. Innerhalb von 30 - 60 Minuten wurde auf Reaktionstemperatur aufgeheizt. Die Reaktionstemperatur betrug 200 °C. Sobald diese erreicht war, wurden über den Dosiertank 40 g Furfurylalkohol rasch eindosiert. Über den Gastank wurde permanent Wasserstoff nachgeliefert und der Reaktionsdruck konstant gehalten. Zur Probenentnahme wurde die Entnahmeleitung vorher mit 1 - 2 ml Reaktionsmischung gespült und dann 0.5 - 1 ml Probe entnommen. Wenn der Wasserstoffdruck im Gastank nicht mehr weiter absank, das war im Falle des Beispiels 23 nach 30 Minuten, im Falle des Beispiels 24 nach 25 Minuten der Fall, wurde der Heizmantel entfernt und der Reaktor an der Luft auf Raumtemperatur abgekühlt. Nach der Entnahme des Reaktionsgemisches wurde der Reaktor gründlich gesäubert und bei 150 °C unter 30 bar Argon für 60 Minuten ausgeheizt.

Das Produktgemisch wurde analysiert und es wurden die folgenden Selektivitäten bei den verschiedenen Temperaturen festgestellt. Die Versuchsergebnisse sind in Tabelle 19 zusammengefasst.

Tabelle 19: Selektivitäten *bei vollständigem Umsatz* von Furfurylalkohol in wässriger Lösung mit 5 % $Ru/Al_2O_3$ (Al1) unter Variation der Rührgeschwindigkeit. Reaktionsbedingungen: m(Kat) = 1.0 g, T = 200 °C, $p(H_2)$ = 100 bar, c(FFOH) = 40 g / 100 ml, 100 mg $Na_2CO_3$.

| Beispiel | Rührgeschwindigkeit [rpm] | t [min] | S(1,2-PD) [%] | S(THFFOH) [%] | Σ [%] |
|---|---|---|---|---|---|
| 23 | 1000 | 30 | 34 | 56 | ≥ 100 |
| 24 | 1600 | 25 | 28 | 52 | 85 |

Diese Beispiele zeigen, dass bei Erhöhung der Rührgeschwindigkeit von 1000 auf 1600 rpm immer noch ein Selektivitätswert erreicht von S(1,2-PD) von 28% erreicht wird und die Selektivität überraschend gut war.

__Beispiele 25 bis 31:__

Um den Einfluss von Zweitmetallen im $Ru/Al_2O_3$-Katalysator zu untersuchen, wurden sechs verschiedene bimetallische [n(Ru):n(Me)] Ru-Me/$Al_2O_3$-Katalysatoren auf Basis von 5 % $Ru/Al_2O_3$ (Al1) mit dem Stoffmengenverhältnis [n(Ru):n(Me)] wie oben (Abschnitt 2.13 bis 2.15) beschrieben präpariert. Als Zweitmetalle wurden Sn, Zn, Fe, Cu, Ni und Pt eingesetzt. Während Sn in situ durch Beimischung von Sn(II)-chlorid eingeführt wurde, erfolgte die Präparation der übrigen Katalysatoren durch Imprägnierung mit einer Lösung des Metallnitrats. Es wurde jeweils 1 h bei 200 °C kalziniert und 1 h bei 250 °C reduziert. Die Hydrogenolyse von Furfurylalkohol wurde im Einzelnen wie folgt durchgeführt: Im Autoklaven wurden 0.5 g des jeweiligen bimetallischen Katalysators (Beispiel 25: 5% $Ru/Al_2O_3$ Al1; Beispiel 26: [17 : 2] Ru-Sn/$Al_2O_3$; Beispiel 27: [5 : 2] Ru-Zn/$Al_2O_3$; Beispiel 28: Ru-[5 : 2] Fe/$Al_2O_3$; Beispiel 29: [1 : 2] Ru-Cu/$Al_2O_3$ Beispiel 30: [1 : 2] Ru-Ni/$Al_2O0_3$; ; Beispiel 31: [1 : 2] Ru-Pt/$Al_2O_3$) Die Reaktionsapparatur wurde verschlossen und es wurde eine Rührgeschwindigkeit von 1000 rpm eingestellt. Der Reaktor wurde dreimal mit 10 bar Argon, sowie einmal mit 10 bar Wasserstoff gespült. Anschließend wurde der Wasserstoffdruck so eingestellt, dass der erwartete Druck bei Erreichen der Reaktionstemperatur 10 - 20 bar unter dem jeweiligen Reaktionsdruck von 100 bar liegen würde. Innerhalb von 30 - 60 Minuten wurde auf Reaktionstemperatur aufgeheizt. Die Reaktionstemperatur betrug 200 °C. Sobald diese erreicht war, wurden über den Dosiertank 40 g Furfurylalkohol rasch eindosiert. Über den Gastank wurde permanent Wasserstoff nachgeliefert und der Reaktionsdruck konstant gehalten. Zur Probenentnahme wurde die Entnahmeleitung vorher mit 1 - 2 ml Reaktionsmischung gespült und dann 0.5 - 1 ml Probe entnommen. Wenn der Wasserstoffdruck im Gastank nicht mehr weiter absank, das war im Falle der Beispiele 25 und 30 nach 60 Minuten, im Falle des Beispiels 26 nach 20 Minuten der Fall, im Falle der Beispiele 27 und 28 nach 90 Minuten im Falle des Beispiels 29 nach 15 Minuten der Fall, im Falle des Beispiels 31 nach 180 Minuten der Fall. Dann wurde der Heizmantel entfernt und der Reaktor an der Luft auf Raumtemperatur abgekühlt. Nach der Entnahme des Reaktionsgemisches wurde der Reaktor gründlich gesäubert und bei 150 °C unter 30 bar Argon für 60 Minuten ausgeheizt.

Das Produktgemisch wurde analysiert und es wurden die folgenden Selektivitäten bei den verschiedenen Katalysatoren festgestellt. Die Versuchsergebnisse sind in Tabelle 20 zusammengefasst.

Tabelle 20: Umsätze und Selektivitäten bei der Hydrogenolyse von Furfurylalkohol in wässriger Lösung mit bimetallischen Ru-Me/$Al_2O_3$ Katalysatoren. Reaktionsbedingungen: Rührgeschwindigkeit = 1000 rpm, m(Kat) = 0.5 g, T = 200 °C, $p(H_2)$ = 100 bar, c(FFOH) = 40 g / 100 ml, 100 mg $Na_2CO_3$.

| Beispiel | Katalysator | t [min] | X (FFOH) [%] | S (1,2-PD) [%] | S (THFF OH) [%] | S (CpOH) [%] | Σ [%] |
|---|---|---|---|---|---|---|---|
| 25 | 5% $Ru/Al_2O_3$ (Al1) | 60 | 100 | 32 | 57 | 1 | ≥ 100 |
| 26 | [17:2]Ru-Sn/ $Al_2O_3$ | 20 | 0 | - | - | - | - |
| 27 | [5:2] Ru-Zn/ $Al_2O_3$ | 90 | 100 | 24 | 54 | 1 | 86 |
| 28 | [5:2] Ru-Fe/ $Al_2O_3$ | 90 | 100 | 26 | 48 | 1 | 84 |

(fortgesetzt)

| Beispiel | Katalysator | t [min] | X (FFOH) [%] | S (1,2-PD) [%] | S (THFF OH) [%] | S (CpOH) [%] | Σ [%] |
|---|---|---|---|---|---|---|---|
| 29 | [1:2] Ru-Cu/ $Al_2O_3$ | 15 | 0 | - | - | - | - |
| 30 | [1:2] Ru-Ni/ $Al_2O_3$ | 60 | 100 | 7 | 70 | 0 | 82 |
| 31 | [1:2] Ru-Pt/ $Al_2O_3$ | 180 | 100 | 10 | 37 | 12 | 71 |

Zum Vergleich sind die Versuchsergebnisse unter Einsatz des monometallischen 5 % Ru/$Al_2O_3$ in Beispiel 25 bei identischen Reaktionsbedingungen eingetragen, welcher eine Selektivität von 32 % zu 1,2-Pentandiol lieferte. Der Zusatz von Sn und Cu verringerte die Aktivität des Katalysators so stark, dass kein Wasserstoffverbrauch feststellbar war und die Reaktion nach 20 bzw. 15 Minuten abgebrochen wurde. Ru-Pt/$Al_2O_3$ zeigte mit einer Reaktionszeit von 180 Minuten eine geringe Aktivität, was erwartungsgemäß eine vermehrte Bildung von Cyclopentanol (S = 12 %) sowie vermutlich eine verstärkte Polymerisation (Σ = 71 %) zur Folge hatte. Die Selektivität zu 1,2-Pentandiol war mit 10 % entsprechend gering. Auch Ru-Ni/$Al_2O_3$ zeigte mit 7 % eine kleine Selektivität zu 1,2-Pentandiol, während das Hauptprodukt THFFOH mit einer äußerst hohen Selektivität von 70 % gebildet wurde. Dies lässt sich damit erklären, dass Nickel als aktiver und hochselektiver Katalysator zur Hydrierung von FFOH zu THFFOH fungiert. [23, 26, 27, 28] Somit lässt sich auch nachvollziehen, dass Ru-Ni/$Al_2O_3$ als einziger bimetallischer Katalysator keine verringerte Aktivität im Vergleich zu 5 % Ru/$Al_2O_3$ (Al1) aufwies. Ru-Zn/$Al_2O_3$ und Ru-Fe/$Al_2O_3$ besaßen mit 24 bzw. 26 % eine moderate Selektivität zu 1,2-Pentandiol und zeigten ein ähnliches Selektivitätsverhältnis S(1,2-PD) / S(THFFOH) von etwa 1 : 2 wie der monometallische Katalysator. Zusammenfassend ist zu sagen, dass der Zusatz eines Zweitmetalls unter den gewählten Reaktionsbedingungen stets zur Verringerung der Selektivität zu 1,2-Pentandiol führte.

**Beispiele 32 bis 37:**

Um den Einfluss ionischer Flüssigkeiten auf die Selektivität abschätzen zu können, wurden weitere Versuche unter Zusatz bestimmter ionischer Flüssigkeiten durchgeführt.

Aus der Literatur ist bekannt, dass schon der Zusatz von geringen Mengen einer ionischen Flüssigkeit (IL) die Selektivität einer heterogen katalysierten Reaktion drastisch verändern kann. [43, 44] Die IL wird dabei als dünne Schicht in den Katalysatorporen immobilisiert, weshalb diese Methode auch als *SCILL* (*solid catalyst with ionic liquid layer*) bekannt ist. Im Multibatchreaktor wurde die Hydrogenolyse von Furfurylalkohol an 5 % Ru/$Al_2O_3$ unter Zusatz von 3 unterschiedlichen, auf dem Dicyanamid-Anion basierenden ILs untersucht. Zum Vergleich wurde außerdem ein Versuch unter Zusatz des Salzes Natriumdicyanamid durchgeführt. Um eine Zersetzung der ILs zu vermeiden, wurde die Reaktion bei 180 °C durchgeführt.

Die Versuche zum Vergleich der Katalysator-Performance unter Zusatz unterschiedlicher ionischer Flüssigkeiten wurden in einer Multibatchanlage durchgeführt. Der Versuchsaufbau war dabei wie folgt: Die Multibatchanlage umfasst fünf Reaktoren aus rostfreiem Stahl mit jeweils 40 ml Fassungsvermögen. Die Durchmischung wird mit Hilfe eines fünffachen Magnetrührwerkes gewährleistet. Jedem Reaktor ist ein 75 ml Gastank aus rostfreiem Stahl vorgeschaltet, welcher über das Gaszuleitungssystem mit Argon oder Wasserstoff ≤ 100 bar beschickt werden kann. Über eine thermostatgesteuerte Batterie von Heizblöcken können die einzelnen Reaktoren unabhängig voneinander beheizt werden. Die Steuerung und Kontrolle der Reaktionstemperatur erfolgte computergesteuert.

Im Reaktor wird eine Suspension aus 4 g FFOH, 6.1 ml Wasser, 100 mg des 5 % Ru/$Al_2O_3$ Katalysators Al1 und 2.5 mg Natriumdicyanamid (Beispiel 32) bzw. 5 μl ionische Flüssigkeit (Beispiel 33: 1-Butyl-3-methylimidazolium-dicyanamid; Beispiel 34: N-Butyl-3-methylpyridinium-dicyanamid; Beispiel 35: 1-Butyl-1-methylpyrrolidinium-dicyanamid) vorgelegt. Der Reaktor wird verschlossen und an die Anlage angeschlossen. Nachdem dreimal mit 30 bar Argon und einmal mit 50 bar Wasserstoff gespült wurde, werden 85 bar Wasserstoff beaufschlagt und auf 180 °C aufgeheizt. Der Druck erreicht beim Aufheizen zwischenzeitlich bis zu 90 bar und fällt zu Reaktionsende auf etwa 75 bar ab. Der Versuch wird 100 Minuten nach Erreichen der Reaktionstemperatur abgebrochen. Dazu wird die Druckleitung des Reaktors von der Anlage getrennt und der Reaktor im Wasserbad auf Raumtemperatur abgekühlt. Es wird durch vorsichtiges Öffnen des Nadelventils auf Umgebungsdruck entspannt und die Reaktionsmischung aus dem Reaktor entnommen. Die Versuchsergebnisse sind in Tabelle 21 zusammengefasst.

Tabelle 21: Selektivitäten *bei vollständigem Umsatz* von Furfurylalkohol in wässriger Lösung mit 5 % $Ru/Al_2O_3$ (Al1) mit verschiedenen Zusätzen. Reaktionsbedingungen: m(Kat) = 100 mg, T = 180 °C, $p(H_2)$Start = 85 bar, c(FFOH) = 4 g / 10 ml, 10 mg $Na_2CO_3$, [a] 2.5 mg NaDCA, [b] 5 µl IL.

| Beispiel | Zusatz | S(1,2-PD) [%] | S(THFFOH) [%] | Σ [%] |
|---|---|---|---|---|
| 32 | NaDCA [a] | 15 | 65 | 85 |
| 33 | 1-Butyl-3-methylimidazolium-DCA [b] | 15 | 80 | ≥ 100 |
| 34 | N-Butyl-3-methylpyridinium-DCA [b] | 14 | 82 | ≥ 100 |
| 35 | 1-Butyl-1-methylpyrrolidinium-DCA [b] | 13 | 77 | 92 |

In allen Experimenten entstand THFFOH als Hauptprodukt mit hohen Selektivitäten von 65 - 82 %. 1-Butyl-3-methylimidazolium-DCA wies mit 15 % die höchste Selektivität zu 1,2-Pentandiol auf. Um einen besseren Vergleich zu den bisherigen Versuchsergebnissen ohne ILs ziehen zu können, wurde 1-Butyl-3-methylimidazolium-DCA nochmals im Batchreaktor untersucht. Dazu wurde die Hydrogenolyse von Furfurylalkohol bei 180 °C und 100 bar Wasserstoffdruck an 5 % $Ru/Al_2O_3$ (Al1) unter Zusatz von 50 µl 1-Butyl-3-methylimidazolium-DCA bis zu vollständigem Umsatz durchgeführt. Dabei wurde eine Selektivität zu 1,2-Pentandiol von 23 % erzielt, wobei THFFOH mit einer Selektivität von 60 % das Hauptprodukt darstellte (Tabelle 22). Im Vergleich zum vorangegangenen Versuch, der ohne Zusatz von IL bei 200 °C unter ansonsten gleichen Reaktionsbedingungen durchgeführt wurde, hatte sich somit das Selektivitätsverhältnis S(1,2-PD) / S(THFFOH) verringert. Diese Beobachtung lässt sich allerdings auch durch die um 20 °C geringere Reaktionstemperatur erklären, da nach bisherigen Erkenntnissen eine Temperaturminderung zur Verringerung von S(1,2-PD) / S(THFFOH) führt.

Tabelle 22: Selektivitäten *bei vollständigem Umsatz* von Furfurylalkohol in wässriger Lösung mit 5 % $Ru/Al_2O_3$ (Al1) mit und ohne Zusatz von IL. Reaktionsbedingungen: m(Kat) = 1.0 g, $p(H_2)$ = 100 bar, c(FFOH) = 40 g / 100 ml, 100 mg $Na_2CO_3$, 50 µl IL, [a] t = 25 min, [b] t = 30 min.

| Beispiel | IL | T [°C] | S(1,2-PD) [%] | S(THFFOH) [%] | Σ [%] |
|---|---|---|---|---|---|
| 36 | 1-Butyl-3-methylimidazolium -DCA [a] | 180 | 23 | 60 | 88 |
| 37 | Keine [b] | 200 | 34 | 56 | ≥ 100 |

**Beispiele 2/1 bis 2/4: Screening der PtO(IV)-Kats**

Die Hydrogenolyse von Furfurylalkohol wurde im Batchreaktor an 4 unterschiedlichen Pt(IV)-Oxid-Katalysatoren in Ethanol als Lösungsmittel untersucht. In Anlehnung an die Literatur wurde die Reaktion bei Raumtemperatur und 2 bar Wasserstoffüberdruck durchgeführt. [18] Eine Ausnahme stellte der Katalysator Pt-JA-023 dar. Aufgrund seiner geringen Aktivität wurden Wasserstoffdruck und Reaktionstemperatur während der Reaktion auf bis zu 180 °C und 60 bar erhöht. Die Versuchsergebnisse sind in Tabelle 23 zusammengefasst. Mit Ausnahme von Pt-JA-023 erreichten alle Katalysatoren hohe Umsätze von über 96 % innerhalb von 2 - 5 h Reaktionszeit. Es wurde ein breites Produktspektrum, bestehend aus 1,2-PD, 1,5-PD, THFFOH, 1-POH, 2-MF, 2-MTHF, sowie weitere unbekannte Nebenprodukte erhalten. Dies stimmt im groben mit den aus der Literatur bekannten Reaktionsprodukten überein. [21] Im Vergleich zu den Trägerkatalysatoren (Tabelle 11) wurde ein wesentlich höheres Verhältnis von 1,5-PD zu 1,2-PD erhalten. Im Falle des Pt(IV)-Oxid-Katalysators von Heraeus wurde mit 23 % Selektivität sogar mehr 1,5-PD als 1,2-PD gebildet. Die höchste Selektivität zu 1,2-Pentandiol erzielte mit 25 % der Pt(IV)-Oxid Katalysator von Sigma Aldrich. Es fällt auf, dass die berechnete Bilanz Σ für alle getesteten Katalysatoren deutlich unter 100 % beträgt. Dies ist vermutlich darauf zurückzuführen, dass große Mengen unbekannter Nebenprodukte erzeugt wurden, für die keine GC-Kalibrierdaten vorlagen.

Tabelle 23: Umsätze und Selektivitäten bei der Hydrogenolyse von Furfurylalkohol in Ethanol an Pt(IV)-Oxid Katalysatoren. Reaktionsbedingungen: Rührgeschwindigkeit = 1000 rpm, m(Kat) = 0.5 g, c(FFOH) = 10 g / 100 ml.

| Beispiel | Katalysator | t [min] | T [°C] | $p(H_2)$ [bar Überdruck] | X(FFOH) [%] | Selektivitäte n [%] | Σ [%] |
|---|---|---|---|---|---|---|---|
| 2/1 | Pt(IV)-Oxid Pt-JA-023 | 120 | 25-180 | 20 - 60 | 64 | 1,2-PD: 13<br>1,5-PD: 11<br>THFFOH: 46<br>1-POH: 7<br>Sonstige: 15 | 94 |
| 2/2 | Pt(IV)-Oxid Heraeus | 300 | 25-50 | 2 | 99 | 1,2-PD: 11<br>1,5-PD: 23<br>THFFOH: 53<br>1-POH: 2<br>Sonstige: 6 | 94 |
| 2/3 | Pt(IV)-Oxid Pt-JA-021 | 120 | 25 | 2 | 100 | 1,2-PD: 19<br>1,5-PD: 3<br>THFFOH: 24<br>1-POH: 14<br>2-MTHF: 12<br>Sonstige: 16 | 89 |
| 2/4 | Pt(IV)-Oxid Sigma Aldrich | 270 | 25 | 2 | 96 | 1,2-PD: 25<br>1,5-PD: 3<br>THFFOH: 24<br>1-POH: 11<br>2-MTHF: 6<br>Sonstige: 20 | 93 |

**Beispiele 2/5 bis 2/6: Variation des Lösungsmittels**

Neben Ethanol ist in der Literatur auch Essigsäure als Lösungsmittel für die Hydrogenolyse von Furfurylalkohol an Pt(IV)-Oxid bekannt. [22] Mit Pt(IV)-Oxid (Heraeus) wurde daher ein Versuch in Essigsäure durchgeführt. Während der Reaktion war ein starkes Abfallen des Wasserstoffverbrauchs zu beobachten, weshalb die Temperatur nach 1 h Reaktionszeit auf 75 °C erhöht wurde. Bereits bei 38 % Umsatz stoppte die Reaktion und es war kein Wasserstoffverbrauch mehr feststellbar. Die Selektivität zu 1,2-Pentandiol war mit 6 % nur etwa halb so hoch wie bei Durchführung der Reaktion in Ethanol (Tabelle 24). Das Verhältnis von 1,2-PD zu 1,5-PD hingegen konnte in Essigsäure von 1:2 auf 6:1 verbessert werden. Es entstanden große Mengen an unbekannten Nebenprodukten mit einer Gesamtselektivität von etwa 38 %.

Tabelle 24: Vergleich der Umsätze und Selektivitäten bei der Hydrogenolyse von Furfurylalkohol an Pt(IV)-Oxid (Heraeus) in Ethanol und Essigsäure. Reaktionsbedingungen: Rührgeschwindigkeit = 1000 rpm, m(Kat) = 0.5 g, c(FFOH) = 10 g / 100 ml, [a] Lösungsmittel: Ethanol, [b] Lösungsmittel: Essigsäure.

| Beispiel | Katalysator | t [min] | T [°C] | $p(H_2)$ [bar Überdruck] | X(FFOH) [%] | Selektivitäten [%] | Σ [%] |
|---|---|---|---|---|---|---|---|
| 2/5 | Pt(IV)-Oxid [a]<br>Heraeus | 300 | 25-50 | 2 | 99 | 1,2-PD: 11<br>1,5-PD: 23<br>THFFOH: 53<br>1-POH: 2<br>Sonstige: 6 | 94 |
| 2/6 | Pt(IV)-Oxid [b]<br>Heraeus | 150 | 25 - 75 | 2 | 38 | 1,2-PD: 6<br>1,5-PD: 1<br>THFFOH: 7 | 83 |

(fortgesetzt)

| Beispiel | Katalysator | t [min] | T [°C] | $p(H_2)$ [bar Überdruck] | X(FFOH) [%] | Selektivitäten [%] | Σ [%] |
|---|---|---|---|---|---|---|---|
| | | | | | | 1,2-BD: 1<br>1,5-HD: 1<br>1-POH: 1<br>Sonstige: 38 | |

**Beispiele 2/7 bis 2/10: Einfluss von Salzsäure**

Bei der Durchführung der Reaktion in Ethanol werden in der Literatur meist geringe Mengen von Additiven zugesetzt. Während Adams Eisenchlorid verwendete, benutzte Nishimura geringe Mengen verdünnter Salzsäure. [19, 21] Um den Einfluss von Salzsäure auf Aktivität und Selektivität zu untersuchen, wurden Versuche mit zwei verschiedenen Pt(IV)-Oxid-Katalysatoren in Ethanol unter Zusatz von je 1 ml 2 N Salzsäure bei Raumtemperatur und 2 bar Wasserstoffüberdruck durchgeführt. Die Versuchsergebnisse sind in Tabelle 25 im Vergleich mit den Ergebnissen, die ohne Zusatz von Salzsäure unter gleichen Reaktionsbedingungen erzielt wurden, aufgelistet. In beiden Fälle führte die Zugabe von Salzsäure zu einer Verringerung der Selektivität zu THFFOH. Während es mit Pt-JA-021 zu einer Verbesserung von S(1,2-PD) von 19 auf 22 % kam, verschlechterte diese sich beim Katalysator von Sigma Aldrich von 25 auf 20 %. Gleichzeitig wurde beim letztgenannten Katalysator eine drastische Steigerung der Aktivität beobachtet. So war unter Verwendung von Salzsäure nach 90 Minuten Reaktionszeit vollständiger Umsatz von FFOH erreicht, während der Umsatz ohne Zugabe von Salzsäure nach 270 Minuten 96 % betrug. Bei Pt-JA-021 hingegen wirkte sich die Anwesenheit von Salzsäure kaum auf die Aktivität aus.

Tabelle 25: Vergleich der Umsätze und Selektivitäten bei der Hydrogenolyse von Furfurylalkohol an Pt(IV)-Oxid in Ethanol mit und ohne Zusatz von Salzsäure. Reaktionsbedingungen: Rührgeschwindigkeit = 1000 rpm, m(Kat) = 0.5 g T = 25 °C, $p(H_2)$ = 2 bar Überdruck, c(FFOH) = 10 g / 100 ml.

| Beispiel | Katalysator | t [min] | V(2 N Salzsäure) [ml] | X (FFOH) [%] | S(1,2-PD) [%] | S(THFF OH) [%] |
|---|---|---|---|---|---|---|
| 2/7 | Pt(IV)-Oxid (Pt-JA-021) | 120 | 0 | 100 | 19 | 24 |
| 2/8 | Pt(IV)-Oxid (Pt-JA-021) | 130 | 1 | 100 | 22 | 20 |
| 2/9 | Pt(IV)-Oxid (Sigma Aldrich) | 270 | 0 | 96 | 25 | 24 |
| 2/10 | Pt(IV)-Oxid (Sigma Aldrich) | 90 | 1 | 100 | 20 | 11 |

**Beispiele 2/11 bis 2/15: Einfluss von Druck und Temperatur**

Um die Abhängigkeit der Aktivität und Selektivitätsverteilung von den Reaktionsbedingungen näher zu untersuchen, wurden Versuche mit Pt(IV)-Oxid (Sigma Aldrich), dem selektivsten der getesteten Adams-Katalysatoren, unter Variation von Wasserstoffdruck und Temperatur in Ethanol durchgeführt.

Eine Erhöhung der Reaktionstemperatur von 25 auf 75 °C bei 2 bar Wasserstoffüberdruck führte zu keiner nennenswerten Änderung von S(1,2-PD) (Tabelle 26). Während sich S(THFFOH) von 24 auf 17 % verringerte, wurden vermehrte andere, meist unbekannte, Nebenprodukte gebildet. Der Wasserstoffverbrauch nahm während der Reaktion außerordentlich schnell ab, sodass bereits bei 87 % Umsatz kein Wasserstoffverbrauch mehr feststellbar war. Diese Beobachtung legt die Vermutung nahe, dass es unter diesen Bedingungen zu einer Desaktivierung des Katalysators kommt.

Tabelle 26: Vergleich der Umsätze und Selektivitäten bei der Hydrogenolyse von Furfurylalkohol an Pt(IV)-Oxid (Sigma Aldrich) in Ethanol bei verschiedenen Temperaturen. Reaktionsbedingungen: Rührgeschwindigkeit = 1000 rpm, m(Kat) = 0.5 g $p(H_2)$ = 2 bar Überdruck, c(FFOH) = 10 g / 100 ml.

| Beispiel | T [°C] | t [min] | X (FFOH) [%] | S(1,2-PD) [%] | S(THFFOH) [%] |
|---|---|---|---|---|---|
| 2/11 | 25 | 270 | 96 | 25 | 24 |
| 2/12 | 75 | 270 | 87 | 25 | 17 |

Um den Einfluss des Wasserstoffdrucks zu untersuchen, wurde die Hydrogenolyse unter 5 bar Überdruck sowie unter Atmosphärendruck bei Raumtemperatur unter Zusatz von 1 ml 2 N Salzsäure durchgeführt (Tabelle 27). Im Gegensatz zum sonst üblichen Batchreaktor wurde zur Durchführung der Reaktion unter Atmosphärendruck eine *Glasapparatur* verwendet, wobei Wasserstoff kontinuierlich in die Reaktionslösung eingeleitet wurde. Es wurde eine starke Abhängigkeit der Katalysatoraktivität vom Wasserstoffdruck beobachtet. Die Reaktion verlief bei $p(H_2)$ = 5 bar so rasch, dass die Temperatur aufgrund der Reaktionswärme auf bis zu 50 °C zunahm. Außerdem führte die Druckerhöhung zu einer Verbesserung der Selektivität zu 1,2-PD von 20 auf 23 %. Bei Durchführung der Reaktion unter Atmosphärendruck wurde nach 270 Minuten Reaktionszeit lediglich 42 % Umsatz erzielt, wobei aber die Selektivität zu 1,2-PD 31 % betrug.

Tabelle 27: Vergleich der Umsätze und Selektivitäten bei der Hydrogenolyse von Furfurylalkohol an Pt(IV)-Oxid (Sigma Aldrich) in Ethanol bei verschiedenen Wasserstoffdrücken. Reaktionsbedingungen: Rührgeschwindigkeit = 1000 rpm, m(Kat) = 0.5 g T = 25 °C, c(FFOH) = 10 g / 100 ml, 1 ml 2 N Salzsäure, [a] Erhöhung der Temperatur während der Reaktion auf bis zu 50 °C.

| Beispiel | $p(H_2)$ [bar Überdruck] | t [min] | X (FFOH) [%] | S(1,2-PD) [%] | S(THFFOH) [%] |
|---|---|---|---|---|---|
| 2/13 | 0 | 270 | 42 | 31 | 15 |
| 2/14 | 2 | 90 | 100 | 20 | 11 |
| 2/15 | 5 [a] | 20 | 99 | 23 | 11 |

**Zusammenfassung der Ergebnisse**

In dieser Erfindung sind Untersuchungen zur Hydrogenolyse von Furfurylalkohol an Ru- und Pt-Katalysatoren im Hinblick auf ihre Selektivität zum Wunschprodukt 1,2-Pentandiol dargestellt. Während Pt(IV)-Oxid bereits bei Raumtemperatur und 1 - 5 bar Wasserstoffdruck hinreichend aktiv war, wurden für Ru- und Pt-Trägerkatalysatoren drastischere Reaktionsbedingungen von T ≥ 150 °C und p(H2) ≥ 50 bar benötigt. Es wurden 4 unterschiedliche Pt(IV)-Oxid-Katalysatoren eingesetzt. Dabei traten als Reaktionsprodukte neben 1,2-Pentandiol außerdem 1,5-Pentandiol, 1-Pentanol, 2-Methyltetrahydrofuran, Tetrahydrofurfurylalkohol und große Mengen unidentifizierter Substanzen auf, wobei die Selektivitätsverteilung stark vom Katalysator abhing. Der selektivste unter den 4 getesteten Pt(IV)-Oxid-Katalysatoren, welcher von Sigma Aldrich erworben wurde, erzielte bei Raumtemperatur und 2 bar Wasserstoffüberdruck in Ethanol eine 25%ige Selektivität zu 1,2-Pentandiol bei 96 % Umsatz. Dies entspricht einer Ausbeute von 24 %. Eine Steigerung der Reaktionstemperatur führte zur Minderung der Aktivität, vermutlich aufgrund der Desaktivierung des Katalysators im Laufe der Reaktion. Bei der Durchführung der Reaktion bei Raumtemperatur und Atmosphärendruck konnte eine Selektivität von 31 % bei 42 % Umsatz erzielt werden. Durch Zusatz geringer Mengen verdünnter Salzsäure wurde die Selektivität zu Tetrahydrofurfurylalkohol herabgesetzt. Der Einfluss von Salzsäure auf die Selektivität zu 1,2-Pentandiol war vom Katalysator abhängig. Während sie bei Verwendung von Pt(IV)-Oxid (Sigma Aldrich) von 25 auf 20 % abnahm, erhöhte sie sich bei Einsatz von Pt(IV)-Oxid (Pt-JA-021) von 19 auf 22 %. Bei der Hydrogenolyse von Furfurylalkohol in Wasser an 8 unterschiedlichen Ru- und Pt-Trägerkatalysatoren (m = 0.5 g) bei 200 °C und 100 bar Wasserstoffdruck unter vollständigem Umsatz entstand als Hauptprodukt stets Tetrahydrofurfurylalkohol. Die beste Ausbeute zu 1,2-Pentandiol lieferte unter diesen Reaktionsbedingungen der 5 % $Ru/Al_2O_3$-Katalysator Al1 mit 32 %. Mit diesem Katalysator wurden daher weitere Experimente unter Variation der Reaktionsbedingungen durchgeführt. Als wichtigste Nebenprodukte traten allgemein 1-Butanol, Cyclopentanol und 1,2-Butanol auf. Die Entstehung von 1-Butanol ist wahrscheinlich auf die Hydrogenolyse von Furan zurückzuführen, welches durch Decarboxylierung von Furfurylalkohol entsteht. Die Bildung von Cyclopentanol lässt sich durch eine Isomerisierung von Furfurylalkohol zu 4-Hydroxy-2-cyclopentenon mit anschließender sukzessiver Hydrierung über die Zwischenstufen Cyclopentenon und Cyclopentanon erklären. Die aus der gaschromatographischen Analyse berechnete Stoffbilanz lag häufig unter 100 %, was auf eine Polymerisation von Furfurylalkohol

hindeutet. Sowohl die Polymerisation als auch die Isomerisierung zu 4-Hydroxy-2-cyclopentenon benötigen keinen Katalysator und verlaufen auch ohne Anwesenheit von Wasserstoff, sodass sie vor allem bei geringer Katalysatormasse und niedrigem Wasserstoffdruck in den Vordergrund treten. Eine Erhöhung der Reaktionstemperatur führte zur verstärkten Polymerisation sowie zu einer vermehrten Bildung von Cyclopentanol. Im Gegensatz dazu nahm die Selektivität zu Tetrahydrofurfurylalkohol mit steigender Temperatur ab. Es wurde überraschend gefunden, dass die Kontrolle des pH-Wertes insbesondere bei hohen Reaktionstemperaturen eine entscheidende Rolle spielt und ein überraschender Effekt erzielt werden kann hinsichtlich der verbesserten Selektivität zu 1,2-Pentandiol. Die Erfinder vermuten, dass die Polymerisation von Furfurylalkohol und die Isomerisierung zu 4-Hydroxy-2-cyclopentenon bevorzugt unter sauren Bedingungen stattfinden. Die Zugabe von geringen Mengen Natriumcarbonat konnte die Selektivität zu 1,2-Pentandiol bei 240 °C unter vollständigem Umsatz von 27 % auf bis zu 35 % verbessern. Dies entspricht der besten Selektivität, die erzielt wurde. Wurden größere Menge Base zugesetzt, verringerte sich die Selektivität zu 1,2-Pentandiol. Allgemein war eine Abnahme des pH-Wertes während der Reaktion zu beobachten. Durch Minderung des Wasserstoffdrucks konnte die Selektivität zu Tetrahydrofurfurylalkohol verringert werden. Aufgrund der verstärkten Bildung von Cyclopentanol konnte dadurch jedoch keine Verbesserung der Selektivität zu 1,2-Pentandiol erzielt werden. Die Selektivitäten änderten sich unter Variation der Eduktkonzentration nur geringfügig. Ein Umstieg auf organische Lösungsmittel konnte die Bildung von Cyclopentanol zwar verhindern, führte allerdings zu einer erhöhten Selektivität zu Tetrahydrofurfurylalkohol. Die Selektivität zu 1,2-Pentandiol bei Durchführung der Reaktion in organischer Lösung war mit 20 - 25 % deutlich geringer als in Wasser. Durch Erhöhung der Katalysatormasse konnte S(1,2-PD) bei T = 200 °C und $p(H_2)$ = 100 bar geringfügig von 32 auf 34 % verbessert werden, während eine Verringerung der Katalysatormasse zur Selektivitätsminderung und verstärkter Polymerisation führte. Durch die Zugabe geringer Mengen ionischer Flüssigkeiten unter Anwendung des SCILL Konzepts und den Einsatz bimetallischer Ru-Me/$Al_2O_3$-Katalysatoren wurde unter den eingestellten Bedingungen keine Verbesserung der Selektivität zu 1,2-Pentandiol erreicht.

**Literaturverzeichnis**

[1] http://www.mnforsustain.org/oil_peaking_of_world_oil_production_study_hirsch.htm
[2] http://www.fnr.de
[3] http://www.eere.energy.gov
[4] http://www.suschem.org
[5] http://www.cefic.org
[6] A. Corma, S. Iborra, A. Velty, Chem. Rev. 2007, 107, 241 - 2502
[7] H. E. Hoydonckx et al., Ullmann's Encyclopedia of Industrial Chemistry WILEY-VCH, DOI: 10.1002/14356007.a12_119.pub2
[8] P. Gallezot, Chem. Soc. Rev. 2012, 41, 1538-1558
[9] P. Anastas, N. Eghbali, Chem. Soc. Rev. 2010, 39, 301 - 312
[10] P. Anastas, M. Kirchhoff, T. Williamson, Applied Catalysis A: General 2001, 221, 3-13
[11] J. W. Döbereiner, Berichte der deutschen chemischen Gesellschaft 1832, 3, 141 - 146
[12] B. A. Tokay, Ullmann's Encyclopedia of Industrial Chemistry WILEY-VCH, DOI: 10.1002/14356007.a04_099
[13] W. Lazier, 1931, US Patent 2077422
[14] H. Adkins, R. Conner, 1932, US Patent 2094975
[15] I. S. Goldstein, W. A. Dreher, Industrial and Engineering Chemistry 1960, 52, 57 - 58
[16] Degussa, DE 2937840, 1981 (G. Käbisch, H. Malitius, S. Raupach, R. Trübe, H. Wittmann)
[17] R. Kadyrov et al., Angew. Chem. Int. Ed. 2009, 48, 7556 - 7559
[18] L. B. Hunt, Platinum Metals Rev. 1962, 6, 150 - 152
[19] W. E. Kaufmann, R. Adams, J. Am. Chem. Soc. 1923, 45, 3029 - 3044
[20] S. Nishimura, Handbook of Heterogeneous Catalytic Hydrogenation for Organic Synthesis, John Wiley & Sons (2001)
[21] S. Nishimura, Bulletin of the Chemical Society of Japan 1961, 34, 32 - 36
[22] H. A. Smith, J. F. Fuzek, J. Am. Chem. Soc. 1949, 71, 415 - 419
[23] W. Xu et al., Chem. Commun. 2011, 47, 3924 - 3926
[24] H. Adkins, R. Connor, J. Am. Chem. Soc. 1931, 53, 1091 - 1095
[25] D. G. Manly, A. P. Dunlop, J. Org. Chem. 1958, 23, 1093 - 1095
[26] N. Merat, C. Godawa, A. Gaset, J. Chem. Tech. Biotechnol. 1990, 48, 145 - 159
[27] Y. Song et al., Front. Chem. Eng. China 2007, 1, 151 - 154
[28] B. H. Wojcik, Ind. Eng. Chem. 1948, 40, 210 - 216
[29] G. J. Leuck, J. Pokorny, F. N. Peters **1934,** US Patent 2097493

[30] F. A. Khan, A. Vallat, G. Süss-Fink, Catal. Commun. 2011, 12, 4128 - 1431
[31] M. H. Tike, V. V. Mahajani, Ind. Eng. Chem. Res. 2007, 46, 3275 - 3282
[32] P. D. Seemuth, **1984,** US Patent 4459419
[33] K. Tomishige et al., Chem. Comm. 2009, 15, 2035 - 2037
[34] K. Tomishige, S. Koso, Y. Nakagawa, Journal of Catalysis 2011, 2, 221 - 229
[35] A. Suzuki et al., Catal. Sci. Technol. 2011, 1, 1466 - 1471
[36] P. Walden, Bull Acad. Sci. St. Petersburg 1914, 405 - 422
[37] J. S. Sandhu, Green Chemistry Letters and Reviews 2011, 4, 289 - 310
[38] S. E. Fry, N. J. Pienta, J. Am. Chem. Soc. 1985, 107, 6399 - 6400
[39] J. S. Wilkes et al., J. Org. Chem 1986, 51, 480 - 483
[40] A. Jess et al., Chem. Eng. Technol. 2007, 30, 985 - 994
[41] M. Stark et al., Adv. Mater 2011, 23, 2571 - 2587
[42] P. Claus et al., J. Phys. Chem. 2010, 114, 10520 - 10526
[43] P. Claus et al., Chem. Commun. 2008, 4058 - 4060
[44] P. Claus et al., Chem. Commun. 2009, 11, 716 - 723
[45] Y.-W. Li et al., Journal of Molecolar Catalysis A: Chemical 2006, 246, 18 - 23
[46] M. Hronec, K. Fulajtarova, Catalysis Communications 2012, 24, 100 - 104
[47] K. Ulbrich, P. Kreitmeier, O. Reiser, SYNLETT 2010, 13, 2037 - 2040
[48] T. A. Krishnan, M. Chanda, Die Angewandte Makromolekulare Chemie 1974, 43, 145 - 156
[49] B. Zhang, Y. Zhang, G. Ding, H. Zheng, Y. Li, Green Chem. 2012, 14, 3402 - 3409
[50] X. Wang, L. Meng, F. Wu, Y. Jiang, L. Wand, X. Mu, Green Chem. 2012, 14, 758 - 765
[51] H. Zhao, J, Xing, C. Liu, J. Univ. Petrol., China 2003, 27, 1 - 19
[52] Y. Hachihama, M. Imoto, K. Kawata, J. Soc. Chem. Ind., Japan 1942, 45, 189 - 190
[53] D. Papa, E. Schwenk, H.F. Ginsberg, J. Org. Chem. 1951, 16, 253 - 261
[54] R. Connor, H. Adkins, J. Am. Chem. Soc. 1932, 54, 4678 - 4690
[55] W. Klaffke, P. Pudlo, D. Springer, J, Thiem, Liebigs Ann. Chemie 1991, 509 - 512
[56] S.R. Vidyarthi, P.C. Nigam, J.K. Gehlawat, J. Sci. Ind. Res. 1983, 42, 268 - 272
[57] S. Poncet, Speciality Chemicals Magazine 2012, March, 24
[58] M. Chatterjee, H. Kawanami, T. Ishizaka, M. Sato, T. Suzuki, A. Suzuki, Catal. Sci. Tech. 2011, 1, 1466 - 1471
[59] EP0576828B2
[60] US4,459,419
[61] EP1767520A1
[62] EP1020473B1

**Weitere Aspekte der Erfindung**

4.1. Verfahren zur Herstellung von Tetrahydrofurfurylalkohol, dadurch gekennzeichnet, dass man Furfurylalkohol mit Wasserstoff in Gegenwart eines Ru/$AlO_x$-Katalysatorsystems umsetzt.
4.2. Verfahren nach Punkt 4.1, dadurch gekennzeichnet, dass das Aluminiumoxid $Al_2O_3$ ist.
4.3. Verfahren nach Punkt 4.1 oder 4.2, dadurch gekennzeichnet, dass der Gehalt an Ruthenium 0.01 bis 30 Gew.-% bezogen auf das Gesamtgewicht des Ru/$AlO_x$-Katalysatorsystems beträgt.
4.4. Verfahren nach einem oder mehreren der Punkte 4.1 bis 4.3, dadurch gekennzeichnet, dass das Ru/$AlO_x$-Katalysatorsystem eine BET-Oberfläche von 50 bis 250 $m^2$/g Ru/$AlO_x$-Katalysatorsystem aufweist.
4.5. Verfahren nach einem oder mehreren der Punkte 4.1 bis 4.4, dadurch gekennzeichnet, dass das Ru/$AlO_x$-Katalysatorsystem ein durchschnittliches Porenvolumen von 0.2 bis 0.8 ml/g Ru/$AlO_x$-Katalysatorsystem aufweist.
4.6. Verfahren nach einem oder mehreren der Punkte 4.1 bis 4.5, dadurch gekennzeichnet, dass das Ru/$AlO_x$-Katalysatorsystem das folgende Röntgenbeugungsmuster aufweist:

| Pos. [°2Th.] | Relative Intensität [%] |
|---|---|
| 18.3 | 5 - 10 |
| 18.8 | 12 - 17 |
| 20.33 | 6 - 11 |
| 31.37 | 47 - 53 |
| 32.68 | 25 - 32 |

(fortgesetzt)

| Pos. [°2Th.] | Relative Intensität [%] |
|---|---|
| 32.825 | 80 - 85 |
| 35.09 | 10 - 15 |
| 36.758 | 53 - 59 |
| 38.97 | 27 - 32 |
| 39.8 | 30 - 35 |
| 40.63 | 8 - 13 |
| 43.359 | 14 - 18 |
| 44.85 | 62 - 67 |
| 45.47 | 45 - 50 |
| 46.54 | 25 - 30 |
| 47.61 | 30 - 35 |
| 50.77 | 8 - 13 |
| 51.5 | 7 - 12 |
| 57.48 | 7 - 11 |
| 59.97 | 15 - 20 |
| 62.38 | 8 - 13 |
| 62.848 | 8 - 13 |
| 64.05 | 10 - 16 |
| 65.7 | 10 - 15 |
| 66.52 | 54 - 59 |
| 67 | 31 - 37 |
| 67.4 | 100 |
| 73.5 | 5 - 10 |

4.7. Verfahren nach einem oder mehreren der Punkte 4.1 bis 4.6, dadurch gekennzeichnet, dass man es bei einer Temperatur von 100 °C - 280 °C durchführt.
4.8. Verfahren nach einem oder mehreren der Punkte 4.1 bis 4.7, dadurch gekennzeichnet, dass man es unter autoklaven Bedingungen durchführt. Dabei bedeutet "autoklave Bedingungen" im Sinne der Erfindung ein Druck von gleich oder mehr als 10 bar.
4.9. Verfahren nach einem oder mehreren der Punkte 4.1 bis 4.8, dadurch gekennzeichnet, dass man das Verfahren in einem Lösemittel ausgewählt aus der Gruppe bestehend aus Wasser, Ethanol, Tetrahydrofuran und 1,4 - Dioxan durchführt.
4.10. Verfahren nach einem oder mehreren der Punkte 4.1 bis 4.9, dadurch gekennzeichnet, dass man es bei einem pH-Wert von pH 5.3 bis pH 10.5 durchführt.
4.11. Verfahren nach einem oder mehreren der Punkte 4.1 bis 4.10, dadurch gekennzeichnet, dass man es kontinuierlich durchführt.
4.12. Verfahren nach einem oder mehreren der Punkte 4.1 bis 4.11, dadurch gekennzeichnet, dass man es im Batchbetrieb durchführt.
4.13. Verfahren nach einem oder mehreren der Punkte 4.1 bis 4.12, dadurch gekennzeichnet, dass das Ru/$AlO_x$-Katalysatorsystem erhalten wird durch folgende Schritte:

a) Herstellung einer Mischung (i) umfassend eine Rutheniumsalzlösung (ii) und eine wässrige Suspension von Aluminiumoxid (iii);

b) Einstellen der Mischung (i) auf eine Temperatur im Bereich von größer 0°C und kleiner 100 °C und einen pH-Wert von pH 0 - 14;

c) Abtrennen des Ru/$AlO_x$-Katalysatorsystems durch Filtration der Mischung (i);

d) Trocknen des in Schritt c) abgetrennten Ru/$AlO_x$-Katalysatorsystems.

5.1. Verfahren zur Herstellung von Tetrahydrofurfurylalkohol, dadurch gekennzeichnet, dass man Furfurylalkohol mit Wasserstoff in Gegenwart eines Ru/AC-Katalysatorsystems umsetzt.
5.2. Verfahren nach Punkt 5.1, dadurch gekennzeichnet, dass der Gehalt an Ruthenium 0.01 bis 30 Gew.-% bezogen auf das Gesamtgewicht des Ru/AC-Katalysatorsystems beträgt.
5.3. Verfahren nach Punkt 5.1 oder 5.2, dadurch gekennzeichnet, dass das Katalysatorsystem eine BET-Oberfläche von 300 bis 2000 $m^2$/g Ru/AC-Katalysatorsystem aufweist.
5.4. Verfahren nach einem oder mehreren der Punkte 5.1 bis 5.3, dadurch gekennzeichnet, dass das Ru/AC-Katalysatorsystem ein durchschnittliches Porenvolumen von 0.3 bis 2.0 ml/g Ru/AC-Katalysatorsystem aufweist.
5.5. Verfahren nach einem oder mehreren der Punkte 5.1 bis 5.4, dadurch gekennzeichnet, dass man es bei einer Temperatur von 100 °C - 280 °C durchführt.
5.6. Verfahren nach einem oder mehreren der Punkte 5.1 bis 5.5, dadurch gekennzeichnet, dass man es unter autoklaven Bedingungen durchführt. Dabei bedeutet der Begriff "autoklave Bedingungen" im Sinne der Erfindung ein Druck von gleich oder mehr als 10 bar.
5.7. Verfahren nach einem oder mehreren der Punkte 5.1 bis 5.6, dadurch gekennzeichnet, dass man das Verfahren in einem Lösemittel ausgewählt aus der Gruppe bestehend aus Wasser, Ethanol, Tetrahydrofuran und 1,4 - Dioxan durchführt.
5.8. Verfahren nach einem oder mehreren der Punkte 5.1 bis 5.7, dadurch gekennzeichnet, dass man es bei einem pH-Wert von pH 5.3 bis pH 10.5 durchführt.
5.9. Verfahren nach einem oder mehreren der Punkte 5.1 bis 5.8, dadurch gekennzeichnet, dass man es kontinuierlich durchführt.
5.10. Verfahren nach einem oder mehreren der Punkte 5.1 bis 5.9, dadurch gekennzeichnet, dass man es im Batchbetrieb durchführt.
5.11. Verfahren nach einem oder mehreren der Punkte 5.1 bis 5.10, dadurch gekennzeichnet, dass das Ru/AC-Katalysatorsystem erhalten wird durch folgende Schritte:

a) Herstellung einer Mischung (i) umfassend eine Rutheniumsalzlösung (ii) und eine wässrige Suspension von Aktivkohle (iii);

b) Einstellen der Mischung (i) auf eine Temperatur im Bereich von größer 0 °C und kleiner 100 °C und einen pH-Wert von pH 0 - 14;

c) Abtrennen des Ru/AC-Katalysatorsystems durch Filtration der Mischung (i).

6.1. Verfahren zur Herstellung von Tetrahydrofurfurylalkohol, dadurch gekennzeichnet, dass man Furfurylalkohol mit Wasserstoff in Gegenwart von Pt(IV)-Oxid umsetzt.
6.2. Verfahren nach Punkt 6.1, dadurch gekennzeichnet, dass man es bei einer Temperatur von größer 0 °C und kleiner 100 °C durchführt.
6.3. Verfahren nach Punkt 6.1 oder 6.2, dadurch gekennzeichnet, dass man es bei einem Druck von 1 - 10 bar durchführt.
6.4. Verfahren nach einem oder mehreren der Punkte 6.1 bis 6.3, dadurch gekennzeichnet, dass man das Verfahren in einem Lösemittel ausgewählt aus der Gruppe bestehend aus Wasser, Essigsäure, Ethanol durchführt.
6.5. Verfahren nach einem oder mehreren der Punkte 6.1 bis 6.4, dadurch gekennzeichnet, dass man das Verfahren unter Zusatz von Salzsäure durchführt.
7.1. Verfahren zur Herstellung von 1,2-Pentandiol und Tetrahydrofurfurylalkohol, dadurch gekennzeichnet, dass man Furfurylalkohol mit Wasserstoff in Gegenwart eines Ru/$AlO_x$-Katalysatorsystems umsetzt.
7.2. Verfahren nach Punkt 7.1, dadurch gekennzeichnet, dass das Aluminiumoxid $Al_2O_3$ ist.
7.3. Verfahren nach Punkt 7.1 oder 7.2, dadurch gekennzeichnet, dass der Gehalt an Ruthenium 0.01 bis 30 Gew.-% bezogen auf das Gesamtgewicht des Ru/$AlO_x$-Katalysatorsystems beträgt.
7.4. Verfahren nach einem oder mehreren der Punkte 7.1 bis 7.3, dadurch gekennzeichnet, dass das Ru/$AlO_x$-Katalysatorsystem eine BET-Oberfläche von 50 bis 250 $m^2$/g Ru/$AlO_x$-Katalysatorsystem aufweist.
7.5. Verfahren nach einem oder mehreren der Punkte 7.1 bis 7.4, dadurch gekennzeichnet, dass das Ru/$AlO_x$-Katalysatorsystem ein durchschnittliches Porenvolumen von 0.2 bis 0.8 ml/g Ru/$AlO_x$-Katalysatorsystem aufweist.

7.6. Verfahren nach einem oder mehreren der Punkte 7.1 bis 7.5, dadurch gekennzeichnet, dass das Ru/$AlO_x$-Katalysatorsystem das folgende Röntgenbeugungsmuster aufweist:

| Pos. [°2Th.] | Relative Intensität [%] |
|---|---|
| 18.3 | 5 - 10 |
| 18.8 | 12 - 17 |
| 20.33 | 6 - 11 |
| 31.37 | 47 - 53 |
| 32.68 | 25 - 32 |
| 32.825 | 80 - 85 |
| 35.09 | 10 - 15 |
| 36.758 | 53 - 59 |
| 38.97 | 27 - 32 |
| 39.8 | 30 - 35 |
| 40.63 | 8 - 13 |
| 43.359 | 14 - 18 |
| 44.85 | 62 - 67 |
| 45.47 | 45 - 50 |
| 46.54 | 25 - 30 |
| 47.61 | 30 - 35 |
| 50.77 | 8 - 13 |
| 51.5 | 7 - 12 |
| 57.48 | 7 - 11 |
| 59.97 | 15 - 20 |
| 62.38 | 8 - 13 |
| 62.848 | 8 - 13 |
| 64.05 | 10 - 16 |
| 65.7 | 10 - 15 |
| 66.52 | 54 - 59 |
| 67 | 31 - 37 |
| 67.4 | 100 |
| 73.5 | 5 - 10 |

7.7. Verfahren nach einem oder mehreren der Punkte 7.1 bis 7.6, dadurch gekennzeichnet, dass man es bei einer Temperatur von 100 °C - 280 °C durchführt.

7.8. Verfahren nach einem oder mehreren der Punkte 7.1 bis 7.7, dadurch gekennzeichnet, dass man es unter autoklaven Bedingungen durchführt. Dabei bedeutet "autoklave Bedingungen" im Sinne der Erfindung ein Druck von gleich oder mehr als 10 bar.

7.9. Verfahren nach einem oder mehreren der Punkte 7.1 bis 7.8, dadurch gekennzeichnet, dass man das Verfahren in einem Lösemittel ausgewählt aus der Gruppe bestehend aus Wasser, Ethanol, Tetrahydrofuran und 1,4 - Dioxan durchführt.

7.10. Verfahren nach einem oder mehreren der Punkte 7.1 bis 7.9, dadurch gekennzeichnet, dass man es bei einem pH-Wert von pH 5.3 bis pH 10.5 durchführt.

7.11. Verfahren nach einem oder mehreren der Punkte 7.1 bis 7.10, dadurch gekennzeichnet, dass man es kontinuierlich

durchführt.
7.12. Verfahren nach einem oder mehreren der Punkte 7.1 bis 7.11, dadurch gekennzeichnet, dass man es im Batchbetrieb durchführt.
7.13. Verfahren nach einem oder mehreren der Punkte 7.1 bis 7.12, dadurch gekennzeichnet, dass das Ru/$AlO_x$-Katalysatorsystem erhalten wird durch folgende Schritte:

a) Herstellung einer Mischung (i) umfassend eine Rutheniumsalzlösung (ii) und eine wässrige Suspension von Aluminiumoxid (iii);

b) Einstellen der Mischung (i) auf eine Temperatur im Bereich von größer 0°C und kleiner 100 °C und einen pH-Wert von pH 0 - 14;

c) Abtrennen des Ru/$AlO_x$-Katalysatorsystems durch Filtration der Mischung (i);

d) Trocknen des in Schritt c) abgetrennten Ru/$AlO_x$-Katalysatorsystems.

8.1. Verfahren zur Herstellung von 1,2-Pentandiol und Tetrahydrofurfurylalkohol, dadurch gekennzeichnet, dass man Furfurylalkohol mit Wasserstoff in Gegenwart eines Ru/AC-Katalysatorsystems umsetzt.
8.2. Verfahren nach Punkt 8.1, dadurch gekennzeichnet, dass der Gehalt an Ruthenium 0.01 bis 30 Gew.-% bezogen auf das Gesamtgewicht des Ru/AC-Katalysatorsystems beträgt.
8.3. Verfahren nach Punkt 8.1 oder 8.2, dadurch gekennzeichnet, dass das Katalysatorsystem eine BET-Oberfläche von 300 bis 2000 $m^2$/g Ru/AC-Katalysatorsystem aufweist.
8.4. Verfahren nach einem oder mehreren der Punkte 8.1 bis 8.3, dadurch gekennzeichnet, dass das Ru/AC-Katalysatorsystem ein durchschnittliches Porenvolumen von 0.3 bis 2.0 ml/g Ru/AC-Katalysatorsystem aufweist.
8.5. Verfahren nach einem oder mehreren der Punkte 8.1 bis 8.4, dadurch gekennzeichnet, dass man es bei einer Temperatur von 100 °C - 280 °C durchführt.
8.6. Verfahren nach einem oder mehreren der Punkte 8.1 bis 8.5, dadurch gekennzeichnet, dass man es unter autoklaven Bedingungen durchführt. Dabei bedeutet der Begriff "autoklave Bedingungen" im Sinne der Erfindung ein Druck von gleich oder mehr als 10 bar.
8.7. Verfahren nach einem oder mehreren der Punkte 8.1 bis 8.6, dadurch gekennzeichnet, dass man das Verfahren in einem Lösemittel ausgewählt aus der Gruppe bestehend aus Wasser, Ethanol, Tetrahydrofuran und 1,4 - Dioxan durchführt.
8.8. Verfahren nach einem oder mehreren der Punkte 8.1 bis 8.7, dadurch gekennzeichnet, dass man es bei einem pH-Wert von pH 5.3 bis pH 10.5 durchführt.
8.9. Verfahren nach einem oder mehreren der Punkte 8.1 bis 8.8, dadurch gekennzeichnet, dass man es kontinuierlich durchführt.
8.10. Verfahren nach einem oder mehreren der Punkte 8.1 bis 8.9, dadurch gekennzeichnet, dass man es im Batchbetrieb durchführt.
8.11. Verfahren nach einem oder mehreren der Punkte 8.1 bis 8.10, dadurch gekennzeichnet, dass das Ru/AC-Katalysatorsystem erhalten wird durch folgende Schritte:

a) Herstellung einer Mischung (i) umfassend eine Rutheniumsalzlösung (ii) und eine wässrige Suspension von Aktivkohle (iii);

b) Einstellen der Mischung (i) auf eine Temperatur im Bereich von größer 0 °C und kleiner 100 °C und einen pH-Wert von pH 0 - 14;

c) Abtrennen des Ru/AC-Katalysatorsystems durch Filtration der Mischung (i).

9.1. Verfahren zur Herstellung von 1,2-Pentandiol und Tetrahydrofurfurylalkohol, dadurch gekennzeichnet, dass man Furfurylalkohol mit Wasserstoff in Gegenwart von Pt(IV)-Oxid umsetzt.
9.2. Verfahren nach Punkt 9.1, dadurch gekennzeichnet, dass man es bei einer Temperatur von größer 0 °C und kleiner 100 °C durchführt.
9.3. Verfahren nach Punkt 9.1 oder 9.2, dadurch gekennzeichnet, dass man es bei einem Druck von 1 - 10 bar durchführt.
9.4. Verfahren nach einem oder mehreren der Punkte 9.1 bis 9.3, dadurch gekennzeichnet, dass man das Verfahren in einem Lösemittel ausgewählt aus der Gruppe bestehend aus Wasser, Essigsäure, Ethanol durchführt.
9.5. Verfahren nach einem oder mehreren der Punkte 9.1 bis 9.4, dadurch gekennzeichnet, dass man das Verfahren unter Zusatz von Salzsäure durchführt.

## Patentansprüche

**1.** Verfahren zur Herstellung von 1,2-Pentandiol, **dadurch gekennzeichnet, dass** man Furfurylalkohol mit Wasserstoff in Gegenwart eines Katalysatorsystems umfassend

(I) Ruthenium und
(II) einen Träger aus Aluminiumoxid, auf welchem das Ruthenium geträgert ist,
umsetzt.

**2.** Verfahren nach Anspruch 1, **dadurch gekennzeichnet, dass** das Aluminiumoxid $Al_2O_3$ ist.

**3.** Verfahren nach Anspruch 1 oder 2, **dadurch gekennzeichnet, dass** der Gehalt an Ruthenium 0.01 bis 30 Gew.-% bezogen auf das Gesamtgewicht des Katalysatorsystems beträgt.

**4.** Verfahren nach einem oder mehreren der Ansprüche 1 bis 3, **dadurch gekennzeichnet, dass** das Katalysatorsystem eine BET-Oberfläche von 50 bis 250 $m^2$/g Katalysatorsystem aufweist.

**5.** Verfahren nach einem oder mehreren der Ansprüche 1 bis 4, **dadurch gekennzeichnet, dass** das Katalysatorsystem ein durchschnittliches Porenvolumen von 0.2 bis 0.8 ml/g Katalysatorsystem aufweist.

**6.** Verfahren nach einem oder mehreren der Ansprüche 1 bis 5, **dadurch gekennzeichnet, dass** das Katalysatorsystem das folgende Röntgenbeugungsmuster aufweist

| Pos. [°2Th.] | Relative Intensität [%] |
|---|---|
| 18.3 | 5 - 10 |
| 18.8 | 12 - 17 |
| 20.33 | 6 - 11 |
| 31.37 | 47 - 53 |
| 32.68 | 25 - 32 |
| 32.825 | 80 - 85 |
| 35.09 | 10 - 15 |
| 36.758 | 53 - 59 |
| 38.97 | 27 - 32 |
| 39.8 | 30 - 35 |
| 40.63 | 8 - 13 |
| 43.359 | 14 - 18 |
| 44.85 | 62 - 67 |
| 45.47 | 45 - 50 |
| 46.54 | 25 - 30 |
| 47.61 | 30 - 35 |
| 50.77 | 8 - 13 |
| 51.5 | 7 - 12 |
| 57.48 | 7 - 11 |
| 59.97 | 15 - 20 |
| 62.38 | 8 - 13 |
| 62.848 | 8 - 13 |

(fortgesetzt)

| Pos. [°2Th.] | Relative Intensität [%] |
|---|---|
| 64.05 | 10 - 16 |
| 65.7 | 10 - 15 |
| 66.52 | 54 - 59 |
| 67 | 31 - 37 |
| 67.4 | 100 |
| 73.5 | 5 - 10 |

7. Verfahren nach einem oder mehreren der Ansprüche 1 bis 6, **dadurch gekennzeichnet, dass** man es bei einer Temperatur von 100°C - 280°C durchführt.

8. Verfahren nach einem oder mehreren der Ansprüche 1 bis 7, **dadurch gekennzeichnet, dass** man es unter autoklaven Bedingungen durchführt.

9. Verfahren nach einem oder mehreren der Ansprüche 1 bis 8, **dadurch gekennzeichnet, dass** man das Verfahren in einem Lösemittel ausgewählt aus der Gruppe bestehend aus Wasser, Ethanol, Tetrahydrofuran und 1,4 - Dioxan durchführt.

10. Verfahren nach einem oder mehreren der Ansprüche 1 bis 9, **dadurch gekennzeichnet, dass** man es bei einem pH-Wert von pH 5.3 bis pH 10.5 durchführt.

11. Verfahren nach einem oder mehreren der Ansprüche 1 bis 10, **dadurch gekennzeichnet, dass** man es kontinuierlich durchführt.

12. Verfahren nach einem oder mehreren der Ansprüche 1 bis 11, **dadurch gekennzeichnet, dass** man es im Batchbetrieb durchführt.

13. Verfahren nach einem oder mehreren der Ansprüche 1 bis 12, **dadurch gekennzeichnet, dass** das Katalysatorsystem erhalten wird durch folgende Schritte:

    a) Herstellung einer Mischung (i) umfassend eine Rutheniumsalzlösung (ii) und eine wässrige Suspension von Aluminiumoxid (iii);
    b) Einstellen der Mischung (i) auf eine Temperatur im Bereich von größer 0 °C und kleiner 100 °C und einen pH-Wert von pH 0 - 14;
    c) Abtrennen des Katalysatorsystems durch Filtration der Mischung (i);
    d) Trocknen des in Schritt c) abgetrennten Katalysatorsystems.

14. Verfahren zur Herstellung eines Katalysatorsystems umfassend

    a) Herstellung einer Mischung (i) umfassend eine Rutheniumsalzlösung (ii) und eine wässrige Suspension von Aluminiumoxid (iii);
    b) Einstellen der Mischung (i) auf eine Temperatur im Bereich von größer 0°C und kleiner 100°C und einen pH-Wert von pH 0 - 14;
    c) Abtrennen des Katalysatorsystems durch Filtration der Mischung (i);
    d) Trocknen des in Schritt c) abgetrennten Katalysatorsystems.

15. Katalysatorsystem, welches durch ein Verfahren nach Anspruch 14 erhalten wird.

16. Verfahren zur Herstellung von Tetrahydrofurfurylalkohol, **dadurch gekennzeichnet, dass** man Furfurylalkohol mit Wasserstoff in Gegenwart eines Katalysatorsystems umfassend

    (I) Ruthenium und
    (II) einen Träger aus Aluminiumoxid, auf welchem das Ruthenium geträgert ist,

umsetzt.

17. Verfahren nach Anspruch 16, **dadurch gekennzeichnet, dass** das Aluminiumoxid $Al_2O_3$ ist.

18. Verfahren nach Anspruch 16 oder 17, **dadurch gekennzeichnet, dass** der Gehalt an Ruthenium 0.01 bis 30 Gew.-% bezogen auf das Gesamtgewicht des Katalysatorsystems beträgt.

19. Verfahren nach einem oder mehreren der Ansprüche 16 bis 18, **dadurch gekennzeichnet, dass** das Katalysatorsystem eine BET-Oberfläche von 50 bis 250 $m^2/g$ Katalysatorsystem aufweist.

20. Verfahren nach einem oder mehreren der Ansprüche 16 bis 19, **dadurch gekennzeichnet, dass** das Katalysatorsystem ein durchschnittliches Porenvolumen von 0.2 bis 0.8 ml/g Katalysatorsystem aufweist.

21. Verfahren nach einem oder mehreren der Ansprüche 16 bis 20, **dadurch gekennzeichnet, dass** das Katalysatorsystem das folgende Röntgenbeugungsmuster aufweist

| Pos. [°2Th.] | Relative Intensität [%] |
|---|---|
| 18.3 | 5 - 10 |
| 18.8 | 12 - 17 |
| 20.33 | 6 - 11 |
| 31.37 | 47 - 53 |
| 32.68 | 25 - 32 |
| 32.825 | 80 - 85 |
| 35.09 | 10 - 15 |
| 36.758 | 53 - 59 |
| 38.97 | 27 - 32 |
| 39.8 | 30 - 35 |
| 40.63 | 8 - 13 |
| 43.359 | 14 - 18 |
| 44.85 | 62 - 67 |
| 45.47 | 45 - 50 |
| 46.54 | 25 - 30 |
| 47.61 | 30 - 35 |
| 50.77 | 8 - 13 |
| 51.5 | 7 - 12 |
| 57.48 | 7 - 11 |
| 59.97 | 15 - 20 |
| 62.38 | 8 - 13 |
| 62.848 | 8 - 13 |
| 64.05 | 10 - 16 |
| 65.7 | 10 - 15 |
| 66.52 | 54 - 59 |
| 67 | 31 - 37 |
| 67.4 | 100 |

(fortgesetzt)

| Pos. [°2Th.] | Relative Intensität [%] |
|---|---|
| 73.5 | 5 - 10 |

**22.** Verfahren nach einem oder mehreren der Ansprüche 16 bis 21, **dadurch gekennzeichnet, dass** man es bei einer Temperatur von 100°C - 280°C durchführt.

**23.** Verfahren nach einem oder mehreren der Ansprüche 16 bis 22, **dadurch gekennzeichnet, dass** man es unter autoklaven Bedingungen durchführt.

**24.** Verfahren nach einem oder mehreren der Ansprüche 16 bis 23, **dadurch gekennzeichnet, dass** man das Verfahren in einem Lösemittel ausgewählt aus der Gruppe bestehend aus Wasser, Ethanol, Tetrahydrofuran und 1,4 - Dioxan durchführt.

**25.** Verfahren nach einem oder mehreren der Ansprüche 16 bis 24, **dadurch gekennzeichnet, dass** man es bei einem pH-Wert von pH 5.3 bis pH 10.5 durchführt.

**26.** Verfahren nach einem oder mehreren der Ansprüche 16 bis 25, **dadurch gekennzeichnet, dass** man es kontinuierlich durchführt.

**27.** Verfahren nach einem oder mehreren der Ansprüche 16 bis 26, **dadurch gekennzeichnet, dass** man es im Batchbetrieb durchführt.

**28.** Verfahren nach einem oder mehreren der Ansprüche 16 bis 27, **dadurch gekennzeichnet, dass** das Katalysatorsystem erhalten wird durch folgende Schritte:

a) Herstellung einer Mischung (i) umfassend eine Rutheniumsalzlösung (ii) und eine wässrige Suspension von Aluminiumoxid (iii);
b) Einstellen der Mischung (i) auf eine Temperatur im Bereich von größer 0 °C und kleiner 100 °C und einen pH-Wert von pH 0 - 14;
c) Abtrennen des Katalysatorsystems durch Filtration der Mischung (i);
d) Trocknen des in Schritt c) abgetrennten Katalysatorsystems.

Europäisches Patentamt
European Patent Office
Office européen des brevets

# EUROPÄISCHER RECHERCHENBERICHT

Nummer der Anmeldung
EP 13 19 2811

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | MANOJ A. TIKE ET AL: "Kinetics of Liquid-Phase Hydrogenation of Furfuryl Alcohol to Tetrahydrofurfuryl Alcohol over a Ru/TiO 2 Catalyst", INDUSTRIAL & ENGINEERING CHEMISTRY RESEARCH, Bd. 46, Nr. 10, 1. Mai 2007 (2007-05-01), Seiten 3275-3282, XP055122236, ISSN: 0888-5885, DOI: 10.1021/ie061137m * Absätze [02.2] - [02.4], [03.1]; Abbildungen 2,3 * | 1-28 | INV. C07D307/12 C07C29/17 B01J21/04 B01J23/46 ADD. C07C31/20 |
| X | EP 1 162 186 A2 (BAYER AG [DE]) 12. Dezember 2001 (2001-12-12) * Beispiel 12 * | 14,15 | |
| A,D | BIN ZHANG ET AL: "Selective conversion of furfuryl alcohol to 1,2-pentanediol over a Ru/MnOx catalyst in aqueous phase", GREEN CHEMISTRY, Bd. 14, Nr. 12, 1. Januar 2012 (2012-01-01), Seite 3402, XP055122248, ISSN: 1463-9262, DOI: 10.1039/c2gc36270h * das ganze Dokument * | 1-28 | |
| | | | RECHERCHIERTE SACHGEBIETE (IPC) |
| | | | C07D C07C B01J |

Der vorliegende Recherchenbericht wurde für alle Patentansprüche erstellt

1

| Recherchenort | Abschlußdatum der Recherche | Prüfer |
|---|---|---|
| Den Haag | 12. Juni 2014 | Ginoux, Claude |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 13 19 2811

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-06-2014

| Im Recherchenbericht angeführtes Patentdokument | Datum der Veröffentlichung | Mitglied(er) der Patentfamilie | Datum der Veröffentlichung |
|---|---|---|---|
| EP 1162186 A2 | 12-12-2001 | CN 1323775 A | 28-11-2001 |
| | | DE 10023283 A1 | 15-11-2001 |
| | | EP 1162186 A2 | 12-12-2001 |
| | | JP 2001322960 A | 20-11-2001 |
| | | US 2002019573 A1 | 14-02-2002 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 2077422 A, W. Lazier
- US 2094975 A, H. Adkins, R. Conner
- DE 2937840, Degussa
- US 2097493 A
- US 4459419 A
- EP 0576828 B2
- EP 1767520 A1
- EP 1020473 B1


### In der Beschreibung aufgeführte Nicht-Patentliteratur


- **A. CORMA ; S. IBORRA ; A. VELTY.** *Chem. Rev.,* 2007, vol. 107, 241-2502
- **H. E. HOYDONCKX et al.** Ullmann's Encyclopedia of Industrial Chemistry. WILEY-VCH
- **P. GALLEZOT.** *Chem. Soc. Rev.,* 2012, vol. 41, 1538-1558
- **P. ANASTAS ; N. EGHBALI.** *Chem. Soc. Rev.,* 2010, vol. 39, 301-312
- **P. ANASTAS ; M. KIRCHHOFF ; T. WILLIAMSON.** *Applied Catalysis A: General,* 2001, vol. 221, 3-13
- **J. W. DÖBEREINER.** *Berichte der deutschen chemischen Gesellschaft,* vol. 3, 141-146
- **B. A. TOKAY.** Ullmann's Encyclopedia of Industrial Chemistry. WILEY-VCH
- **I. S. GOLDSTEIN ; W. A. DREHER.** *Industrial and Engineering Chemistry,* 1960, vol. 52, 57-58
- **R. KADYROV et al.** *Angew. Chem. Int. Ed.,* 2009, vol. 48, 7556-7559
- **L. B. HUNT.** *Platinum Metals Rev.,* 1962, vol. 6, 150-152
- **W. E. KAUFMANN ; R. ADAMS.** *J. Am. Chem. Soc.,* 1923, vol. 45, 3029-3044
- **S. NISHIMURA.** Handbook of Heterogeneous Catalytic Hydrogenation for Organic Synthesis. John Wiley & Sons, 2001
- **S. NISHIMURA.** *Bulletin of the Chemical Society of Japan,* 1961, vol. 34, 32-36
- **H. A. SMITH ; J. F. FUZEK.** *J. Am. Chem. Soc.,* 1949, vol. 71, 415-419
- **W. XU et al.** *Chem. Commun.,* 2011, vol. 47, 3924-3926
- **H. ADKINS ; R. CONNOR.** *J. Am. Chem. Soc.,* 1931, vol. 53, 1091-1095
- **D. G. MANLY ; A. P. DUNLOP.** *J. Org. Chem.,* 1958, vol. 23, 1093-1095
- **N. MERAT ; C. GODAWA ; A. GASET.** *J. Chem. Tech. Biotechnol.,* 1990, vol. 48, 145-159
- **Y. SONG et al.** *Front. Chem. Eng. China,* 2007, vol. 1, 151-154
- **B. H. WOJCIK.** *Ind. Eng. Chem.,* 1948, vol. 40, 210-216
- **F. A. KHAN ; A. VALLAT ; G. SÜSS-FINK.** *Catal. Commun.,* 2011, vol. 12, 4128-1431
- **M. H. TIKE ; V. V. MAHAJANI.** *Ind. Eng. Chem. Res.,* 2007, vol. 46, 3275-3282
- **K. TOMISHIGE et al.** *Chem. Comm.,* 2009, vol. 15, 2035-2037
- **K. TOMISHIGE ; S. KOSO ; Y. NAKAGAWA.** *Journal of Catalysis,* 2011, vol. 2, 221-229
- **A. SUZUKI et al.** *Catal. Sci. Technol.,* 2011, vol. 1, 1466-1471
- **P. WALDEN.** *Bull Acad. Sci. St. Petersburg,* 1914, 405-422
- **J. S. SANDHU.** *Green Chemistry Letters and Reviews,* 2011, vol. 4, 289-310
- **S. E. FRY ; N. J. PIENTA.** *J. Am. Chem. Soc.,* 1985, vol. 107, 6399-6400
- **J. S. WILKES et al.** *J. Org. Chem,* 1986, vol. 51, 480-483
- **A. JESS et al.** *Chem. Eng. Technol.,* 2007, vol. 30, 985-994
- **M. STARK et al.** *Adv. Mater,* 2011, vol. 23, 2571-2587
- **P. CLAUS et al.** *J. Phys. Chem.,* 2010, vol. 114, 10520-10526
- **P. CLAUS et al.** *Chem. Commun.,* 2008, 4058-4060
- **P. CLAUS et al.** *Chem. Commun.,* 2009, vol. 11, 716-723
- **Y.-W. LI et al.** *Journal of Molecolar Catalysis A: Chemical,* 2006, vol. 246, 18-23
- **M. HRONEC ; K. FULAJTAROVA.** *Catalysis Communications,* 2012, vol. 24, 100-104
- **K. ULBRICH ; P. KREITMEIER ; O. REISER.** *SYNLETT,* 2010, vol. 13, 2037-2040
- **T. A. KRISHNAN ; M. CHANDA.** *Die Angewandte Makromolekulare Chemie,* 1974, vol. 43, 145-156
- **B. ZHANG ; Y. ZHANG ; G. DING ; H. ZHENG ; Y. LI.** *Green Chem.,* 2012, vol. 14, 3402-3409

- **X. WANG ; L. MENG ; F. WU ; Y. JIANG ; L. WAND ; X. MU.** *Green Chem.,* 2012, vol. 14, 758-765
- **H. ZHAO ; J, XING ; C. LIU.** *J. Univ. Petrol., China,* 2003, vol. 27, 1-19
- **Y. HACHIHAMA ; M. IMOTO ; K. KAWATA.** *J. Soc. Chem. Ind., Japan,* 1942, vol. 45, 189-190
- **D. PAPA ; E. SCHWENK ; H.F. GINSBERG.** *J. Org. Chem.,* 1951, vol. 16, 253-261
- **R. CONNOR ; H. ADKINS.** *J. Am. Chem. Soc.,* 1932, vol. 54, 4678-4690
- **W. KLAFFKE ; P. PUDLO ; D. SPRINGER ; J, THIEM.** *Liebigs Ann. Chemie,* 1991, 509-512
- **S.R. VIDYARTHI ; P.C. NIGAM ; J.K. GEHLAWAT.** *J. Sci. Ind. Res.,* 1983, vol. 42, 268-272
- **S. PONCET.** *Speciality Chemicals Magazine,* 24. Marz 2012
- **M. CHATTERJEE ; H. KAWANAMI ; T. ISHIZAKA ; M. SATO ; T. SUZUKI ; A. SUZUKI.** *Catal. Sci. Tech.,* 2011, vol. 1, 1466-1471